# EUROPEAN PATENT APPLICATION

(11) **EP 3 480 195 A1**
(43) Date of publication of application: **08.05.2019**
(21) Application number: 17200415.2
(22) Date of filing: 07.11.2017
(51) Int. Cl.: C07D 413/14, A01N 43/72, A61K 38/15, A61K 45/06, C07C 269/06, C07C 271/22, C07B 51/00, C07C 231/12, C07D 273/00, C07D 273/08, C07K 11/02

(54) **METHOD FOR THE SYNTHESIS OF CYCLIC DEPSIPEPTIDES**

(71) Applicant: Bayer Animal Health GmbH, 51373 Leverkusen (DE); The Kitasato Institute, Tokyo 108-8641 (JP)
(72) Inventor: The designation of the inventor has not yet been filed
(74) Representative: BIP Patents

(57) **Abstract**

The present invention relates to a method for the synthesis of cyclic depsipeptides, in particular emodepside, involving specific carboxylic acid group protecting tags: 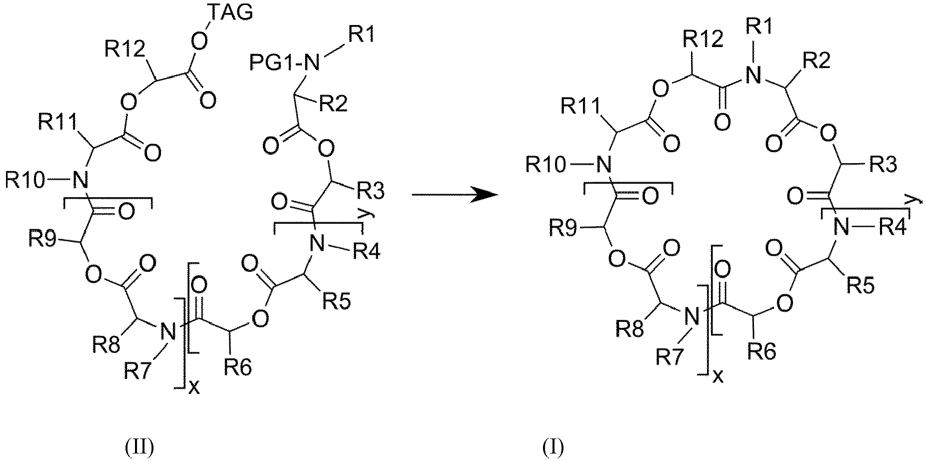 TAG is preferably: 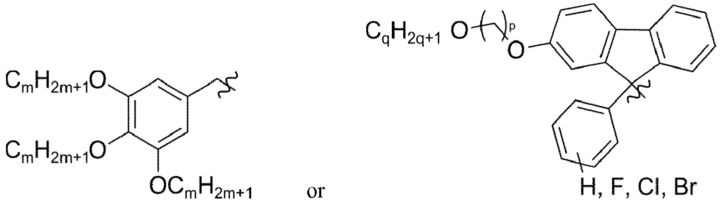 wherein m is ≥ 15 to ≤ 25, p is ≥ 8 to ≤ 18 and q is ≥ 15 to ≤ 25.

## Description

The present invention relates to a method for the synthesis of cyclic depsipeptides, in particular emodepside, involving specific carboxylic acid group protecting tags.

Emodepside (cyclo[(*R*)-lactoyl-*N*-methyl-L-leucyl-(*R*)-3-(*p*-morpholinophenyl)lactoyl-*N*-methyl-L-leucyl-(*R*)-lactoyl-*N*-methyl-*L*-leucyl-(*R*)-3-(*p*-morpholinophenyl)lactoyl-*N*-methyl-*L*-leucyl) is an anthelmintic drug that is effective against a number of gastrointestinal nematodes. Its molecular structure which is depicted below can be described as a cyclic octadepsipeptides, a depsipeptide being a peptide in which one or more of its amide groups are replaced by the corresponding ester groups. On a technical scale emodepside may be obtained by derivatization of the naturally occurring substance PF1022A in which two hydrogen atoms are exchanged for morpholine rings.

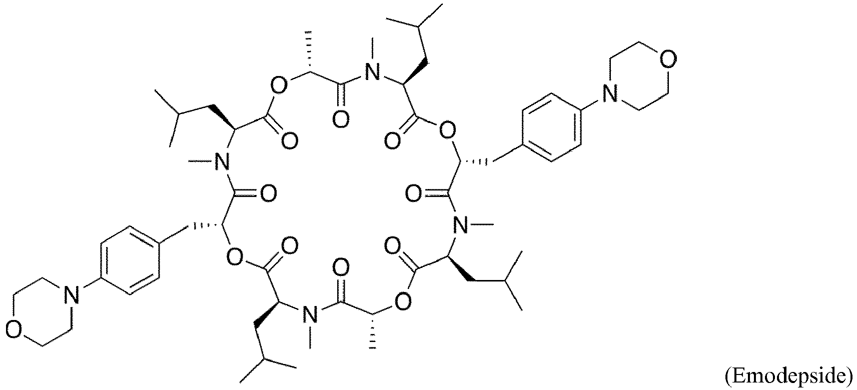

WO 93/19053 A1 (EP 0 634 408 A1) discloses a compound of the general formula: 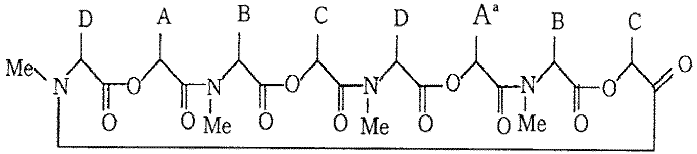 wherein A is benzyl group which has suitable substituent(s) or phenyl group which may have suitable substituent(s), A^{a} is benzyl group which may have suitable substituent(s) or phenyl group which may have suitable substituent(s), B and D are each lower alkyl, C is hydrogen or lower alkyl, and a pharmaceutically acceptable salt thereof.

WO 2005/055973 A2 discloses transdermally applicable agents containing cyclic depsipeptides and/or praziquantel, in addition to the production thereof and to the use thereof for fighting against endoparasites. Combinations of emodepside and praziquantel or epsiprantel and also 1,2-isopropylideneglycol as endoparasiticides are disclosed in WO 2006/094664 A1.

WO 2006/053641 A1 relates to the use of endoparasiticidal depsipeptides for producing pharmaceuticals for preventing vertical infection with endoparasites.

The total synthesis of, amongst others, PF1022A and its derivatization are discussed in the review article "Cyclodepsipeptides: A Rich Source of Biologically Active Compounds for Drug Research" by Sivatharushan Sivanathan and Jürgen Scherkenbeck, Molecules 2014, 19, 12368-12420; doi:10.3390/molecules190812368. For several cyclodepsipeptides total syntheses both in solution and on solid-phase have been established, allowing the production of combinatorial libraries. In addition, the biosynthesis of specific cyclodepsipeptides has been elucidated and used for the chemoenzymatic preparation of nonnatural analogues. The review article also summarizes the recent literature on cyclic tetra- to decadepsipeptides, composed exclusively of α-amino- and α-hydroxy acids.

In the synthesis of polypeptides a solid phase strategy has the advantage of easy separation of the reaction products, whereas a liquid phase strategy has the advantage of homogenous reaction conditions. A hybrid approach is a tag-assisted strategy where compounds having a tag group are easily separated from untagged molecules.

In this respect, JP 2000/044493 A1 discloses a protecting group for synthesizing a compound library which consists of compounds capable of bonding with a compound to be protected in 1:1 ratio, having a single molecular structure and also having >=500 molecular weight, and consists of 3,4,5-tris-(n-octadecyloxy)benzyl alcohol, 3,4,5-tris-(n-octadecyloxy)benzyl chloride or methyl 3,4,5-tris-(n-octadecyloxy)benzoate.

EP 2 003 104 A2 relates to a reagent for organic synthesis with which a chemical reaction can be conducted in a liquid phase and unnecessary compound(s) can be easily separated at low cost from the liquid phase after completion of the reaction. The reagent for organic synthesis which is depicted below is reported to reversibly change from a liquid-phase state to a solid-phase state with changes in solution composition and/or solution temperature, and is for use in organic synthesis reactions.

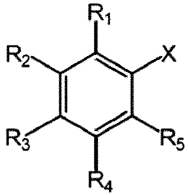

R₁ to R₅ may be the same or different, and represent hydrogen, halogen, alkyl group with a carbon number of 1 to 30 which may have a substituent group, alkoxyl group with a carbon number of 1 to 30 which may have a substituent group, aryl group with a carbon number of 1 to 30 which may have a substituent group, acyl group with a carbon number of 1 to 30 which may have a substituent group, thioalkyl group with a carbon number of 1 to 30 which may have a substituent group, dialkylamino group with a carbon number of 1 to 30 which may have a substituent group, nitro group, or amino group; and at least two of R₁ to R5 are groups with a carbon number of 18 to 30, and X represents a reagent active site having one or more atoms selected from the group consisting of a carbon atom, oxygen atom, sulfur atom, and nitrogen atom.

The publication "Tag-Assisted Liquid-Phase Peptide Synthesis Using Hydrophobic Benzyl Alcohols as Supports" by Yohei Okada, Hideaki Suzuki, Takashi Nakae, Shuji Fujita, Hitoshi Abe, Kazuo Nagano, Toshihide Yamada, Nobuyoshi Ebata, Shokaku Kim, and Kazuhiro Chiba, The Journal of Organic Chemistry 2013, 78, 320-327; doi: 10.1021/jo302127d; reports that a soluble tag-assisted liquid-phase peptide synthesis was successfully established based on simple hydrophobic benzyl alcohols, which can be easily prepared from naturally abundant materials. Excellent precipitation yields are reported to be obtained at each step, combining the best properties of solid-phase and liquid-phase techniques. This approach is reported to be able to be applied efficiently to fragment couplings, allowing chemical synthesis of several bioactive peptides.

The publication "A Novel Protecting Group for Constructing Combinatorial Peptide Libraries" by Hitoshi Tamiaki, Tomoyuki Obata, Yasuo Azefu and Kazunori Toma, Bulletin of the Chemical Society of Japan 2001, 74, 733-738; doi http://dx.doi.org/10.1246/bcsj.74.733; discloses 3,4,5-tris(octadecyloxy)benzyl alcohol, HO-Bzl(OC₁₈)₃ which was prepared from gallic acid and stearyl bromide. Using conventional step-wise elongation, N,C-protected peptides, Fmoc-AAₙ-...-AA₁-OBzl(OC₁₈)₃, were synthesized. The substituted benzyl esters were selectively cleaved by a treatment with 4 M hydrogen chloride in ethyl acetate to give Fmoc-AAₙ-...-AA₁-OP and HO-Bzl(OC₁₈)₃. Thus, the substituted benzyl group is reported to be effective for the protection of C-terminal carboxyl groups in liquid-phase peptide synthesis. Because the substituted benzyl group has a moderately high molecular weight, Fmoc-AAₙ-...-AA₁-OBzl(OC₁₈)₃ is reported to be easily purified by size-exclusion chromatography; all protected peptides were reported to be eluted in the void fraction of a Sephadex LH-20 gel-filtration column. The combination of the carboxyl-protecting group Bzl(OC₁₈)₃ with simple purification by the gel-filtration is reported to give a novel route for constructing combinatorial peptide libraries in the solution phase.

WO 2017/116702 A1 relates to a cyclic depsipeptide compound or a pharmaceutically or veterinarily acceptable salt of the following formula:

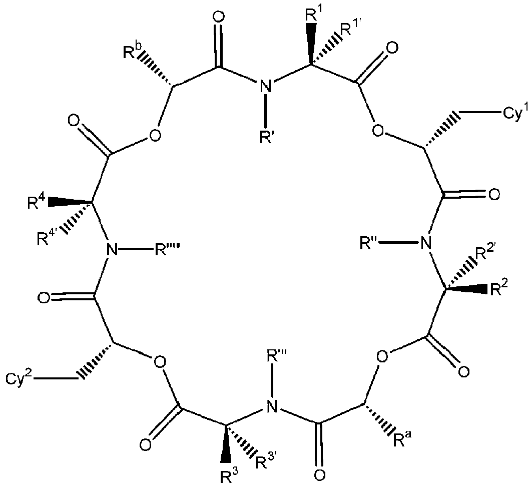

By way of example, a compound with the designation 7-34A as depicted below shows an EC₅₀ value between 0.1 µM and 1.0 µM in an *in vitro* test against microfilaria of *Dirofilaria immitis:*

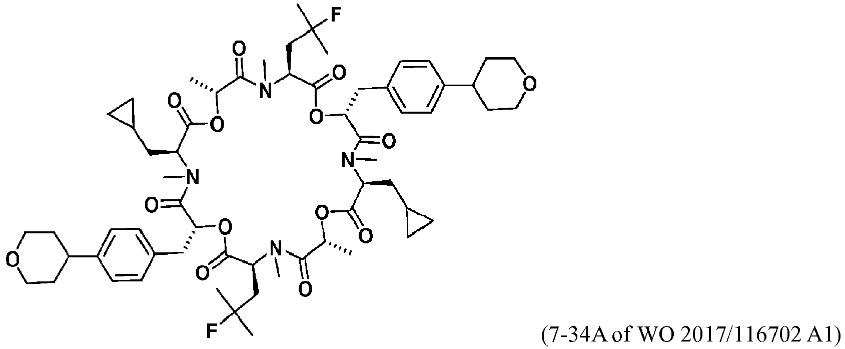

The present invention has the object of providing an improved synthetic route to emodepside and related cyclic depsipeptides. Another object of the invention is to provide novel depsipeptides which may be synthesized using this route.

This object is achieved by a method according to claim 1 and depsipeptides according to claim 15. Advantageous embodiments are the subject of the dependent claims. They may be combined freely unless the context clearly indicates otherwise.

Accordingly, the present invention provides a method for the synthesis of cyclic depsipeptides according to the general formula (1) from depsipeptides according to the general formula (II): 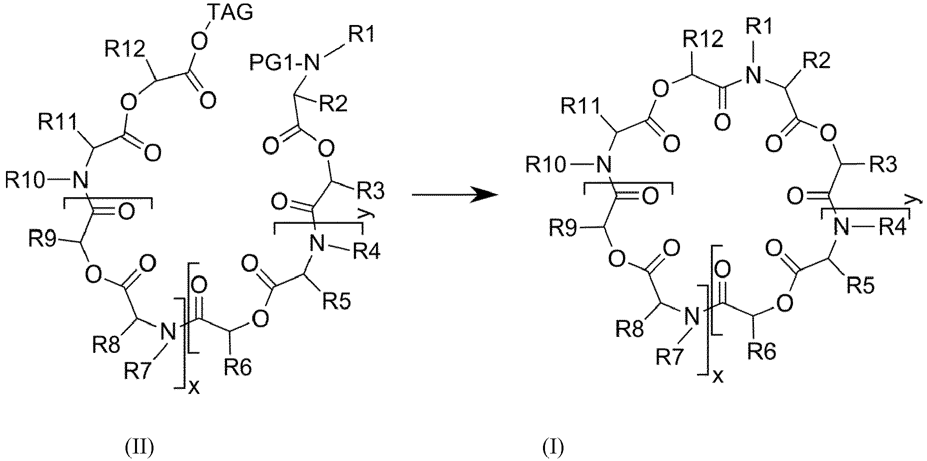 wherein PG1 is an amine protecting group and TAG is a carboxylic acid protecting group, the method comprising the steps of:
- deprotecting the amine group which is protected by the PG1 group in the presence of a base, thereby obtaining a deprotected amine group;
- deprotecting the carboxylic acid which is protected by the TAG group in the presence of an acid, thereby obtaining a deprotected carboxylic acid group;
- condensation of the deprotected amine and carboxylic acid groups, thereby obtaining the cyclic depsipeptide (I)

The TAG group comprises a moiety Aryl-O-(CH₂)ₙ with Aryl representing an aromatic moiety and n being ≥ 13, x and y are, independent of each other, 0, 1 or 2 with the proviso that x+y ≥ 1 (preferably, x and y are 1) and R1, R2, R3, R4, R5, R6, R7, R8, R9, R10, R11 and R12 each, independent of each other, represent hydrogen, straight-chain or branched C1-C8-alkyl, in particular methyl, ethyl, propyl, isopropyl, butyl, isobutyl, sec-butyl, tert-butyl, pentyl, isopentyl, sec-pentyl, hexyl, isohexyl, sec-hexyl, heptyl, isoheptyl, sec-heptyl, tert-heptyl, octyl, isooctyl, sec-octyl, straight-chain or branched halogenated C1-C8 alkyl, in particular fluorinated sec-butyl, hydroxy-C1-C6-alkyl, in particular hydroxymethyl, 1-hydroxyethyl, C1-C4-alkanoyloxy-C1-C6-alkyl, in particular acetoxymethyl, 1-acetoxyethyl, C1-C4-alkoxy-C1-C6-alkyl, in particular methoxymethyl, 1-methoxyethyl, aryl-C1-C4-alkyloxy-C1-C6-alkyl, in particular benzyloxymethyl, 1-benzyloxyethyl, mercapto-C1-C6-alkyl, in particular mercaptomethyl, C1-C4-alkylthio-C1-C6-alkyl, in particular methylthioethyl, C1-C4-alkylsulphinyl-C1-C6-alkyl, in particular methylsulphinylethyl, C1-C4-alkylsulphonyl-C1-C6-alkyl, in particular methylsulphonylethyl, carboxy-C1-C6-alkyl, in particular carboxymethyl, carboxyethyl, C1-C4-alkoxycarbonyl-C1-C6-alkyl, in particular methoxycarbonylmethyl, ethoxycarbonylethyl, C1-C4-arylalkoxycarbonyl-C1-C6-alkyl, in particular benzyloxycarbonylmethyl, carbamoyl-C1-C6-alkyl, in particular carbamoylmethyl, carbamoylethyl, amino-C1-C6-alkyl, in particular aminopropyl, aminobutyl, C1-C4-alkylamino-C1-C6-alkyl, in particular methylaminopropyl, methylaminobutyl, C1-C4-dialkylamino-C1-C6-alkyl, in particular dimethylaminopropyl, dimethylaminobutyl, guanidino-C1-C6-alkyl, in particular guanidinopropyl, C1-C4-alkoxycarbonylamino-C1-C6-alkyl, in particular tert-butoxycarbonylaminopropyl, tert-butoxycarbonylaminobutyl, 9-fluorenylmethoxycarbonyl(Fmoc)amino-C1-C6-alkyl, in particular 9-fluorenylmethoxycarbonyl(Fmoc)aminopropyl, 9-fluorenylmethoxycarbonyl(Fmoc)aminobutyl, C2-C8-alkenyl, in particular vinyl, allyl, butenyl, C3-C7-cycloalkyl, in particular cyclopentyl, cyclohexyl, cycloheptyl, C3-C7-cycloalkyl-C1-C4-alkyl, in particular cyclopentylmethyl, cyclohexylmethyl, cycloheptylmethyl, benzyl, substituted benzyl, phenyl, phenyl-C1-C4-alkyl, in particular phenylmethyl which may optionally be substituted by radicals from the group consisting of halogen, in particular fluorine, chlorine, bromine or iodine, hydroxyl, C1-C4-alkoxy, in particular methoxy or ethoxy, C1-C4-alkyl, in particular methyl.

In the method according to the invention it has surprisingly been found that cyclic depsipeptides (I), in particular emodepside and closely related structures, can be synthesized in high overall yields using hydrophobic carboxylic acid protecting groups TAG. These groups can render the molecules to which they are bound ("tagged" molecules) insoluble in polar solvents such as methanol. Hence, the tagged molecules can be precipitated out of a reaction mixture and the tagging group itself, after deprotection, can also be separated using this technique. Furthermore, the hydrophobic tagging group allows tagged molecules to be soluble in unpolar solvents such as dichloromethane.

The deprotection of the amine group by removing the protection group PG1 can be performed using standard base-assisted procedures such as treatment with a piperidine solution in dichloromethane. Likewise, the deprotection of the carboxylic acid group by removing the TAG group can be performed using protocols for removing a benzyl group such as treatment with a solution of trifluoroacetic acid (TFA) in dichloromethane.

After deprotection the amine and carboxylic acid groups are reacted to form a peptide bond. Suitable coupling agents together with a base such as N,N-Diisopropylethylamine include PyBOP (benzotriazol-1-yl-oxytripyrrolidinophosphonium hexafluorophosphate), BOP ((Benzotriazol-1-yloxy)tris(dimethylamino)phosphonium hexafluorophosphate), DEPBT (3-(diethoxyphosphoryloxy)-1,2,3-benzotriazin-4(3H)-one), HATU (1-[Bis(dimethylamino)methylene]-1H-1,2,3-triazolo[4,5-b]pyridinium 3-oxid hexafluorophosphate), HBTU (2-(1H-benzotriazol-1-yl)-1,1,3,3-tetramethyluronium hexafluorophosphate) and, most preferred, T3P® (Propylphosphonic anhydride, 2,4,6-Tripropyl-1,3,5,2,4,6-trioxatriphosphorinane-2,4,6-trioxide, PPACA).

In one embodiment of the method according to the invention x is 1, y is 1, R1, R4, R7 and R10 are methyl, R6 and R12 are methyl, R5 and R11 are, independent of each other, straight-chain or branched C1-C4-alkyl or straight-chain or branched halogenated C1-C4-alkyl and R3 and R9 are, independent of each other, benzyl or substituted benzyl.

In another embodiment of the method according to the invention PG1 is 9-fluorenylmethoxycarbonyl (Fmoc), t-butyl carbamate (Boc), benzyl carbamate (Z), acetamide, trifluoroacetamide, phthalimide, benzyl (Bn), triphenylmethyl (Tr), benzylidene or p-toluenesulfonamide (Ts)
and TAG is: 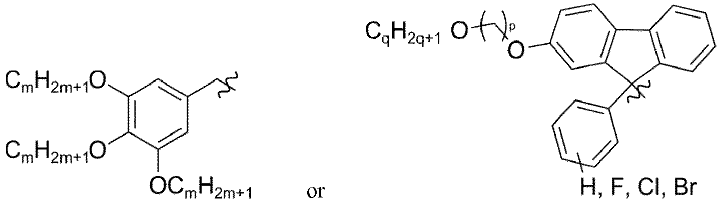 wherein m is ≥ 15 to ≤ 25, p is ≥ 8 to ≤ 18 and q is ≥ 15 to ≤ 25. Preferably, m is 18, 19, 20, 21 or 22, p is 11, 12 or 13 and q is 21, 22 or 23.

In another embodiment of the method according to the invention the depsipeptide according to the general formula (II) is obtained from precursors according to the general formulas (IV) and (III): 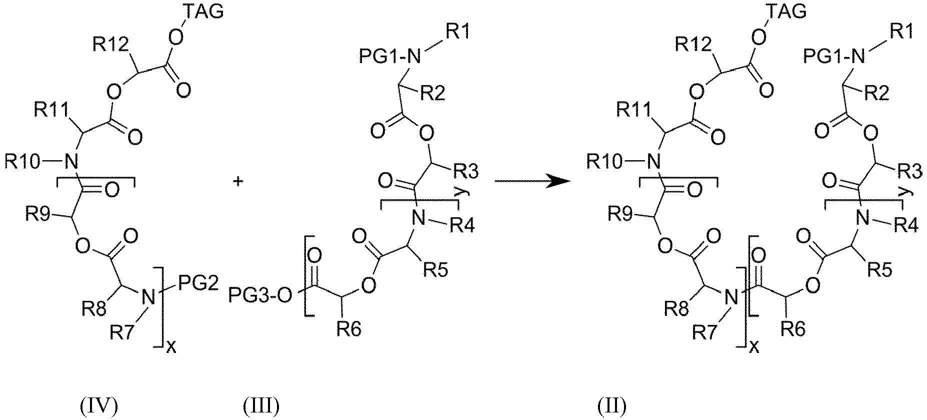 by:
- in precursor (IV), deprotecting the amine group which is protected by the PG2 group in the presence of a base, thereby obtaining a deprotected amine group;
- in precursor (III), deprotecting the carboxylic acid which is protected by the PG3 group in the presence of an acid, thereby obtaining a deprotected carboxylic acid group;
- condensation of the deprotected amine and carboxylic acid groups, thereby obtaining the depsipeptide (II);
wherein R1 to R12, TAG, PG1, x, and y have a meaning as defined above (in particular, x and y may be 1), PG2 is an amine protecting group and PG3 is a carboxylic acid protecting group. The deprotection and condensation methods may be the same as outlined in connection with the reaction of compounds (II) to (I) above.

Preferably the precursor according to the general formula (IV) is obtained from precursors according to the general formulas (VI) and (V): 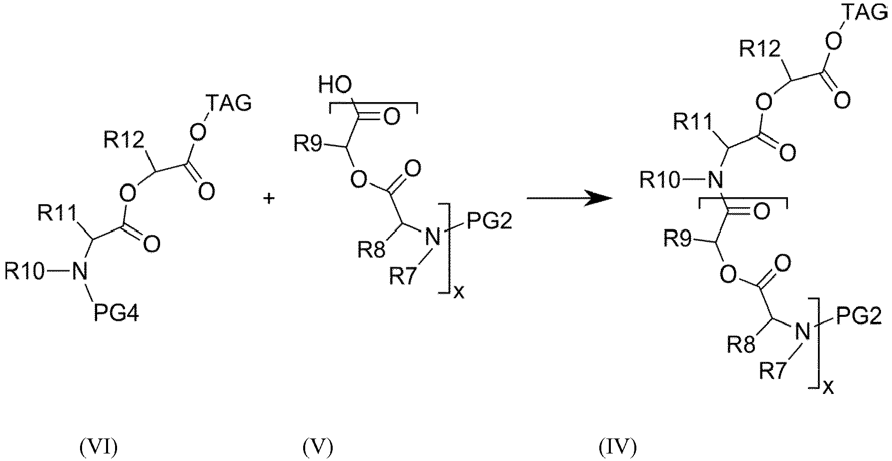 by:
- in precursor (VI), deprotecting the amine group which is protected by the PG4 group in the presence of a base, thereby obtaining a deprotected amine group;
- condensation of the deprotected amine group of precursor (VI) and carboxylic acid group of precursor (V), thereby obtaining the precursor (IV);
wherein R7 to R12, TAG and x have a meaning as defined above (in particular, x may be 1), PG2 has a meaning as defined above and PG4 is an amine protecting group. The deprotection and condensation methods may be the same as outlined in connection with the reaction of compounds (II) to (I) above.

It is also preferred that the precursor according to the general formula (III) is obtained from precursors according to the general formulas (VIII) and (VII): 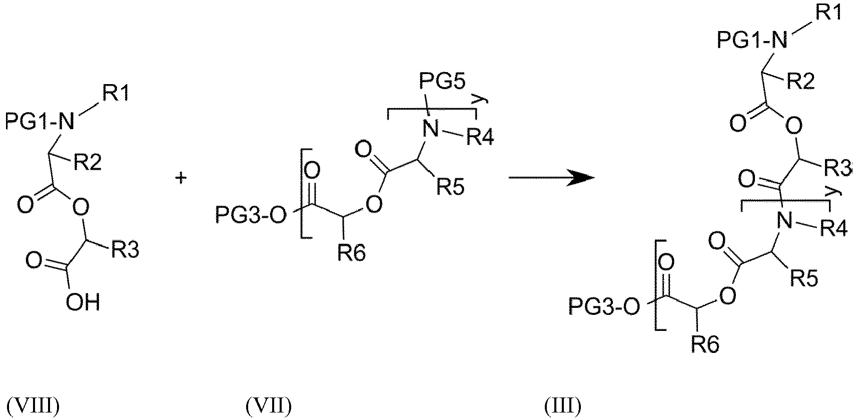 by:
- in precursor (VII), deprotecting the amine group which is protected by the PG5 group in the presence of a base, thereby obtaining a deprotected amine group;
- condensation of the deprotected amine group of precursor (VII) and carboxylic acid group of precursor (VIII), thereby obtaining the precursor (III);
wherein R1 to R6, PG1 and y have a meaning as defined above (in particular, y may be 1), PG3 has a meaning as defined above and PG5 is an amine protecting group. The deprotection and condensation methods may be the same as outlined in connection with the reaction of compounds (II) to (I) above.

It is also preferred that the precursor according to the general formula (VI) is obtained from the esterification of a precursor according to the general formula (IX) with TAG-OH: 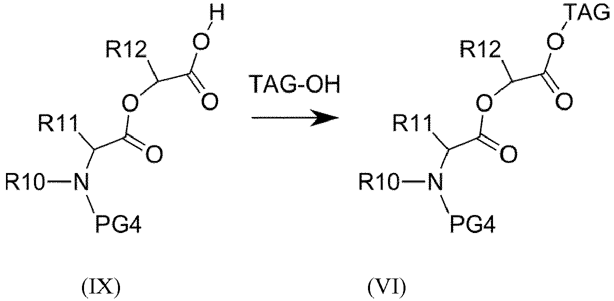 wherein R10 to R12 and TAG have a meaning as defined above and PG4 has a meaning also as defined above. The protection or tagging of the carboxylic acid group in (IX) may be effected using a standard condensation system such as DCC/DMAP.

It is also preferred that the precursor according to the general formula (VII) is obtained from the esterification of a precursor according to the general formula (X) with PG3-OH: 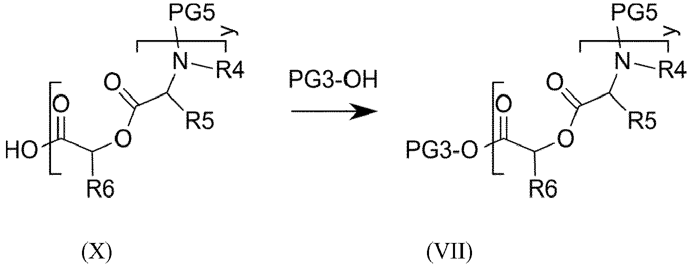 wherein R4 to R6 and y have a meaning as defined above, PG3 also has a meaning as defined above and PG5 also has a meaning as defined above.

It is also preferred that PG3 is TAG as defined above.

In another embodiment of the method according to the invention at least one reaction step of the reaction steps resulting in a TAG-bearing molecule is followed by precipitation of crude reaction product in methanol, thereby purifying the crude reaction product.

In another embodiment of the method according to the invention at least one step of the reaction steps in which TAG-protected carboxylic acid groups are deprotected is followed by precipitation of cleaved TAG-OH in methanol and removal of the precipitate by filtration, thereby purifying the crude reaction product.

It is also preferred that precursors (III) and (IV) are identical.

It is also preferred that R3 and R9 are different from each other, R1 and R7 are identical, R2 and R8 are identical, R4 and R10 are identical, R5 and R11 are identical and R6 and R12 are identical.

In another embodiment of the method according to the invention the depsipeptide (II) is selected from one of the general formulas (II-1) to (II-14): 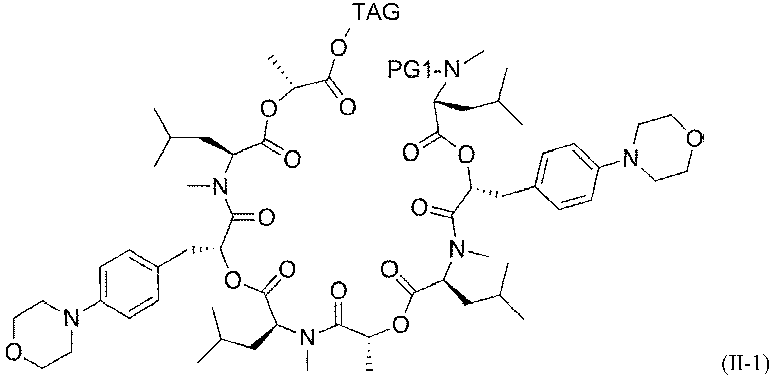 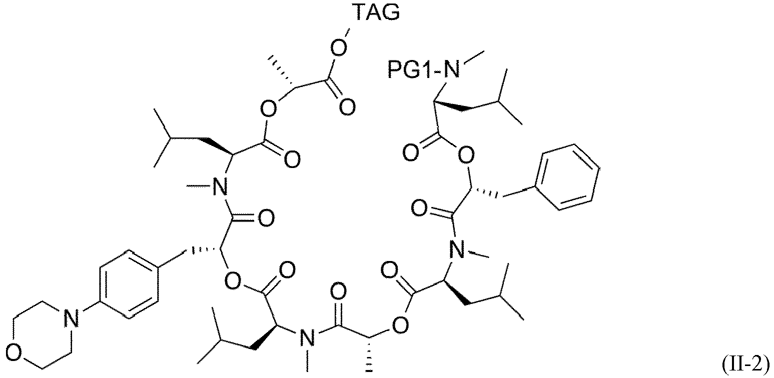 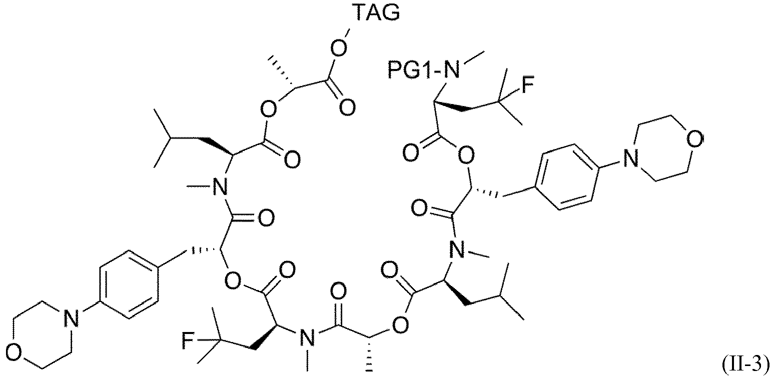 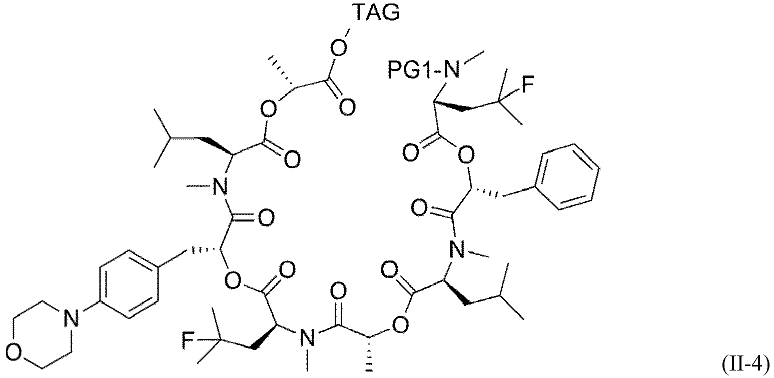 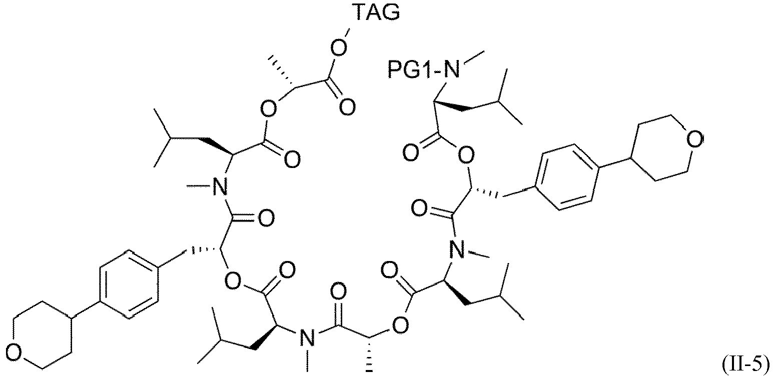 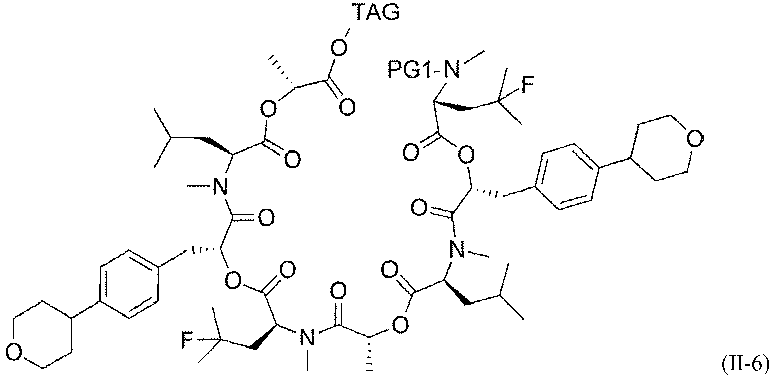 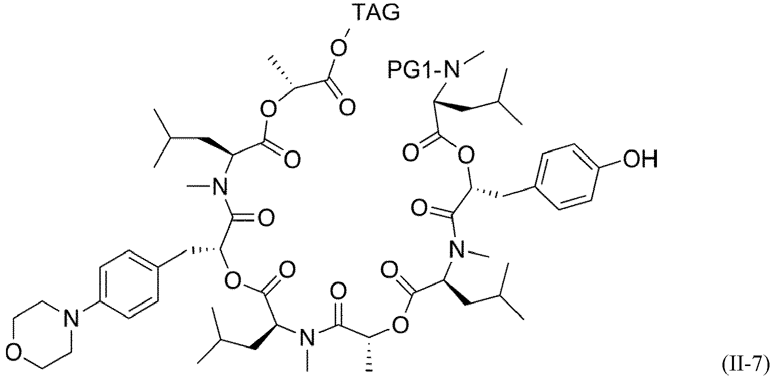 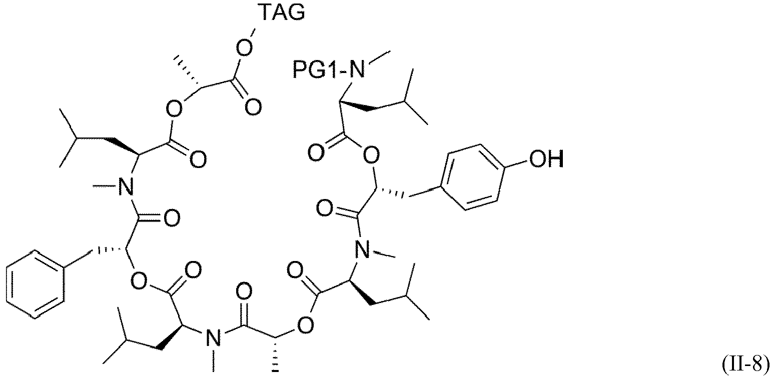 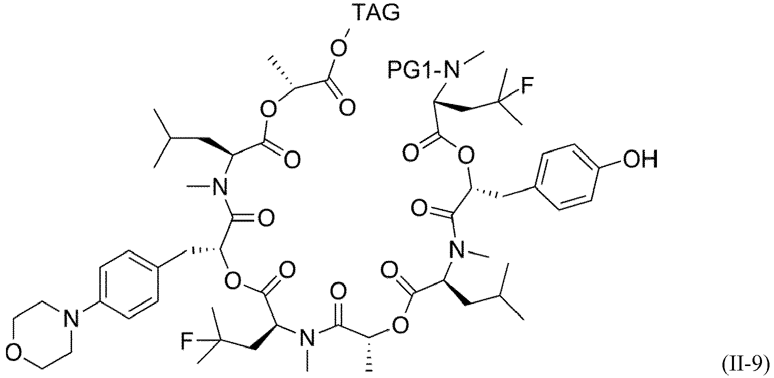 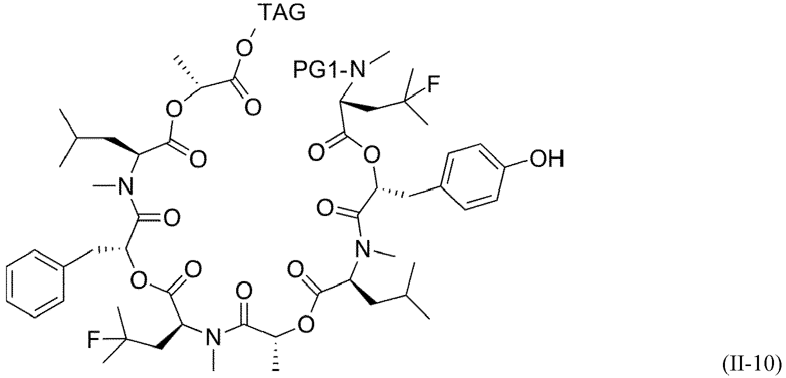 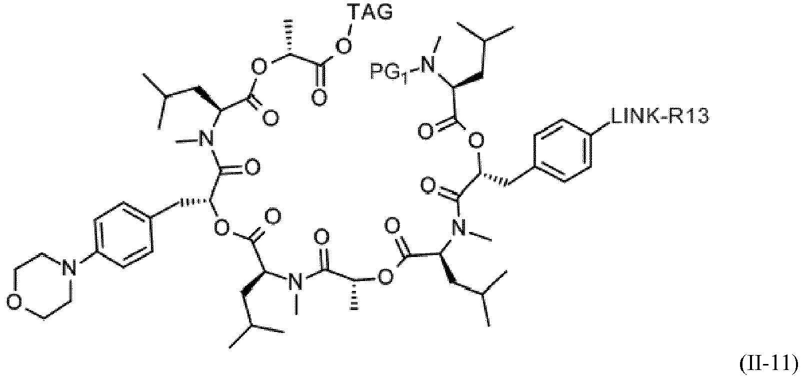 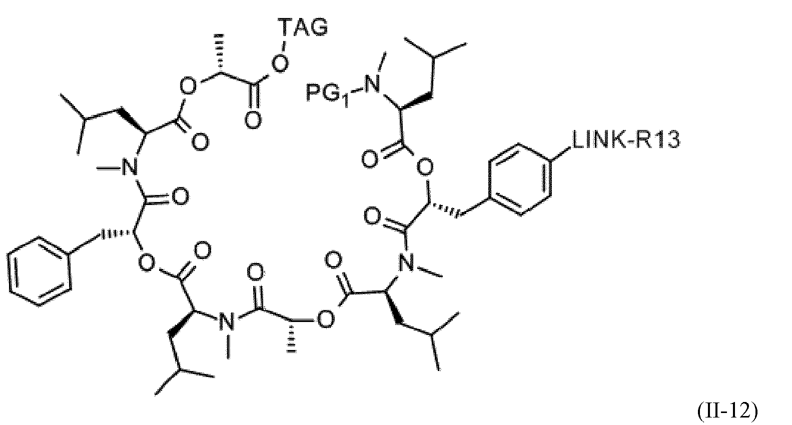 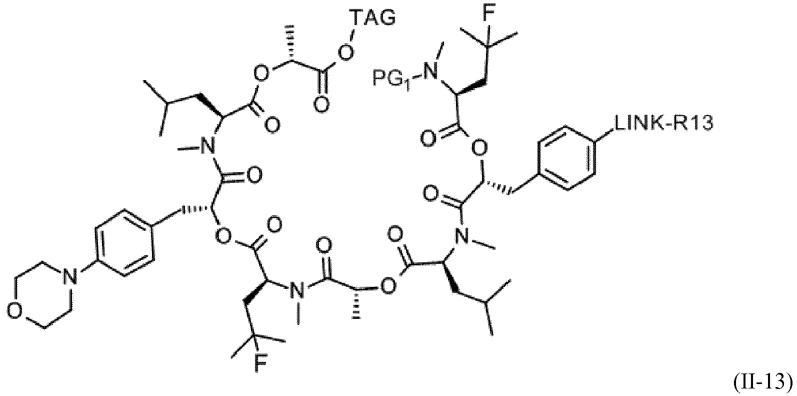 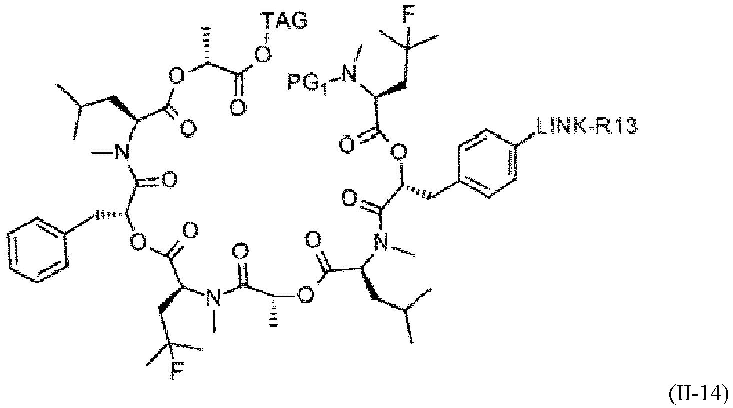 wherein in formulas (II-11) to (II-14) -LINK- is selected from: 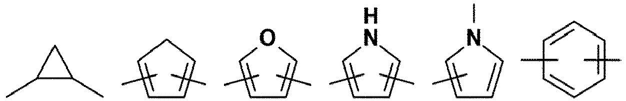 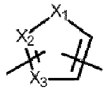 wherein X1 can be C, N, S, O, X2 and X3 can be C or N; 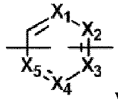 wherein X1 can be C, N, S, O, X2, X3 and X4 can be C or N; 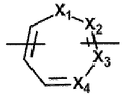 wherein X1, X2, X3 and X4 can be C or N; and wherein R13 is selected from SO₂NH(CH₃), SO₂NH₂, OC(O)CH₃, CF₃ or one the following lactone structures:

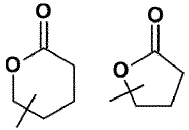

The present invention is also directed towards a linear or cyclic depsipeptide selected from one of the general formulas (II-1) to (II-14) or (I-1) to (1-9) as depicted below or a pharmaceutically or veterinarily acceptable salt thereof: 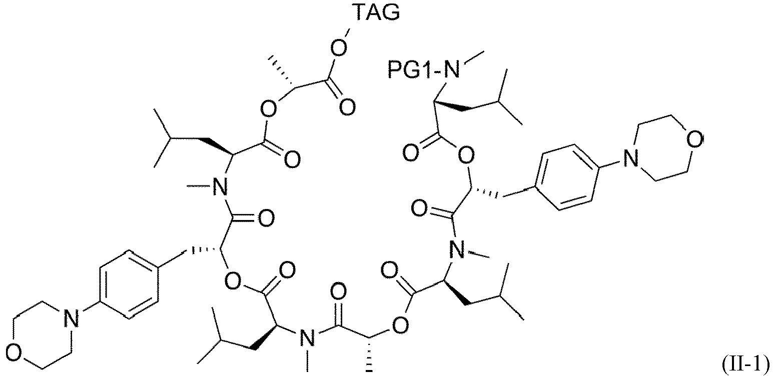 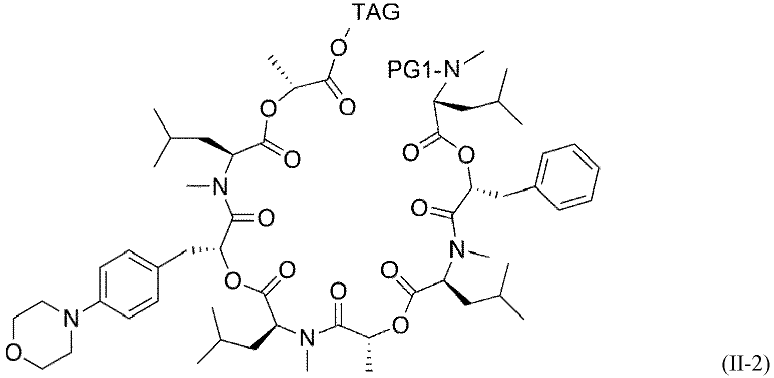 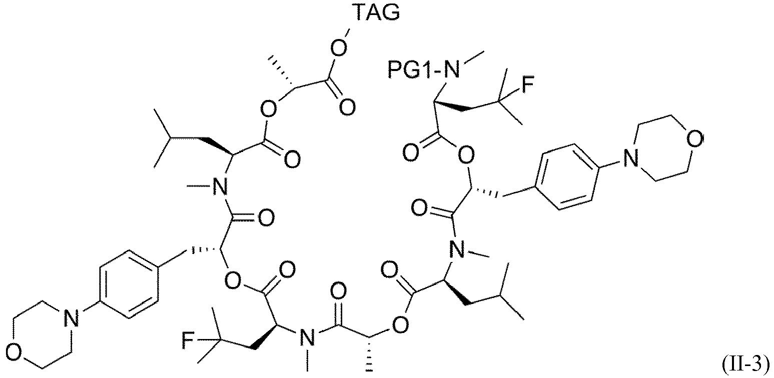 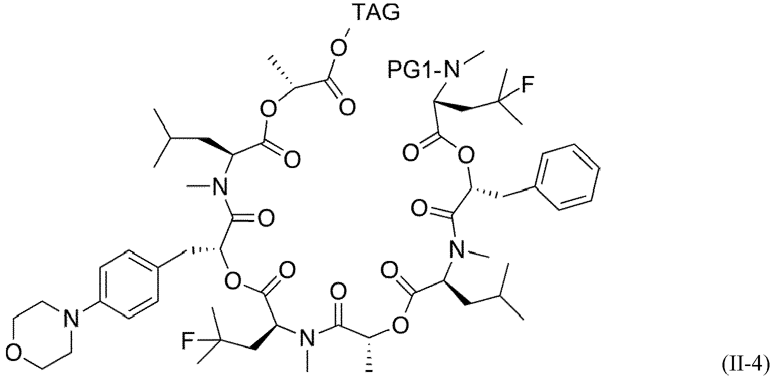 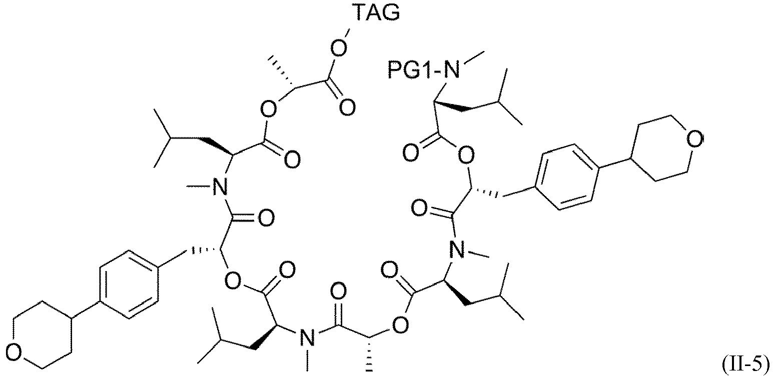 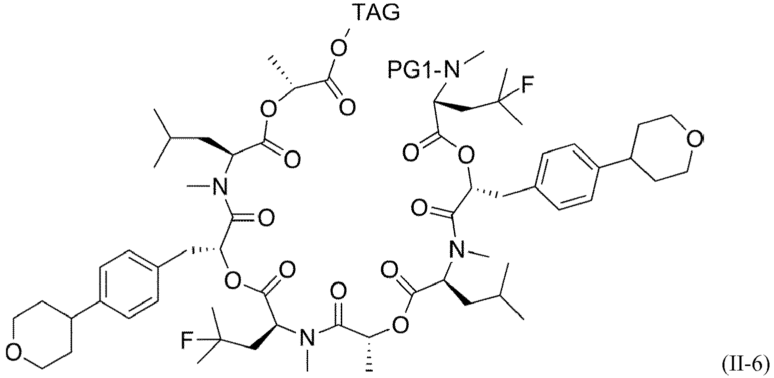 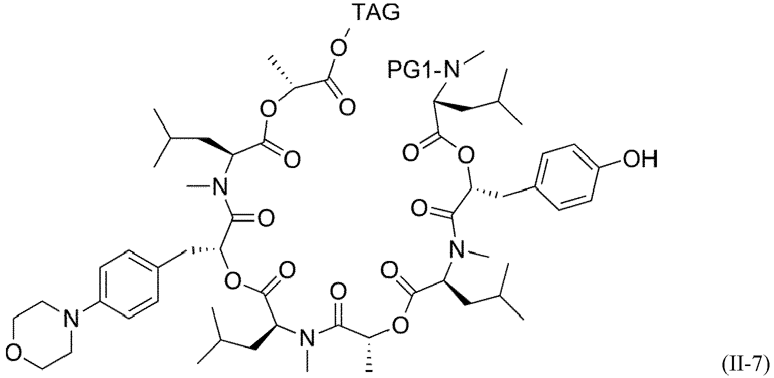 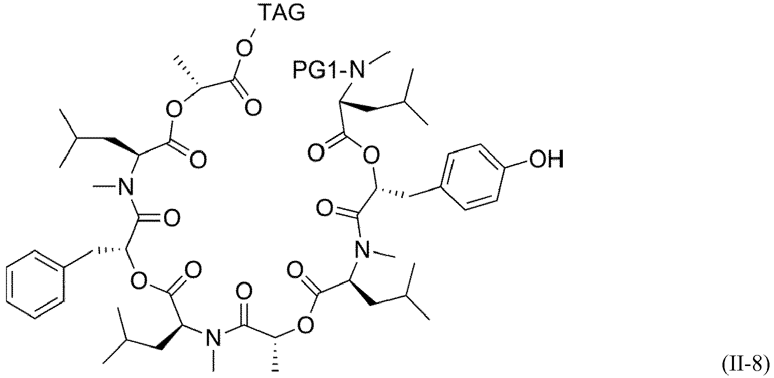 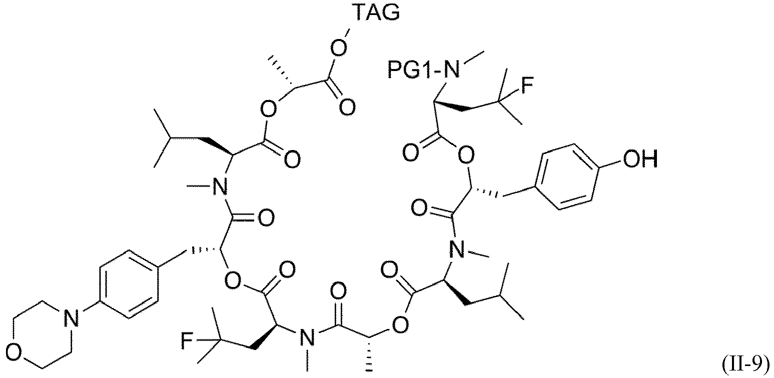 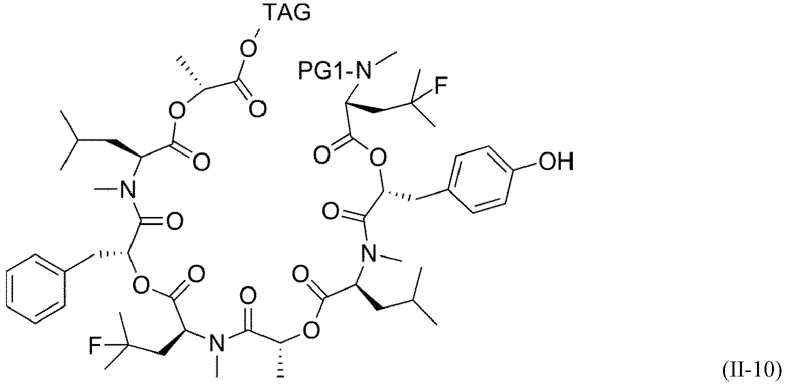 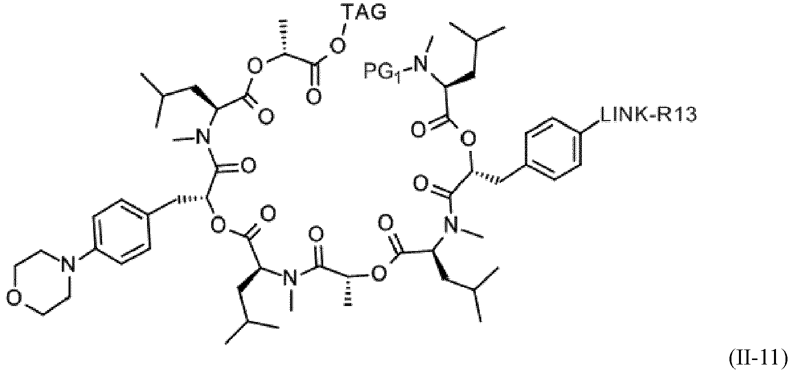 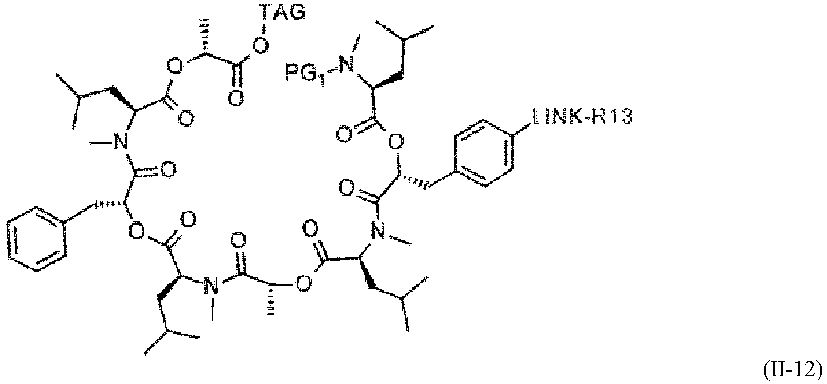 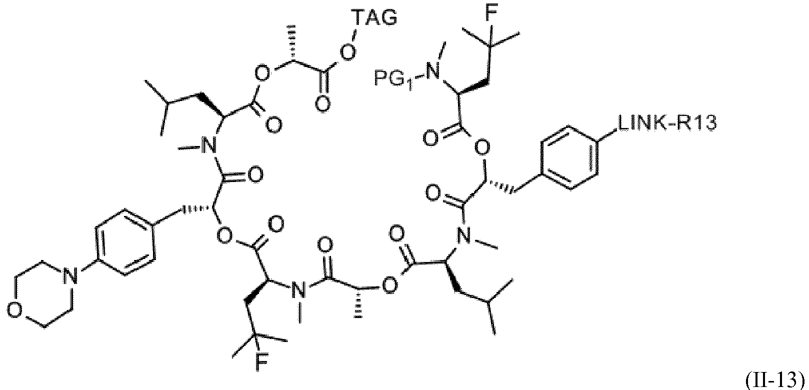 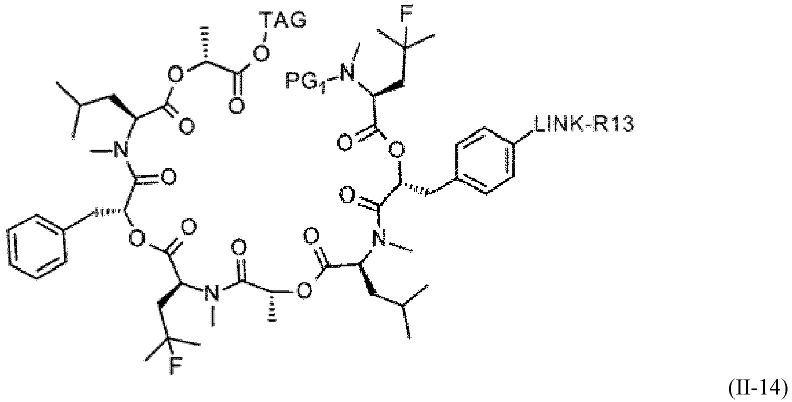 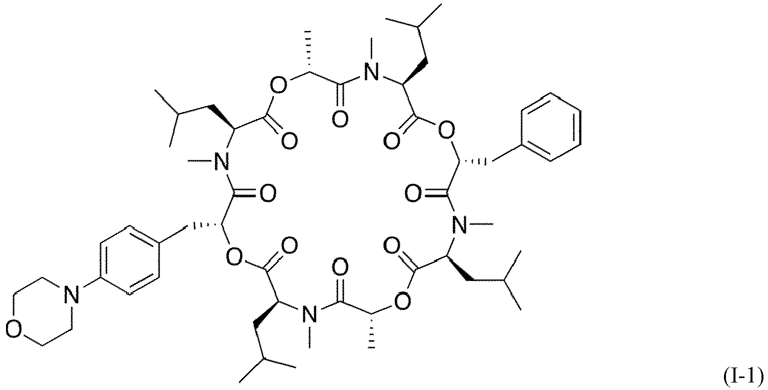 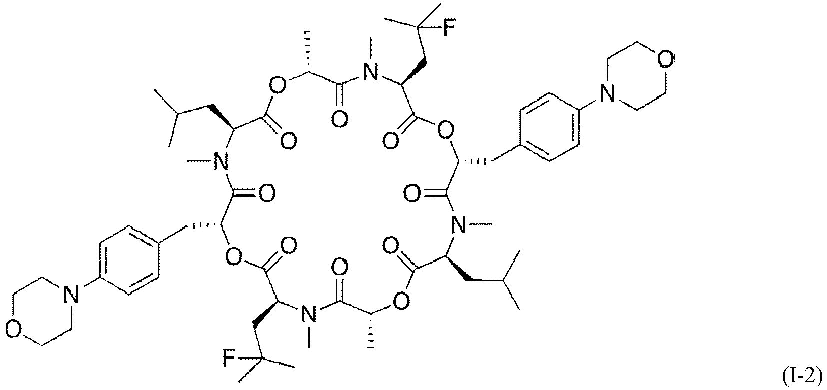 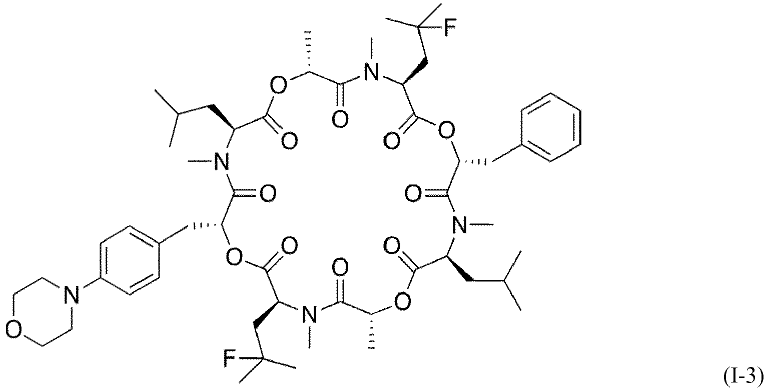 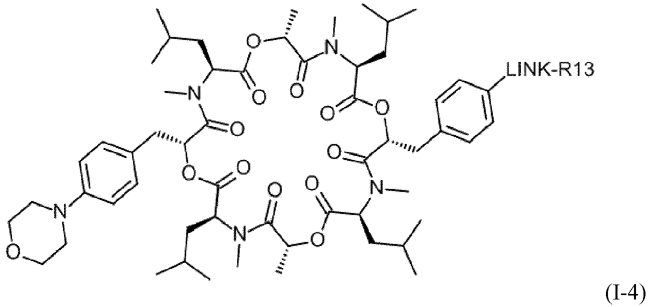 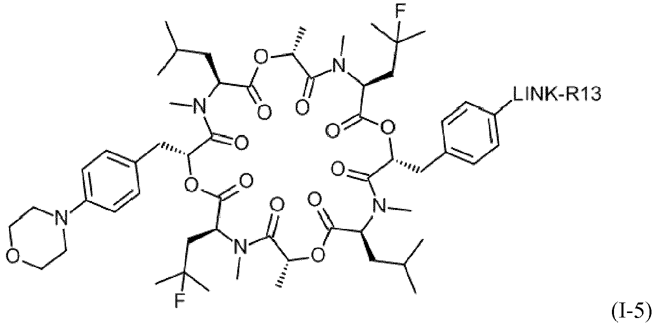 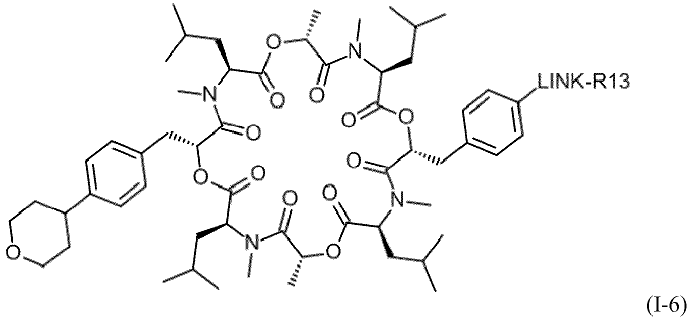 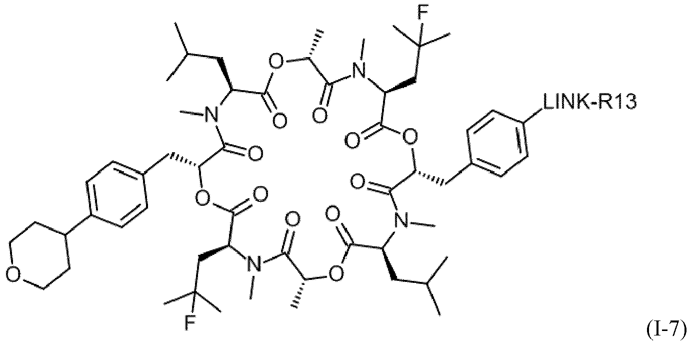 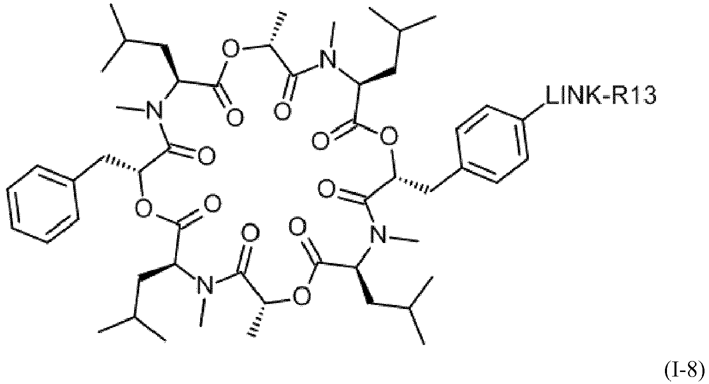 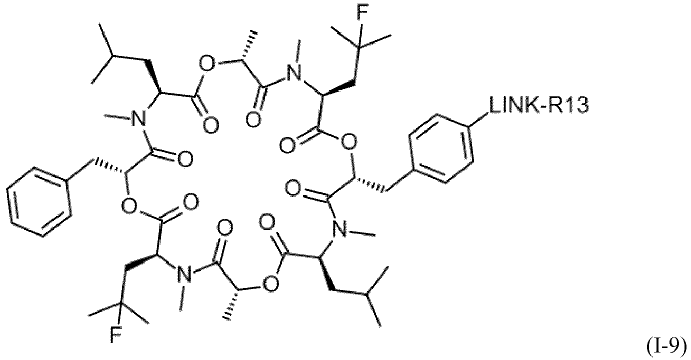 wherein in formulas (II-11) to (II-14) and (I-1) to (1-9), respectively:
PG1 is 9-fluorenylmethoxycarbonyl (Fmoc), t-butyl carbamate (Boc), benzyl carbamate (Z), acetamide, trifluoroacetamide, phthalimide, benzyl (Bn), triphenylmethyl (Tr), benzylidene or p-toluenesulfonamide (Ts),
TAG is: 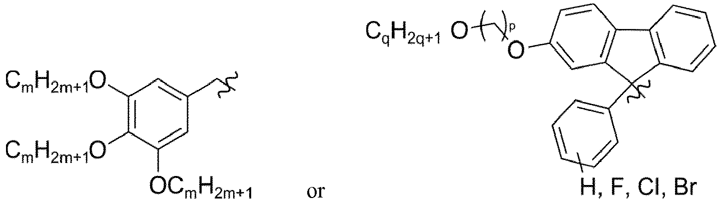 wherein m is ≥ 15 to ≤ 25, p is ≥ 8 to ≤ 18 and q is ≥ 15 to ≤ 25,
-LINK- is selected from: 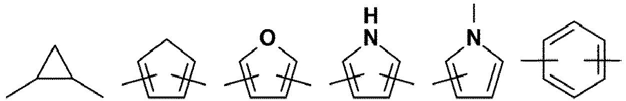
and R13 is selected from SO₂NH(CH₃), SO₂NH₂, OC(O)CH₃, CF₃ or one the following lactone structures:

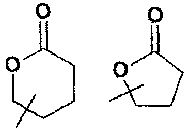

The terms "veterinarily acceptable salt" and "pharmaceutically acceptable salt" are used throughout the specification to describe any salts of the compounds that are acceptable for administration for pharmaceutical or veterinary applications, and which provides the active compound upon administration.

Veterinarily acceptable salts include those derived from veterinarily acceptable inorganic or organic bases and acids. Suitable salts include those comprising alkali metals such as lithium, sodium or potassium, alkaline earth metals such as calcium, magnesium and barium. Salts comprising transition metals including, but not limited to, manganese, copper, zinc and iron are also suitable. In addition, salts comprising ammonium cations as well as substituted ammonium cations, in which one or more of the hydrogen atoms are replaced by alkyl or aryl groups are encompassed by the invention. Salts derived from inorganic acids including, but not limited to, hydrohalide acids (HCl, HBr, HF, HI), sulfuric acid, nitric acid, phosphoric acid, and the like are particularly suitable. Suitable inorganic salts also include, but not limited to, bicarbonate, and carbonate salts. In some embodiments, examples of veterinarily and agriculturally acceptable salts are organic acid addition salts formed with organic acids including, but not limited to, maleate, dimaleate, fumarate, tosylate, methanesulfonate, acetate, citrate, malonate, tartarate, succinate, benzoate, ascorbate, α-ketoglutarate, and α-glycerophosphate. Of course, other acceptable organic acids may be used.

Alkali metal (for example, sodium, potassium or lithium) or alkaline earth metal (for example calcium) salts of the compounds can also be made by reacting a sufficiently acidic residue on the compounds with a hydroxide of the alkali metal or alkaline earth metal.

Veterinarily acceptable salts may be obtained using standard procedures well known in the art, for example by reacting a sufficiently basic compound such as an amine with a suitably acid functional group present in the compound, or by reacting a suitable acid with a suitably basic functional group on the compounds of the invention.

### Examples

The present invention will be further described with reference to the following examples without wishing to be limited by them.

### 1. General method

Benzyl (*R*)-2-Hydroxy-3-(4-morpholinophenyl)propionate was prepared by standard literature procedure *(*Eur. J. Org. Chem, 2012, 1546-1553) from (*R*)-2-hydroxy-3-(4-morpholinophenyl)propionic acid, which was supplied from Bayer Animal Health. The starting building blocks were prepared from commercial available reagents, benzyl (*S*)-lactate, benzyl (*R*)-2-hydroxy-3-phenylpropionate, and N-Fmoc-*N*-methyl-L-leucine by standard literature procedures *(*Eur. J. Org. Chem, 2012, 1546-1553). 3,4,5-Tris(octadecyloxy)benzyl alcohol (HO-TAGa) was prepared by the literature procedure *(*Tetrahedron, 2011, 67, 6633-6643) from commercial available methyl gullate. Unless otherwise noted, reagents and solvents were purchased at highest commercial quality and used without further purification. Methanol and dry toluene, CH₂Cl₂ were purchased from Kanto Chemical Co., Inc. All reactions were monitored by thin-layer chromatography (TLC) using Merck silica gel 60 F254 precoated plates (0.25 mm). Flash chromatography was carried out with Kanto Chemical silica gel (Kanto Chemical, silica gel 60N, spherical neutral, 0.040-0.050 mm, Cat.-No. 37563-84). ¹H and ¹³C NMR spectra were recorded on JEOL JNM-ECA-500 (500 MHz for ¹H-NMR and 125 MHz for ¹³C-NMR). Chemical shifts are expressed in ppm downfield from the internal solvent peaks for CDCl₃ (¹H; δ = 7.26 ppm, ¹³C; δ = 77.0 ppm), CD₃OD (¹H; δ = 3.31 ppm, ¹³C; δ = 49.0 ppm) and *J* values are given in Hertz. The following abbreviations were used to explain the multiplicities: s = singlet, d = doublet, t = triplet, q = quartet, dd = double doublet, ddd = double double doublet, dt = double triplet, dq = double quartet, m = multiplet, br = broad. All infrared spectra were measured on a Horiba FT-210 spectrometer. High- and Low-resolution mass spectra were measured on a JEOL JMS-AX505 HA, JEOL JMS-700 MStation and JEOL JMS-T100LP. Optical rotations were measured by using JASCO P-1010 polarimeter. Melting points were measured on a YANACO MP-500P or OptiMelt (Stanford Research Systems) apparatus.

### General method for Fmoc deprotection

Fmoc protected substrate was dissolved into 5% piperidine/CH₂Cl₂ (generally 0.05 M for substrate) at room temperature and solution was stirred for 3 h. The reaction mixture was subsequently cooled to -5 °C and MeOH was added (five times excess of reaction solution). The resulting heterogeneous solution was stirred for further 30 min at -5 °C, and the colorless precipitate was filtered and washed with additional MeOH to afford corresponding amine as a colorless powder.

### General method for the cleavage of TAGa function

Tagged substrates with TAGa dissolved into 50% TFA/CH2Cl2 (0.05 M for substrate) at room temperature and solution was stirred for generally 1 h. The reaction mixture was subsequently concentrated with toluene (x 3) to remove TFA. To a flask was then added CH2Cl2 at -5 oC, followed MeOH was added (five times excess of CH2Cl2). The resulting heterogeneous solution was stirred for further 30 min at -5 oC, and the colorless precipitate was filtered off and washed with additional MeOH. The combined filtrates were concentrated in vacuo. To the resulting product was added 4 M HCl/Dioxane (0.05 M for product) and concentrated with toluene (x 3) to afford corresponding carboxylic acid as a generally brown oil. The crude was used next reaction without further purification.

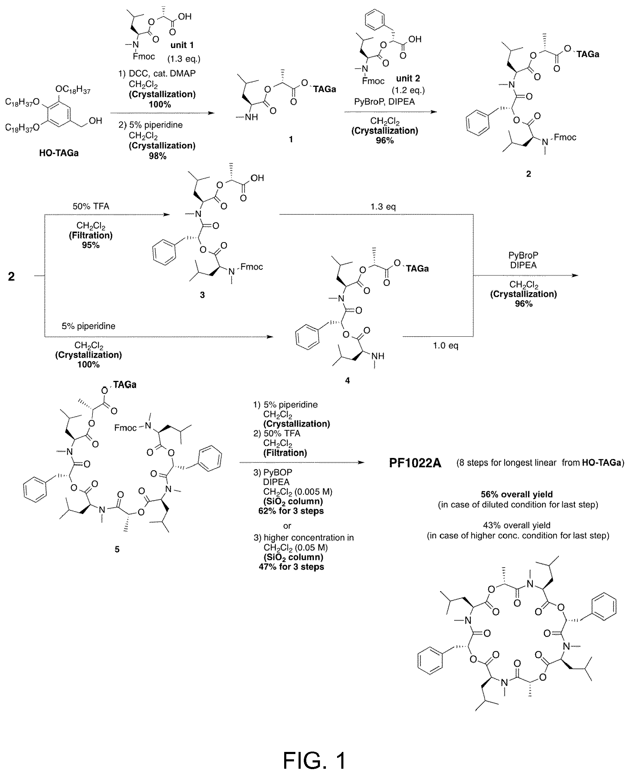

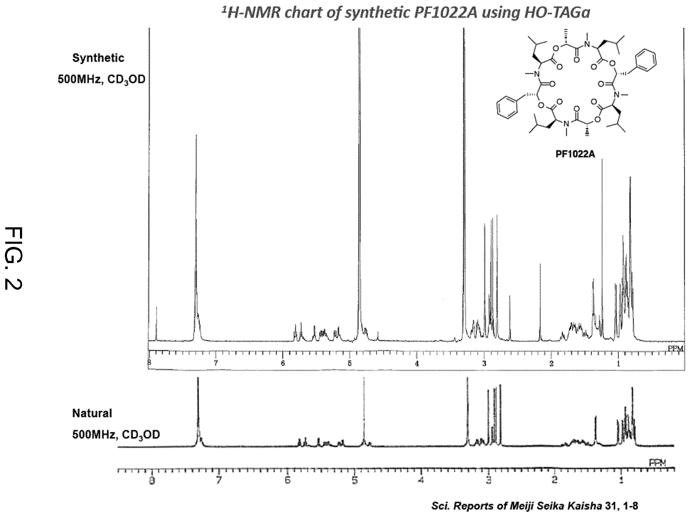

### 2. Preparation of PF1022A (Method 1; cf. reaction scheme in FIG. 1 and NMR spectra in FIG. 2)

### 2-1. Synthetic procedure

### N-Fmoc-N-MeLeu-D-Lac-O-TAGa

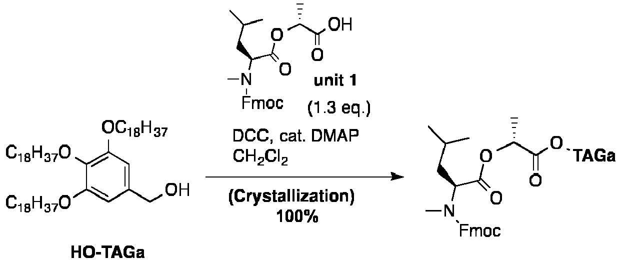

To a stirred solution of **HO-TAGa** (1.69 g, 1.85 mmol) in CH₂Cl₂ (37 mL) was added 0.2 M toluene solution of **unit 1** (12.0 mL, 2.40 mmol), 4-dimethylaminopyridine (12 mg, 93.0 µmol), and *N*,*N'-*dicyclohexylcarbodiimide (1.30 g, 2.78 mmol) at room temperature under N₂ atmosphere. After stirring for 2 h, the reaction mixture was then cooled to -5 °C, and MeOH (185 mL) was added. The resulting heterogeneous solution was stirred for further 15 min at -5 °C, and the colorless precipitate was filtered and washed with additional MeOH (500 mL) to afford ***N*-Fmoc-*N*-MeLeu-D-Lac-O-TAGa** (2.46 g, 100%) as a colorless powder.
mp: 44 - 45 °C
[α]_{D}²⁴: -10.2 (c 1.0, CHCl₃)
¹H-NMR (500 MHz, CDCl₃) δ : 7.78-7.74 (complex m, 2H), 7.60-7.56 (complex m, 2H), 7.39 (m, 2H), 7.29 (m, 2H), 6.50 (s, 4/3H), 6.48 (s, 2/3H), 5.12-5.00 (complex-m, 4H), 4.67 (dd, *J* = 6.3, 9.7 Hz, 3/10H), 4.58 (dd, *J* = 6.3 Hz, 10.9 Hz, 4/10H), 4.50 (dd, *J* = 6.9 Hz, 10.3 Hz, 6/10H), 4.38-4.34 (complex m, 9/10H), 4.30 (m, 5/10H), 4.23 (t, *J* = 6.3 Hz, 3/10H), 3.93 (m, 6H), 2.86 (s, 2H), 2.83 (s 1H), 1.76 (m, 6H), 1.64-1.42 (complex m, 9H), 1.31-1.14 (complex m, 87H), 0.96-0.87 (complex m, 14H), 0.78 (d, *J* = 6.9 Hz, 1H).
HRMS (FAB, NBA matrix) *m*/*z* : 1334.0748 (M⁺, calcd for C₈₆H₁₄₃NO₉: 1334.0763)

### N-MeLeu-D-Lac-O-TAGa (1)

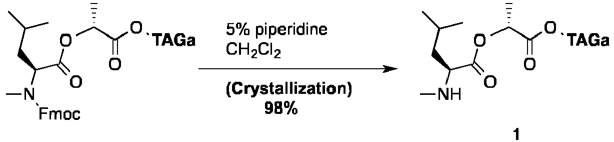

Following the procedure described for general procedure of Fmoc deprotection, ***N*-Fmoc-*N*-MeLeu-D-Lac-O-TAGa** (1.00 g, 0.749 mmol) was converted to **1** (816 mg, 98%) as a colorless powder.
¹H-NMR (500 MHz, CDCl₃) δ : 6.50 (s, 2H), 5.16 (q, *J* = 6.9 Hz, 1H), 5.08 (d, *J* = 12.0 Hz, 1H), 5.04 (d, *J* = 12.0 Hz, 1H), 3.93 (m, 6H), 3.30 (t, *J* = 7.5 Hz, 1H), 2.37 (s, 3H), 1.75 (m, 6H), 1.53-1.42 (complex m, 10H), 1.32-1.25 (complex m, 86H), 0.93-0.86 (complex m, 15H).
¹³C-NMR (125 MHz, CDCl₃) δ : 170.6, 153.3, 138.4, 130.2, 106.4, 73.5, 69.2, 68.9, 67.6, 61.4, 42.2, 34.5, 32.0, 30.4, 29.8 (x2), 29.5 (x2), 26.2, 25.0, 22.8, 22.6, 22.5, 17.1, 14.2.
HRMS (FAB, NBA matrix) *m*/*z* : 1113.0151 [(M+H)⁺, calcd for C₇₁H₁₃₄NO₇: 1113.0160]

### N-Fmoc-N-MeLeu-D-PhLac-N-MeLeu-D-LacO-TAGa (2)

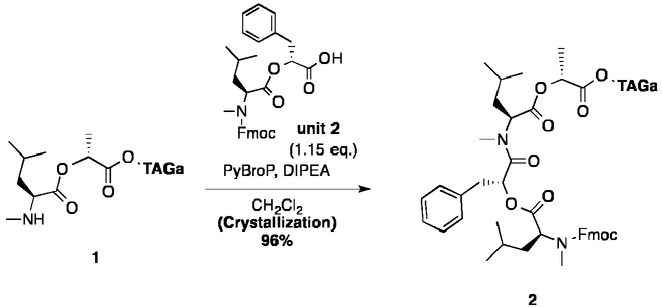

To a stirred solution of **1** (800 mg, 0.719 mmol) in CH₂Cl₂ (25 mL) was added **unit 2** (426 mg, 0.827 mmol), *N*,*N*-diisopropylethylamine (0.429 mL, 2.52 mmol), and PyBroP (496 mg, 1.222 mmol) at room temperature. After stirring for 88 h, the reaction mixture was crystallized by the procedure described in synthesis of ***N*-Fmoc-*N*-MeLeu-D-Lac-O-TAGa** to afford **2** (1.11 g, 96%) as a colorless powder.
¹H-NMR (500 MHz, CDCl₃) δ : 7.76-7.72 (complex m, 2H), 7.62-7.40 (complex m, 2H), 7.39-7.17 (complex m, 9H), 6.47 (m, 2H), 5.47-5.27 (complex m, 2H), 5.15-4.97 (complex m, 4H), 4.69-4.13 (complex m, 3H), 3.92 (complex m, 6H), 3.07 (m, 2H), 2.85 (complex, 6H), 1.80-1.25 (complex m, 105H), 1.02-0.72 (complex m, 21H).
HRMS (FAB, NBA matrix) *m*/*z* : 1632.2163 [(M+Na)⁺, calcd for C₁₀₂H₁₆₄N₂O₁₂Na: 1632.2182]

### N-MeLeu-D-PhLac-N-MeLeu-D-Lac-OH (3)

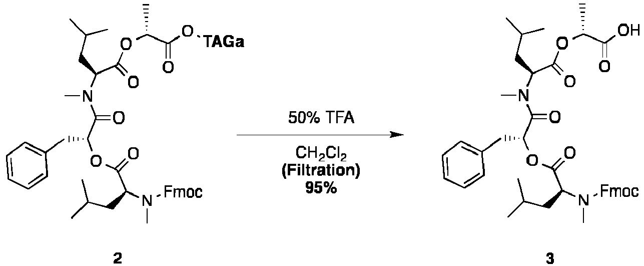

Following the procedure described for general procedure of TAGa cleavage, **2** (614 mg, 0.381 mmol) was converted to **3** (261 mg, 95%) as an yellow oil, which was used next reaction without further purification.

### N-MeLeu-D-PhLac-N-MeLeu-D-Lac-O-TAGa (4)

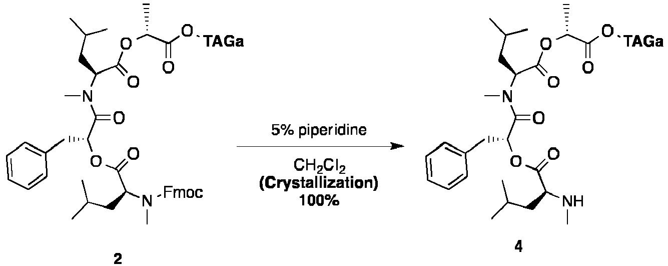

Following the procedure described for general procedure of Fmoc deprotection, **2** (472 mg, 0.293 mmol) was converted to **4** (404 mg, 100%) as a colorless powder.
¹H-NMR (500 MHz, CDCl₃) δ : 7.27 (m, 5H), 6.49 (s, 2H), 5.50 (dd, *J* = 5.7, 8.6 Hz, 1H), 5.32 (dd, *J =* 4.6, 10.9 Hz, 1H), 5.09-5.00 (complex m, 3H), 3.93 (m, 6H), 3.28 (t, *J* = 6.9 Hz, 1H), 3.09 (m, 2H), 2.92 (s, 3H), 2.27 (s, 3H), 1.82-1.25 (complex m, 105H), 0.89-0.77 (complex m, 21H).
¹³C-NMR (125 MHz, CDCl₃) δ : 175.6, 175.1, 175.0, 174.8, 170.9, 170.5 (x2), 170.4, 169.8, 169.7, 166.7, 165.1, 153.3, 138.5, 138.3, 135.8 (x2), 130.2, 129.5, 129.3, 128.7, 128.6, 128.5 (x2), 127.2, 107.0, 106.9, 105.4, 78.1, 73.5, 71.9, 71.6, 69.4, 69.2, 68.9, 67.7, 67.5, 66.9, 65.6, 61.3, 61.2, 59.7, 57.7, 54.7, 42.3, 42.2, 42.1, 40.0, 38.8, 37.5, 36.9, 34.4, 34.3, 32.8, 32.0, 31.3, 30.4, 29.8 (x2), 29.6, 29.5 (x2), 26.2, 25.0, 24.8, 24.7, 24.6, 23.4, 22.9, 22.8, 22.5 (x2), 22.4 (x2), 21.9, 21.4, 20.5, 17.0, 16.9, 16.8, 14.2.
HRMS (FAB, NBA matrix) *m*/*z* : 1388.1664 [(M+H)⁺, calcd for C₈₇H₁₅₅N₂O₁₀: 1388.1682]

### N-Fmoc-N-MeLeu-D-PhLac-N-MeLeu-D-Lac-N-MeLeu-D-PhLac-N-MeLeu-D-Lac-O-TAGa (5)

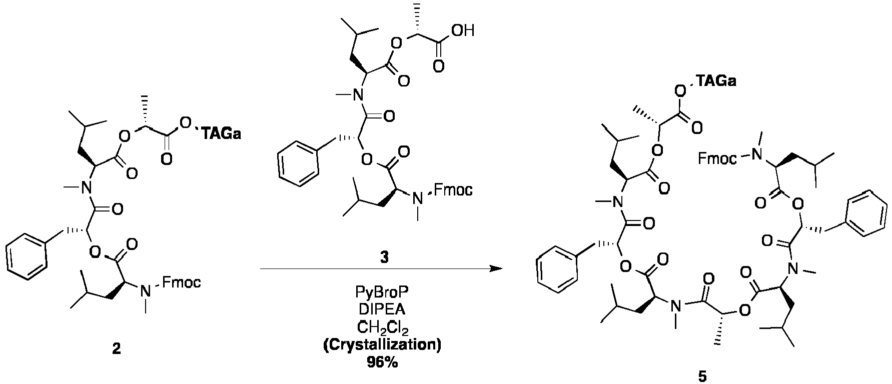

To a stirred solution of **2** (330 mg, 0.238 mmol) in CH₂Cl₂ (4.8 mL) was added 3 (221 mg, 0.309 mmol), *N*,*N*-diisopropylethylamine (0.150 mL, 0.881 mmol), and PyBroP (197 mg, 0.428 mmol) at room temperature. After stirring for 42 h, the reaction mixture was crystallized by the procedure described in synthesis of ***N*-Fmoc-*N*-MeLeu-D-Lac-O-TAGa** to afford **5** (477 mg, 96%) as a colorless powder.
¹H-NMR (500 MHz, CDCl₃) δ : 7.74 (m, 2H), 7.53 (m, 2H), 7.38 (m, 2H), 7.32-7.15 (complex m, 12H), 6.48 (m, 2H), 5.51-4.96 (complex m, 10H), 4.70-4.12 (complex m, 3H), 3.93 (m, 6H), 3.23 (dd, *J* = 6.9, 13.7 Hz, 1H), 3.08-2.70 (complex m, 15H), 1.80-1.25 (complex m, 114H), 0.95-0.78 (complex m, 33H).
HRMS (FAB, NBA matrix) *m*/*z* : 2106.4949 [(M+Na)⁺, calcd for C₁₂₈H₂₀₂N₄O₁₈Na₁: 2106.4912]

### N-MeLeu-D-PhLac-N-MeLeu-D-Lac-N-MeLeu-D-PhLac-N-MeLeu-D-Lac-O-TAGa

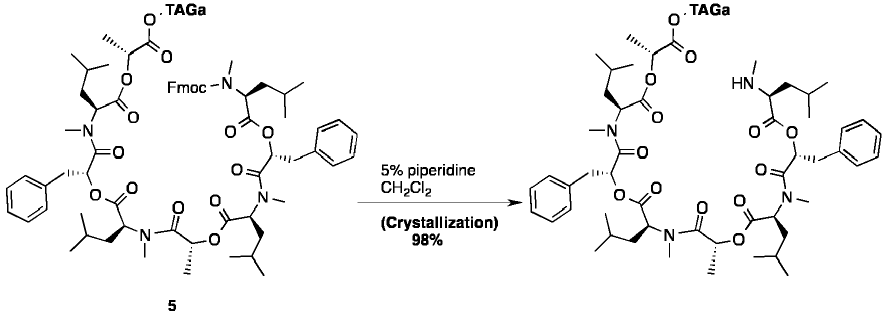

Following the procedure described for general procedure of Fmoc deprotection, **5** (450 mg, 0.206 mmol) was converted to the corresponding amine (398 mg, 98%) as a colorless powder.
¹H-NMR (500 MHz, CDCl₃) δ : 7.25 (m, 10H), 6.48 (s, 2H), 5.57-4.99 (complex m, 9H), 3.93 (m, 6H), 3.26-2.78 (complex m, 14H), 2.24 (s, 3H), 1.80-1.25 (complex m, 114H), 0.90-0.78 (complex m, 33H).
¹³C-NMR (125 MHz, CDCl₃) δ : 175.6, 175.2 (x2), 171.5, 171.1, 170.8 (x2), 170.6, 170.5 (x2), 170.3, 153.3, 138.5 (x2), 138.3, 136.1, 135.9, 135.5, 130.2, 130.1, 130.0, 129.8, 129.7, 129.6, 129.4 (x2), 128.8, 128.7, 128.6, 128.5, 127.4 (x2), 127.2, 127.1, 126.9, 126.8, 107.0 (x2), 106.9, 73.5, 72.5, 72.2, 71.7, 71.6, 69.8, 69.4, 69.2, 68.1, 68.0, 67.8, 67.5 (x2), 66.9, 61.4, 57.4, 55.2, 54.8, 54.6 (x2), 38.8, 37.7, 37.6, 37.4 (x2), 37.1 (x2), 37.0, 36.9, 34.7, 34.6, 32.2, 32.0, 31.9, 31.7, 31.6, 31.4, 31.3, 31.2, 30.4, 29.8 (x2), 29.6, 29.5 (x2), 26.2, 24.9, 24.8 (x2), 24.7, 24.6, 24.5, 23.5, 23.4, 23.3, 23.1 (x2), 23.0, 22.8, 22.7, 22.5, 22.4, 22.3, 22.0, 21.5, 21.4, 21.3 (x2), 20.5, 16.9, 16.8, 16.6, 14.2.
HRMS (FAB, NBA matrix) *m*/*z* : 1862.4425 [(M+H)⁺, calcd for C₁₁₃H₁₉₃N₄O₁₆: 1862.4412]

### N-MeLeu-D-PhLac-N-MeLeu-D-Lac-N-MeLeu-D-PhLac-N-MeLeu-D-Lac-OH

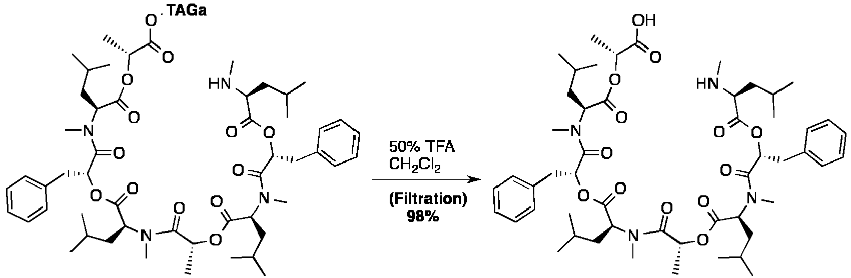

Following the procedure described for general procedure of TAGa cleavage, ***N*-MeLeu-D-PhLac-*N-*MeLeu-D-Lac-*N*-MeLeu-D-PhLac-*N*-MeLeu-D-LacO-TAGa** (380 mg, 0.204 mmol) was converted to the corresponding carboxylic acid (198 mg, 98%) as a yellow oil, which was used next reaction without further purification.

### PF1022A

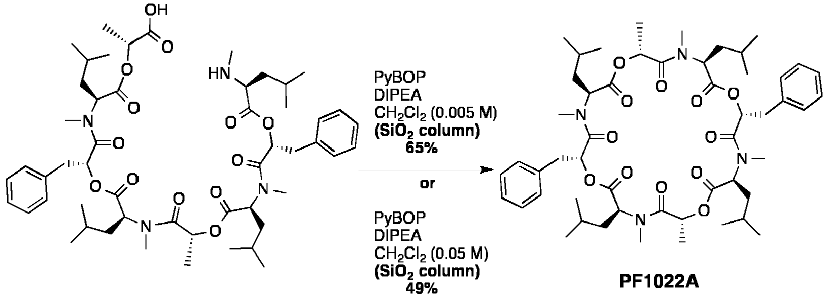

### Reaction for 0.005M concentration of substrate

A crude of previous reaction (90 mg, 0.0895 mmol) was dissolved in CH₂Cl₂ (18 mL, 0.005 M). The reaction mixture was added *N*,*N*-diisopropylethylamine (76 *µL*, 4.48 mmol) and PyBOP (93 mg, 0.179 mmol) at room temperature. After being stirred for 48 h, the reaction mixture was quenched with saturated aqueous NaHCO₃ (18 mL) at 0 °C and this mixture was extracted with CHCl₃ (20 mL x 2). The combined organic layers were washed with 10% aqueous NaHSO₄ (60 mL) and brine (60 mL), dried over Na₂SO₄, filtered and concentrated *in vacuo.* The crude was purified by column chromatography on silica gel (CHCl₃: MeOH = 400 : 1 to 40 : 1) to provide **PF1022A** (55 mg, 65%) as a colorless solid.

### Reaction for 0.05M concentration of substrate

According to the procedure for cyclization mentioned above, a crude of previous reaction (90 mg, 0.0895 mmol) was cyclized in CH₂Cl₂ (1.8 ml, 0.05 M) with *N*,*N*-diisopropylethylamine (76 µL, 4.48 mmol) and PyBOP (93 mg, 0.179 mmol) at room temperature for 48 h to provide **PF1022A** (40 mg, 49%) as a colorless solid.
mp: 100-103 °C
[α]²²D : -99.4 °C (c 0.06, MeOH)
IR (KBr) ṽ: 1743, 1666, 1466, 1412, 1265, 1188, 1126, 1080, 1026, 748, 701
¹H-NMR (500 MHz, CD₃OD) δ : 7.29 (m, 10H), 5.82 (t, *J* = 7.5 Hz, 1H), 5.72 (m, 2H, rotamer), 5.54 (q, *J* = 6.9 Hz, 1H), 5.44 (dd, *J* = 4.6, 11.7 Hz, 1H), 5.40 (dd, *J* = 4.6, 11.7 Hz, 1H, rotamer), 5.23 (dd, *J* = 4.6, 11.7 Hz, 1H), 5.18 (q, *J* = 6.9 Hz, 1H, rotamer), 4.77 (dd, *J* = 3.4, 11.2 Hz, 1H), 3.14 (m, 4H), 3.00 (s, 5/2H, rotamer), 2.91 (m, 7H, rotamer), 2.82 (s, 5/2H, rotamer), 1.84 (m, 1H), 1.77-1.47 (complex m, 11H), 1.39 (d, *J* = 6.3 Hz, 3H), 1.05 (d, *J* = 6.3 Hz, 3H), 0.98 (d, *J* = 6.9 Hz, 3H), 0.80 (d, *J* = 6.3 Hz, 3H), 0.95-0.82 (complex m, 18H).
¹³C-NMR (125 MHz, CD₃OD) δ : 174.4, 173.5, 173.1, 173.1, 172.3, 172.0, 171.0, 170.7, 136.4, 136.2, 130.8, 130.7, 130.7, 129.8, 129.7, 129.7, 128.4, 128.3, 72.5, 72.3, 69.9, 68.5, 58.6, 55.7, 55.5, 55.4, 39.0, 38.9, 38.6, 38.6, 37.8, 37.3, 32.0, 31.3, 31.1, 30.0, 26.2, 26.1, 25.5, 25.2, 23.9, 23.7, 23.7, 23.6, 21.7, 21.6, 21.4, 21.1, 17.5, 17.2.
HRMS (FAB, NBA matrix) *m*/*z* : 971.5353 [(M+Na)⁺, calcd for C₅₂H₇₆N₄O₁₂Na₁ : 971.5357]
*Literature (J. Antibiot., 1992, 45, 692-697)
mp: 104-106 °C
[α]²²_{D} : -102 °C (*c* 0.1, MeOH)
¹H-NMR (400 MHz, CD₃OD) δ : 7.30 - 7.20 (PhH, 10H), 5.80 and 5.75 (C_{α}H-PhLac, 1Hx2), 5.54 and 5.16 (C_{α}H-Lac, 1Hx2), 5.43, 5.42, 5.22 and 4.78 (C_{α}H-Leu, 1Hx4), 3.22-3.15 (C_{β}H₂-PhLac, 2Hx2), 3.00, 2.90, 2.88 and 2.80 (N-Me-Leu, 3Hx4), 1.87-1.50 (C_{β}H₂-Leu, 2Hx4), 1.40 (C_{γ}H-Leu, 1Hx4), 1.38 (C_{β}H₃-Lac, 3H), 1.02-0.75 (C_{δ}H₃-Leu, 6Hx4), 0.88 (C_{β}H₃-Lac, 3H).
¹³C-NMR (100 MHz, CD₃OD) δ : 174.4, 173.4, 172.4, 172.4, 172.1, 172.1, 171.0, 170.8, 136.5, 136.2, 130.7, 130.7, 130.7, 130.7, 129.7, 129.7, 129.7, 129.7, 128.3, 128.2, 72.5, 72.3, 69.9, 68.4, 58.6, 55.7, 55.5, 55.4, 39.0, 38.9, 38.6, 38.6, 37.9, 37.4, 32.0, 31.3, 31.1, 29.9, 26.2, 26.1, 25.6, 25.2, 23.6, 23.6, 23.5, 23.5, 21.7, 21.6, 21.4, 21.0, 17.5, 17.2.

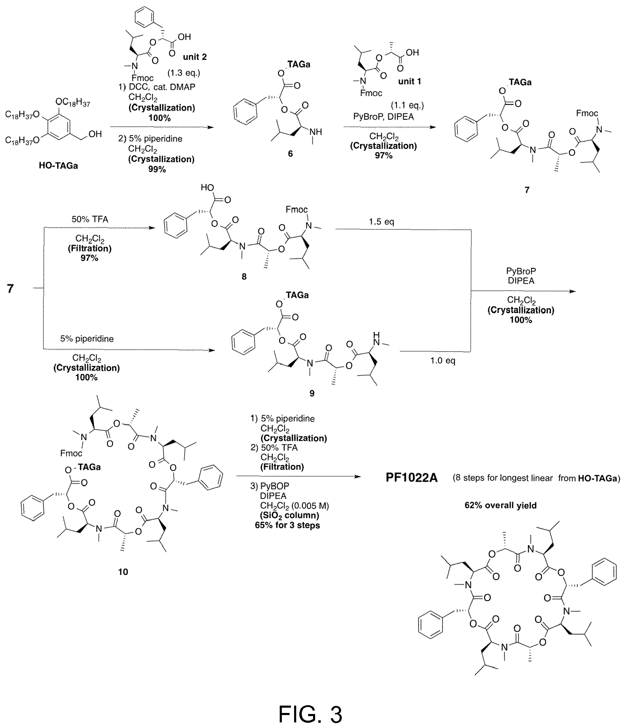

### 3. Preparation of PF1022A(Method 2; cf. reaction scheme in FIG. 3)

### 3-1. Synthetic procedure

### N-Fmoc-N-MeLeu-D- PhLac-O-TAGa

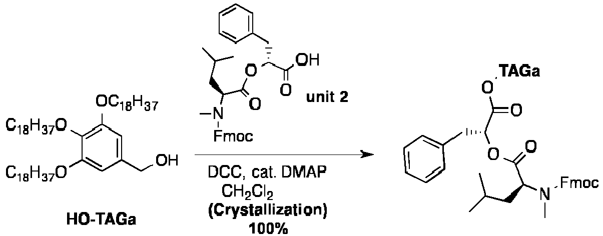

To a stirred solution of **HO-TAGa** (170 mg, 0.186 mmol) in CH₂Cl₂ (3.7 mL) was added 0.1 M toluene solution of **unit 2** (2.42 mL, 0.242 mmol), 4-dimethylaminopyridine (1.1 mg, 9.30 *µmol*), and *N*,*N*'-dicyclohexylcarbodiimide (58 mg, 0.279 mmol) at room temperature under N₂ atmosphere. After stirring for 1 h, the reaction mixture was crystallized by the procedure described in synthesis of ***N*-Fmoc-*N-*MeLeu-D-Lac-O-TAGa** to afford ***N*-Fmoc-*N*-MeLeu-D-PhLac-O-TAGa** (266 mg, 100%) as a colorless powder.
mp: 44 - 45 °C
[α]_{D}²⁷: -6.1 (*c* 1.74, CHCl₃)
¹H-NMR (500 MHz, CDCl₃) δ : 7.76 (complex m, 2H), 7.54 (m, 2H), 7.39 (m, 2H), 7.30 (m, 1H), 7.26-7.14 (complex m, 4H), 7.08 (m, 2H) 6.44 (m, 2H), 5.20 (m, 1H), 5.04-4.94 (complex m, 3H), 4.61-4.15 (complex m, 3H), 3.92 (m, 6H), 3.10 (m, 2H), 2.72 (s, 3H), 1.76 (m, 6H), 1.60-1.25 (complex m, 93H), 0.90-0.71 (complex m, 15H).
¹³C-NMR (125 MHz, CDCl₃) δ : 177.2, 175.2, 169.5, 153.3, 138.4, 135.9, 130.0, 129.6, 129.3, 128.6, 128.4, 127.1, 107.5, 107.2, 73.5, 73.1, 69.2, 67.8, 61.6, 42.5, 37.4, 34.5, 32.0, 30.4, 29.8 (x2), 29.6, 29.5, 26.2, 24.8, 22.8, 22.6, 22.5, 14.2.
HRMS (FAB, NBA matrix) *m*/*z* : 1410.1063 [(M+H)⁺, calcd for C₉₂H₁₄₇NO₉: 1410.1076]

### N-MeLeu-D-PhLac-O-TAGa (6)

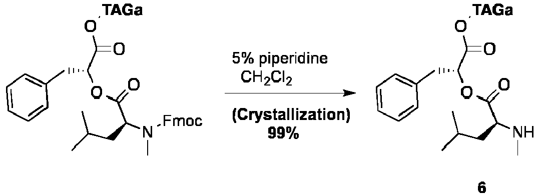

Following the procedure described for general procedure of Fmoc deprotection, ***N*-Fmoc-*N*-MeLeu-D-PhLac-O-TAGa** (457 mg, 0.324 mmol) was converted to **6** (378 mg, 99%) as a colorless powder.
mp: 48 - 49 °C
[α]_{D}²⁶: +4.2 (*c*1.10, CHCl₃)
¹H-NMR (500 MHz, CDCl₃) δ : 7.22 (m, 5H), 6.48 (s, 2H), 5.28 (dd, *J* = 4.0, 10.3 Hz, 1H), 5.08 (d, *J* = 12.0 Hz, 1H), 5.04 (d, *J* = 12.0 Hz, 1H), 3.93 (m, 6H), 3.24 (dd, *J* = 4.0 Hz, 14.3 Hz, 1H), 3.17 (t, *J* = 6.9 Hz, 1H), 3.07 (dd, *J* = 9.7, 14.3 Hz, 1H), 2.18 (s, 3H), 1.76 (m, 6H), 1.48-1.25 (complex m, 93H), 0.90-0.76 (complex m, 15H).
HRMS (FAB, NBA matrix) *m*/*z* : 1189.0458 [(M+H)⁺, calcd for C₇₇H₁₃₈NO₇: 1189.0473]

### N-Fmoc-N-MeLeu-D-Lac-N-MeLeu-D-PhLacO-TAGa (7)

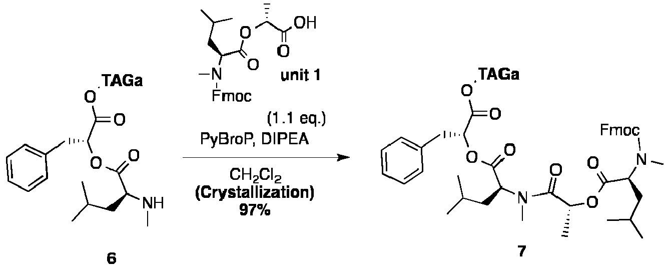

To a stirred solution of **6** (358 mg, 0.301 mmol) in CH₂Cl₂ (6.0 ml) was added 0.1 M toluene solution of **unit 1** (3.31 mL, 0.331 mmol), *N*.*N*-diisopropylethylamine (0.15 mL, 0.903 mmol), and PyBroP (211 mg, 0.452 mm) at room temperature. After stirring for 13 h, reaction mixture was crystallized by the procedure described in synthesis of ***N*-Fmoc-*N*-MeLeu-D-Lac-O-TAGa** to afford 7 (468 mg, 97%) as a colorless powder.
mp: 47 - 48 °C
[α]_{D}²⁶ : -13.9 (c 1.10, CHCl₃)
¹H-NMR (500 MHz, CDCl₃) δ : 7.75 (m, 2H), 7.59 (m, 2H), 7.39 (m, 2H), 7.30-7.09 (complex m, 7H), 6.44 (m, 2H), 5.36-4.96 (complex m, 6H), 4.71-4.24 (complex m, 3H), 3.93 (t, *J* = 6.5 Hz, 6H), 3.14 (m, 2H), 2.90-2.81 (complex s, 6H), 1.80-1.25 (complex m, 105H), 0.96-0.76 (complex m, 21H).
¹³C-NMR (125 MHz, CDCl₃) δ : 171.6, 171.2, 171.1, 170.9, 169.3, 157.0, 153.3, 144.3, 143.9, 141.4 (x2), 138.4, 135.9, 130.0, 129.9, 129.8, 129.7, 129.6, 129.5, 129.3, 128.7, 128.5, 128.4, 127.7, 127.6, 127.0, 125.3, 125.1, 125.0, 124.9, 120.0, 107.5, 107.2, 74.1, 74.0, 73.9, 73.8, 73.5, 71.3, 69.2, 67.9, 67.7, 57.4, 57.2, 56.7, 56.6 (x2), 54.5 (x2), 47.4 (x2), 47.3, 37.6, 37.3, 37.2, 37.1, 37.0, 32.0, 31.1, 30.7, 30.6 (x2), 30.5, 30.4, 30.3, 30.2, 29.8 (x2), 29.6, 29.5 (x2), 26.2, 24.9, 24.8, 24.7, 24.6, 24.4, 23.4, 23.2, 22.9, 22.8, 22.1, 21.4, 21.2, 16.8, 14.2.
HRMS (FAB, NBA matrix) *m*/*z* : 1609.2274 (M⁺, calcd for C₁₀₂H₁₆₈N₂O₁₂: 1609.2284)

### N-MeLeu-D-Lac-N-MeLeu-D-PhLacOH (8)

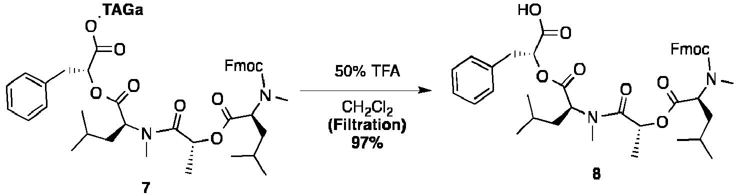

Following the procedure described for general procedure of TAGa cleavage, 7 (244 mg, 0.152 mmol) was converted to **8** (106 mg, 97%) as a yellow oil, which was used next reaction without further purification.

### N-MeLeu-D-Lac-N-MeLeu-D-PhLacO-TAGa (9)

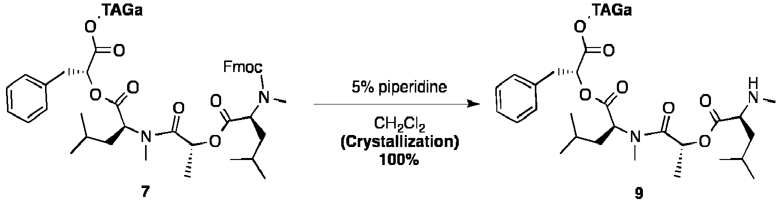

Following the procedure described for general procedure of Fmoc deprotection, **7** (204 mg, 0.127 mmol) was converted to **9** (176 mg, 100%) as a colorless powder.
mp: 47 - 48 °C
[α]_{D}²⁶: -2.1 (c 1.1, CHCl₃)
¹H-NMR (500 MHz, CDCl₃) δ : 7.19 (m, 5H), 6.45 (s, 2H), 5.45 (q, *J* = 6.9 Hz, 1H), 5.31 (ddd, *J* = 4.6, 11.2, 19.6 Hz, 1H), 5.22 (dd, *J* = 5.2, 6.9 Hz, 1H), 5.03 (m, 2H), 3.94 (m, 6H), 3.33-2.95 (complex m, 3H), 2.80 (s, 3H), 2.38 (s, 3H), 1.82-1.25 (complex m, 105H), 0.96-0.75 (complex m, 21H).
¹³C-NMR (125 MHz, CDCl₃) δ : 175.0, 171.1, 171.0, 169.2, 153.3, 130.0, 129.8, 129.7, 129.3, 128.7, 128.6, 128.5, 127.4, 127.1, 126.9, 107.5, 107.2, 73.8, 73.5, 71.3, 69.2, 67.9, 67.7, 67.4, 67.3, 61.2, 57.5, 54.6, 42.3, 42.2, 37.3, 37.2, 37.0, 34.6, 32.0, 31.1, 30.4, 29.8 (x2), 29.6, 29.5 (x2), 26.2, 25.0, 24.8, 23.4, 23.0, 22.8, 22.7, 22.4, 22.1, 21.3, 16.9, 16.8, 14.2.
HRMS (FAB, NBA matrix) *m*/*z*: 1388.1676 [(M+H)⁺, calcd for C₈₇H₁₅₅N₂O₁₀: 1388.1682]

### N-Fmoc-N-MeLeu-D-Lac-N-MeLeu-D-PhLac-N-MeLeu-D-Lac-N-MeLeu-D-PhLac-O-TAGa (10)

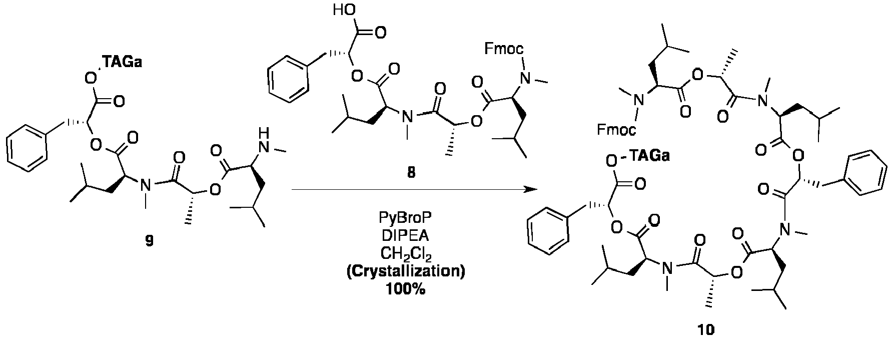

To a stirred solution of **9** (155 mg, 96.2 *µmol*) in CH₂Cl₂ (1.9 mL) was added **8** (103 mg, 0.144 mmol), *N*,*N*-diisopropylethylamine (73 *µL*, 0.434 mmol), and PyBroP (112 mg, 0.241 mmol) at room temperature. After stirring for 66 h, the reaction mixture was crystallized by the procedure described in synthesis of ***N*-Fmoc-*N*-MeLeu-D-Lac-O-TAGa** to afford **10** (201 mg, 100%) as a colorless powder.
mp: 47 - 48 °C
[α]_{D}²⁷: -27.2 (c 1.1, CHCl₃)
¹H-NMR (500 MHz, CDCl₃) δ : 7.75 (m, 2H), 7.59 (m, 2H), 7.38 (m, 2H), 7.30-7.12 (complex m, 12H), 6.44 (m, 2H), 5.43-4.96 (complex m, 10H), 4.69-4.22 (complex m, 3H), 3.93 (m, 6H), 3.24-2.69 (complex m, 16H), 1.80-1.25 (complex m, 114H), 0.95-0.75 (complex m, 33H).
¹³C-NMR (125 MHz, CDCl₃) δ : 174.1, 171.5, 171.4, 171.3, 171.2, 171.1 (x2), 170.9, 170.8, 170.7, 170.6, 170.5, 170.4, 170.0, 169.2, 157.0, 156.5, 153.3, 144.3, 144.2, 144.0 (x2), 141.4 (x2), 138.6, 138.4, 136.1, 136.0, 135.9 (x2), 135.7, 135.6, 135.3, 130.0, 129.8, 129.7, 129.6, 129.5, 129.3, 128.8, 128.7, 128.6, 128.4, 127.7, 127.4, 127.1 (x2), 127.0. 125.3, 125.2, 125.1, 125.0, 120.0, 107.5, 107.2, 107.1, 73.9, 73.5, 72.5, 71.3, 69.2, 68.0, 67.7, 57.5, 56.6 (x2), 55.1, 55.0, 54.9, 54.7, 54.4, 47.4, 47.3, 40.9, 40.6, 38.7, 37.6, 37.3, 37.2, 37.1, 32.0, 31.9, 31.7, 31.5, 31.3, 31.2, 31.0, 30.7, 30.6, 30.4, 30.3, 29.8 (x2), 29.6, 29.5 (x2), 25.0, 24.9, 24.8, 24.7, 24.5, 24.4 (x2), 23.4, 23.3, 23.1, 23.0, 22.9, 22.8, 22.3, 22.1, 22.0, 21.3, 21.2, 16.8, 16.6, 16.5, 14.2.
HRMS (FAB, NBA matrix) *m*/*z* : 2106.4910 [(M+Na)⁺, calcd for C₁₂₈H₂₀₂N₄O₁₈Na: 2106.4912]

### N-MeLeu-D-Lac-N-MeLeu-D- PhLac-N-MeLeu-D-Lac-N-MeLeu-D-PhLac-O-TAGa

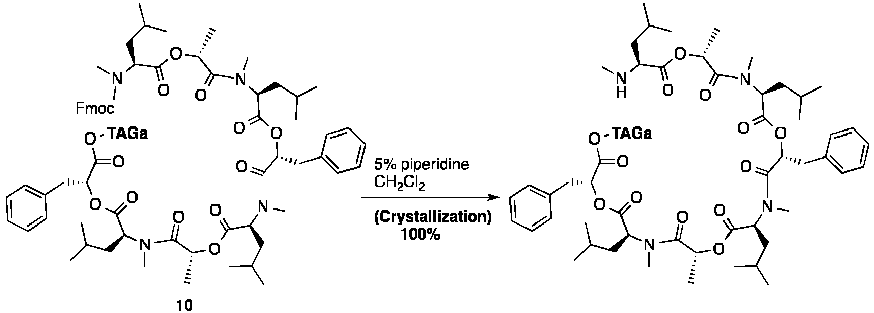

Following the procedure described for general procedure of Fmoc deprotection, **10** (181 mg, 86.8 µmol) was converted to the corresponding amine (163 mg, 100%) as a colorless powder.
mp: 47 - 48 °C
[α]_{D}²⁷ : -18.7 (c 1.3, CHCl₃)
¹H-NMR (500 MHz, CDCl₃) δ : 7.20 (m, 10H), 6.44 (s, 2H), 5.50-4.95 (complex m, 9H), 3.93 (m, 6H), 3.31-2.72 (complex m, 14H), 2.36 (m, 3H), 1.80-1.25 (complex m, 114H), 0.99-0.81 (complex m, 33H).
¹³C-NMR (125 MHz, CDCl₃) δ : 171.4, 171.2, 171.0, 170.9, 170.6, 170.4, 153.3, 138.3, 136.1, 136.0, 135.9, 135.8, 130.0, 129.8 (x2), 129.7, 129.6 (x3), 129.5, 129.4, 129.2, 129.2 (x2), 128.8, 128.7, 128.6, 128.4, 127.4, 127.3, 127.2 (x2), 127.1, 127.0, 107.5, 107.2, 107.1, 73.9, 73.5, 72.3, 72.2, 71.3, 69.2, 68.0 (x2), 67.7, 67.4, 61.3, 55.2, 55.0, 54.8, 54.5, 54.4, 42.4, 40.6, 38.7, 37.7, 37.2, 37.1, 34.7 (x2), 32.0, 31.7, 31.5, 31.0, 30.4, 29.8 (x2), 29.6, 29.5 (x2), 26.2, 25.0, 24.9 (x2), 24.7, 23.5, 23.4 (x2), 23.3, 23.2, 23.0, 22.8, 22.4, 22.1, 21.9, 21.3 (x2), 16.9, 16.8 (x3), 16.7, 16.6, 16.5, 14.2.
HRMS (FAB, NBA matrix) *m*/*z* : 1862.4462 [(M+H)⁺, calcd for C₁₁₃H₁₉₃N₄O₁₆: 1862.4412]

### N-MeLeu-D-Lac-N-MeLeu-D-PhLac-N-MeLeu-D-Lac-N-MeLeu-D-PhLac-OH

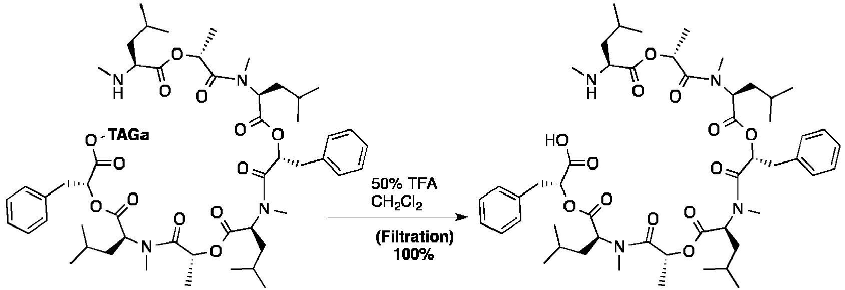

Following the procedure described for general procedure of TAGa cleavage, ***N*-MeLeu-D-Lac-*N-*MeLeu-D-PhLac-*N*-MeLeu-D-Lac-*N*-MeLeu-D-PhLac-O-TAGa** (143 mg, 0.768 mmol) was converted to the corresponding carboxylic acid (78 mg, 100%) as a yellow oil, which was used next reaction without further purification.

### PF1022A

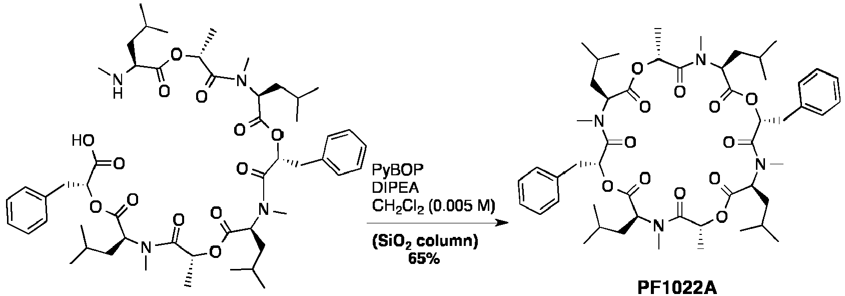

A crude of previous reaction (78 mg, 0.0777 mmol) was dissolved in CH₂Cl₂ (16 mL, 0.005 M). The reaction mixture was added *N*,*N*-diisopropylethylamine (66 *µL*, 0.389 mmol) and PyBOP (81 mg, 0.155 mmol) at room temperature. After stirring for 48 h, the reaction mixture was quenched with saturated aqueous NaHCO₃ (16 mL) at 0 °C and this mixture was extracted with CHCl₃ (20 mL x 2). The combined organic layers were washed with 10% aqueous NaHSO₄ (60 mL) and brine (60 mL), dried over Na₂SO₄, filtered and concentrated *in vacuo.* The crude was purified by column chromatography on silica gel (CHCl₃: MeOH = 400 : 1 to 40 : 1) to provide PF1022A (48mg, 65%) as a colorless solid.

All physical data for synthetic PF1022A matched with the data of authentic PF1022A.

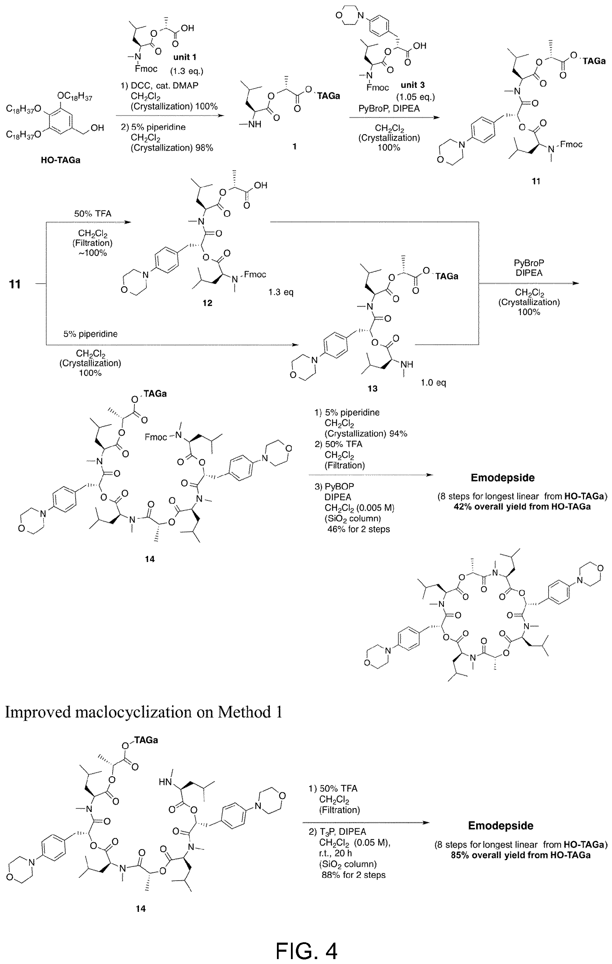

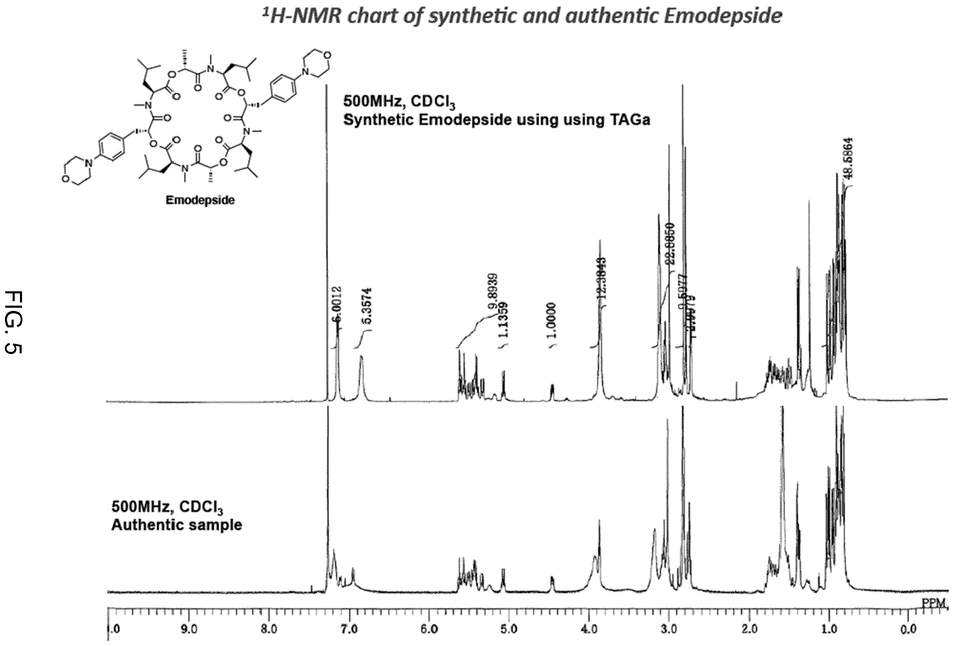

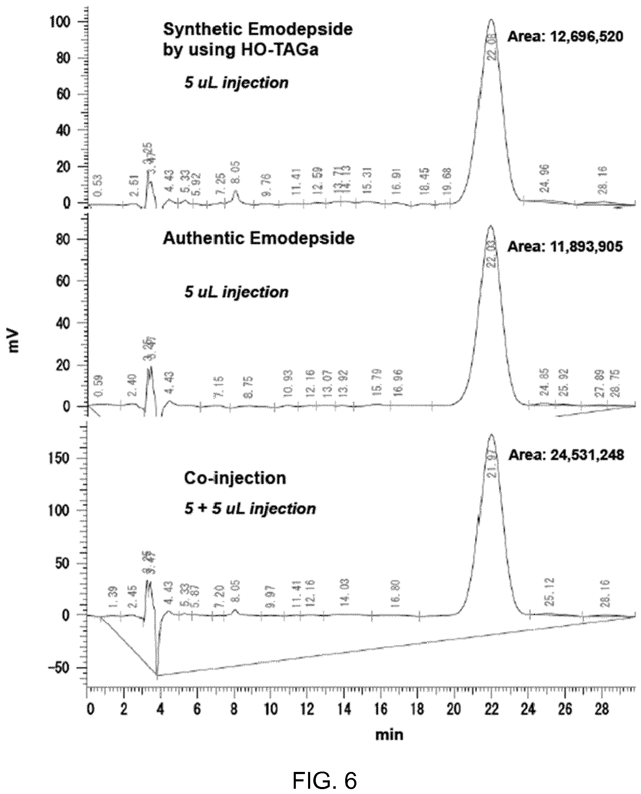

### 4. Preparation of Emodepside (Method 1; cf. reaction schemes in FIG. 4, NMR spectra in FIG. 5 and LC-UV spectra in FIG. 6)

### 4-1. Synthetic procedure

### N-Fmoc-N-MeLeu-D-morphPhLac-N-MeLeu-D-Lac-O-TAGa (11)

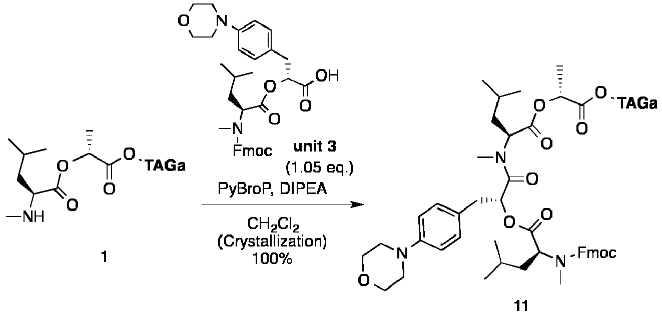

To a stirred solution of **1** (259 mg, 0.233 mmol) in CH₂Cl₂ (4.7 mL) was added 0.2 M toluene solution of **unit 3** (0.122 mL, 0.244 mmol), *N*,*N*-diisopropylethylamine (0.12 mL, 0.698 mmol), and PyBroP (163 mg, 0.349 mmol) at room temperature. After being stirred at 40 h, reaction mixture was crystallized by the procedure described in synthesis of ***N*-Fmoc-*N*-MeLeu-D-Lac-O-TAGa** to afford **11** (395 mg, 100%) as a colorless powder.
¹H-NMR (500 MHz, CDCl₃) δ : 7.74 (m, 2H), 7.57 (m, 2H), 7.42-7.27 (complex m, 4H), 7.05 (m, 2H), 6.70 (d, *J* = 8.6 Hz, 2H), 6.48 (s, 2H), 5.40 (dd, *J* = 6.9, 8.4 Hz, 3/10H, rotamer), 5.35-5.27 (complex m, 17/10H), 5.10-4.97 (complex m, 4H), 4.72-4.12 (complex m, 3H), 3.93 (m, 6H), 3.75 (m, 4H), 3.00-2.79 (complex m, 12H), 1.80-1.60 (complex m, 9H), 1.49-1.42 (complex m, 9H), 1.27 (complex m, 87H), 0.93-0.80 (complex m, 21H).
HRMS (FAB, NBA matrix) *m*/*z*: 1717.2701 [(M+Na)⁺, calcd for C₁₀₆H₁₇₁N₃O₁₃Na: 1717.2710]

### N-Fmoc-N-MeLeu-D-morphPhLac-N-MeLeu-D-Lac-OH (12)

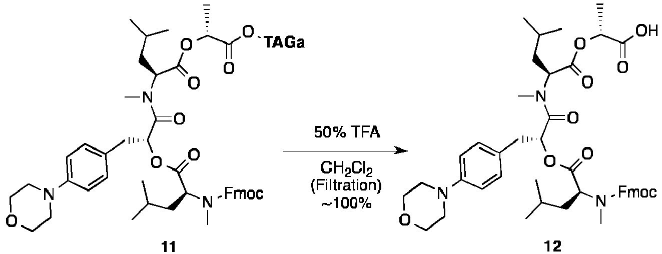

Following the procedure described for general procedure of TAGa cleavage, **11** (210 mg, 0.124 mmol) was converted to **12** (100 mg, ∼0.124 mmol) as a brown oil. This crude was used next reaction without further purification.

### N-MeLeu-D-morphPhLac-N-MeLeu-D-Lac-O-TAGa (13)

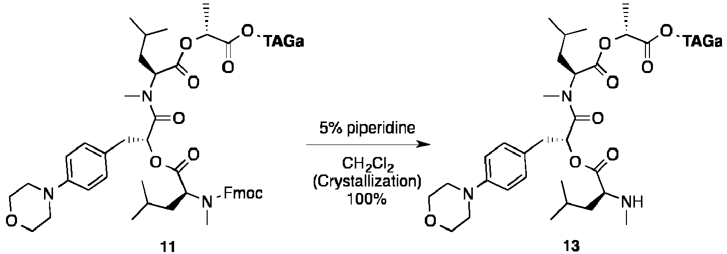

Following the procedure described for general procedure of Fmoc deprotection, **11** (158 mg, 0.0934 mmol) was converted to **13** (138 mg, 100%) as a colorless powder.
¹H-NMR (500 MHz, CDCl₃) δ : 7.15 (m, 2H), 6.83 (d, *J* = 8.6 Hz, 2H), 6.49 (s, 2H), 5.47 (dd, *J* = 6.9, 8.0 Hz, 1H), 5.32 (dd, *J* = 4.9, 11.2 Hz, 1H), 5.09-5.00 (complex m, 3H), 3.94 (m, 6H), 3.85 (m, 4H), 3.27 (t, *J* = 6.9 Hz, 1H), 3.13-2.79 (complex m, 9H), 2.29 (s, 3H), 1.81-1.25 (complex m, 105H), 1.01-0.78 (complex m, 21H).
HRMS (FAB, NBA matrix) *m*/*z*: 1473.2214 [(M+H)⁺, calcd for C₉₁H₁₆₂N₃O₁₁: 1473.2209]

### N-Fmoc-N-MeLeu-D-morphPhLac-N-MeLeu-D-Lac-N-MeLeu-D-morphPhLac-N-MeLeu-D-Lac-O-TAGa (14)

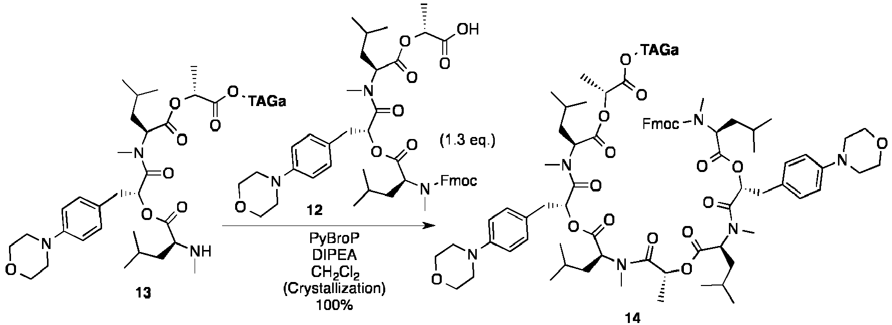

To a stirred solution of **13** (138 mg, 0.934 mmol) in CH₂Cl₂ (1.9 mL) was added **12** (100 mg, ∼0.124 mmol), *N*,*N*-diisopropylethylamine (48 *µL*, 0.280 mmol), and PyBroP (65 mg, 0.140 mmol) at room temperature. After stirring for 18 h, the reaction mixture was crystallized by the procedure described in synthesis of ***N*-Fmoc-*N*-MeLeu-D-Lac-O-TAGa** to afford **14** (211 mg, 100%) as a colorless powder.
¹H-NMR (500 MHz, CDCl₃) δ : 7.75 (m, 2H), 7.58 (m, 2H), 7.42-7.28 (complex m, 4H), 7.09 (m, 4H), 6.77 (m, 4H), 6.48 (s, 2H), 5.45-5.15 (complex m, 6H), 5.06-4.97 (complex m, 4H), 4.73-4.12 (complex m, 3H), 3.95-3.73 (complex m, 14H), 3.15-2.73 (complex m, 24H), 1.80-1.25 (complex m, 114H), 0.96-0.77 (complex m, 33H).
HRMS (FAB, NBA matrix) *m*/*z*: 2276.5962 [(M+Na)⁺, calcd for C₁₃₆H₂₁₆N₆O₂₀Na: 2276.5967]

### N-MeLeu-D-morphPhLac-N-MeLeu-D-Lac-N-MeLeu-D-morphPhLac-N-MeLeu-D-Lac-O-TAGa

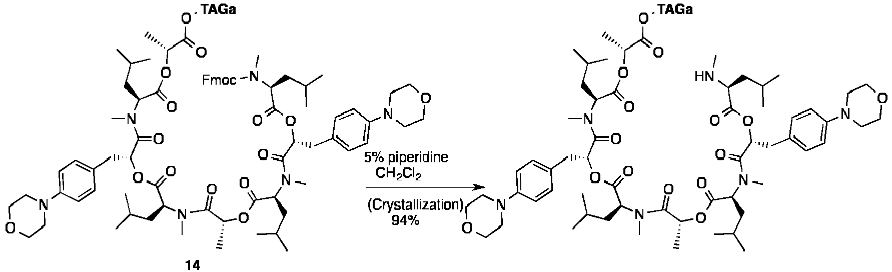

Following the procedure described for general procedure of Fmoc deprotection, **14** (211 mg, 0.0934 mmol) was converted to the corresponding amine (178 mg, 94%) as a colorless powder.
¹H-NMR (500 MHz, CDCl₃) δ : 7.15 (m, 4H), 6.82 (m, 4H), 6.49 (s, 2H), 5.14 (t, *J* = 7.45 Hz, 1H), 5.44-5.09 (complex m, 5H), 5.07-5.00 (complex m, 3H), 3.93 (m, 6H), 3.85 (m, 8H), 3.32 (m, 1H), 3.17-2.74 (complex m, 21H), 2.33 (s, 3H), 1.81-1.64 (complex m, 12H), 1.55-1.25 (complex m, 102H), 1.03-0.77 (complex m, 33H).
HR-MS (FAB, NBA matrix+NaI) *m*/*z*: 2032.5488 [(M+H)⁺, calcd for C₁₂₁H₂₀₇N₆O₁₈: 2032.5467]

### N-MeLeu-D-morphPhLac-N-MeLeu-D-Lac-N-MeLeu-D-morphPhLac-N-MeLeu-D-Lac-OH

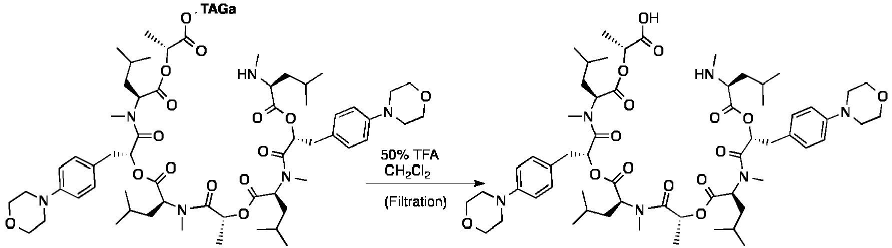

Following the procedure described for general procedure of TAGa cleavage, ***N*-MeLeu-D-morphPhLac-*N*-MeLeu-D-Lac-*N*-MeLeu-D-morphPhLac-*N*-MeLeu-D-LacO-TAGa** (178 mg, 0.0876 mmol) was converted to the corresponding carboxylic acid (∼0.0876 mmol) as a crude oil, which was used next reaction without further purification.

### Emodepside (highly diluted condition using PyBOP)

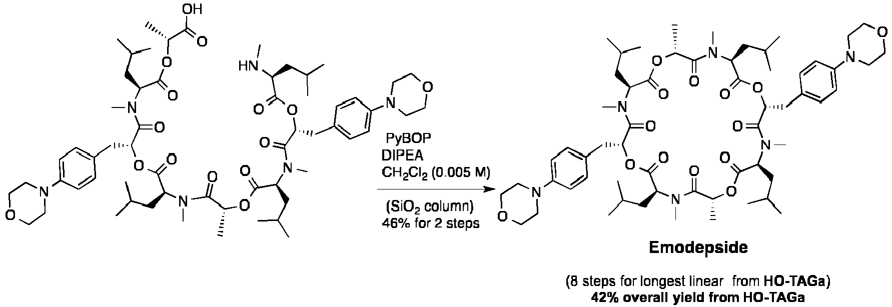

To a crude of carboxylic acid (∼0.0876 mmol) in CH₂Cl₂ (18 mL, 0.005 M) was added *N,N-*diisopropylethylamine (0.10 mL, 0.613 mmol) and PyBOP (91 mg, 0.175 mmol) at room temperature. After stirring for 19 h, the reaction mixture was quenched with saturated aqueous NaHCO₃ (18 mL) at 0 °C and this mixture was extracted with CHCl₃ (20 mL x 3). The combined organic layers were washed with 10% aqueous NaHSO₄ (60 mL) and brine (60 mL), dried over Na₂SO₄, filtered and concentrated *in vacuo.* The crude was purified by column chromatography on silica gel (CHCl₃: MeOH = 400 : 1 to 100 : 1) to provide **Emodepside** (45 mg, 46% for 2 steps) as a light yellow solid.
mp: 98-103 °C
[α]_{D}²⁴ : -40.3 (c 0.67, CHCl₃)
IR (neat) ṽₘₐₓ: 2954, 2862, 1743, 1659, 1520, 1458, 1412, 1265, 1234, 1188, 1119, 1072, 1026, 926, 810.
¹H-NMR (500 MHz, CDCl₃) δ : 7.14-7.11 (complex m, 4H), 6.83-6.79 (complex m), 5.64-5.54 (complex m), 5.52-5.43, 5.43-5.39, 5.33, 5.18, 5.06 and 4.46 (5 m, 6H), 3.85 (apparently t, 8H), 3.15-3.04 (complex m, 8H), 3.04-2.92 (complex m, 4H), 3.00, 2.82, 2.79, 2.72 and 2.71 (5 s, 12H), 1.82-1.24 (complex m, 18H), 1.03-0.79 (complex m, 30H).
¹³C-NMR (125 MHz, CDCl₃) δ : 171.6, 171.2, 171.0, 170.9, 170.3, 170.2, 170.1, 169.8, 169.7, 150.2, 130.4, 130.2, 115.7, 115.6, 71.2, 70.8, 68.5, 66.8, 66.7, 57.1, 54.0, 53.9, 49.3, 49.2, 38.0, 37.5, 37.1, 36.9, 36.8, 36.7, 36.6, 36.1, 31.1, 30.6, 30.4, 29.7, 29.6, 29.3, 25.0, 24.8, 24.6, 24.5, 24.5, 24.1, 23.6, 23.5, 23.4, 23.3, 23.3, 23.1, 22.6, 21.6, 21.5, 21.1, 21.1, 21.0, 20.8, 17.1, 15.7.
HRMS (ESI) *m*/*z*: 1141.6404 [(M+Na)⁺, calcd for C₆₀H₉₀N₆O₁₄Na: 1141.6413]
*Literature *(*Eur. J. Org. Chem., 2012, 1546-1553)
¹H-NMR (400 MHz, CDCl₃) δ : 7.17-7.09 (m, 4 H, Ar-H), 6.86-6.77 (m, 4 H, Ar-H), 5.67-5.54 (m, 2 H, CaH-Lac), 5.53-5.38, 5.34, 5.19, 5.08 and 4.47 (5 m, 6 H, CaH-Leu, CαH-morphPhLac), 3.88-3.81 (pseudo-t, 8 H, OCH₂morpholine), 3.15-3.08 (m, 8 H, N-CH₂-morpholine), 3.07-2.85 (m, 4 H, CβH₂-morphPhLac), 3.00, 2.83, 2.80, 2.74 and 2.73 (5 s, 12 H, NCH₃), 1.83-1.20 (m, 18 H, CβH₂-Leu, CγH-Leu, CH₃-Lac), 1.05-0.77 (m, 30 H, CδH₃-Leu, CβH₃-Lac).
¹³C-NMR (100 MHz, CDCl₃) δ : 171.7, 171.2, 171.0, 170.6, 170.4, 170.2, 169.8, 141.7, 130.6, 130.4, 116.9, 116.1, 71.3, 70.8, 68.6, 66.9, 66.6, 66.5, 57.1, 54.0, 49.9, 38.1, 37.5, 37.2, 36.7, 36.2, 31.2, 30.5, 29.4, 24.9, 24.7, 24.2, 23.6, 23.5, 23.5, 23.4, 21.2, 21.1, 20.9, 17.1, 15.8.

### Emodepside (slow addition condition using T3P)

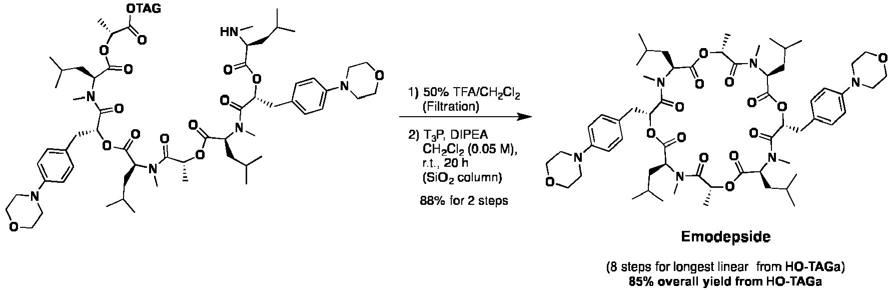

*Cleavage of TAGa:* To a stirring solution of linear compound on TAG (179.0 mg, 0.088 mmol) in DCM (1.8 mL) was added TFA (1.8 mL) at room temperature. After stirring for 6 h at room temperature, the solution was concentrated *in vacuo.* The resulting mixture was dissolved into toluene (10 mL) and concentrated under reducing pressure for 3 times to remove excess TFA. The crude residue was dissolved into CH₂Cl₂ (1.0 mL), then recrystallized for the cleaved TAGa materials by the addition of MeOH (8.0 mL) at room temperature. The precipitates were filtered off through Celite® pad and washed with MeOH (20 mL). The combined filtrates were concentrated *in vacuo.* To the resulting product was added 4 M HCl/Dioxane (0.05 M for product), followed by diluted with toluene (10 mL) and concentrated to afford the TFA salt free product. To remove excess HCl from a crude product, the product was dissolved again into toluene (10 mL) and concentrated under reducing pressure for 2 times.

*Cyclization:* To a stirring solution of T3P® (50% in EtOAc, 110 µL, 0.187 mmol) in DIPEA (110 µL*,* 0.187 mmol) was added dropwise a crude linear compound (0.088 mmol) in DCM (1.8 mL, 0.05 M including wash) over 2.5 h at room temperature. After stirring for 20 h at room temperature, the reaction mixture was quenched with sat. NaHCO₃ aq. (3.0 mL), extracted with CHCl₃ (2.0 mL x 3). The combined organic layers were dried over sodium sulfate, filtered, and concentrated *in vacuo.* The crude residue was purified by silica gel column chromatography on silica gel (CHCl₃/MeOH = 100/1) to provide Emodepside (86.4 mg, 88%) as an amorphous. The analytical data was identified by the authentic sample.

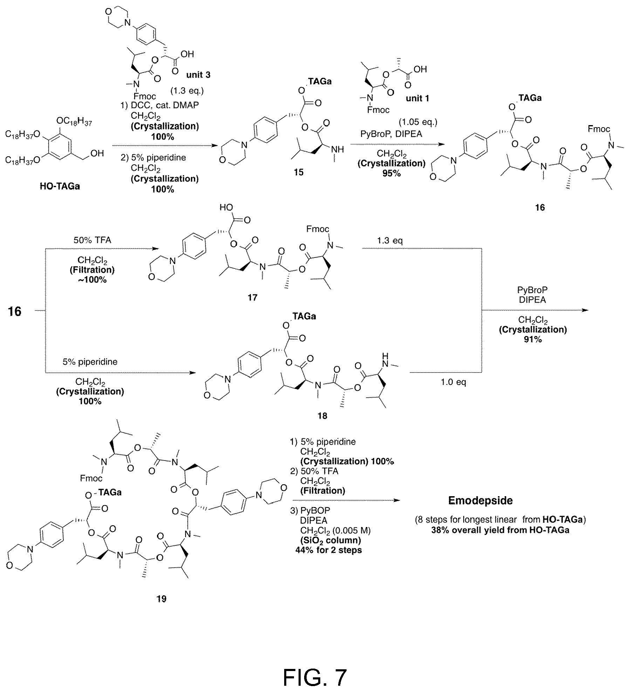

### 5. Preparation of Emodepside (Method 2; cf. reaction scheme in FIG. 7)

### N-Fmoc-N-MeLeu-D-morphPhLac-O-TAGa

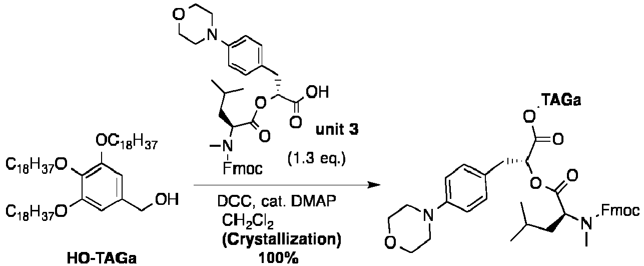

To a stirred solution of **HO-TAGa** (381 mg, 0.417 mmol) in CH₂Cl₂ (8.4 mL) was added 0.2 M toluene solution of **unit** 3 (2.71 mL, 0.542 mmol), 4-dimethylaminopyridine (2.5 mg, 20.8 µmol), and *N*,*N*'-dicyclohexylcarbodiimide (129 mg, 0.626 mmol) at room temperature under N₂ atmosphere. After stirring for 1 h, the reaction mixture was crystallized by the procedure described in synthesis of *N-***Fmoc-*N*-MeLeu-D-Lac-O-TAGa** to afford ***N*-Fmoc-*N*-MeLeu-D-morphPhLac**- **O-TAGa** (628 mg, 100%) as a colorless powder.
mp : 46-47 °C
[α]_{D}²⁷ = -3.1 (*c* 1.0, CHCl₃)
¹H-NMR (500 MHz, CDCl₃) δ : 7.78 (d, *J* = 7.5 Hz, 1H), 7.74 (d, *J* = 7.5 Hz, 1H), 7.57 (m, 2H), 7.39 (m, 2H), 7.27 (m, 2H), 6.98 (d, *J* = 8.6 Hz, 1H), 6.96 (d, *J* = 8.6 Hz, 1H), 6.67 (m, 2H), 6.49 (2 s, rotamer 4:3, 2H), 5.17 (2 dd, rotamer, *J* = 4.0 Hz, 8.0 Hz, 1H), 5.08-4.98 (complex m, 3H), 4.67-4.13 (complex m, 3H), 3.93 (m, 6H), 3.73 (m, 4H), 3.08 (dd, *J* = 4.0 Hz, 14.6 Hz, 1H), 3,07-2.95 (complex m, 5H), 2.80 (rotamer 4:3, 3H), 1.76 (m, 6H), 1.64-1.53 (complex m, 3H), 1.45 (m, 6H), 1.28 (complex m, 84H), 0.93-0.86 (complex m, 14H), 0.76 (d, *J* = 6.3 Hz, 1H).
HRMS (FAB, NBA matrix) *m*/*z*: 1495.1583 (M⁺, calcd for C₉₆H₁₅₄N₂O₁₀: 1495.1604)

### N-MeLeu-D-morphPhLac-O-TAGa (15)

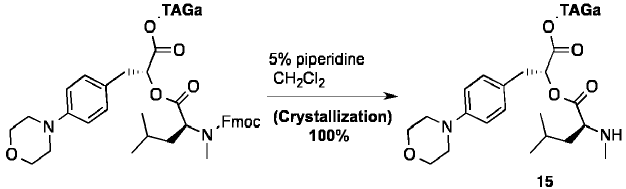

Following the procedure described for general procedure of Fmoc deprotection, ***N*-Fmoc-*N*-MeLeu-D-morphPhLac-O-TAGa** (628 mg, 0.417 mmol) was converted to **15** (530 mg, 100%) as a colorless powder.
mp : 49-50 °C
[α]_{D}²⁷ = +5.7 (c 1.0, CHCl₃)
¹H-NMR (500 MHz, CDCl₃) δ : 7.08 (d, *J* = 8.6 Hz, 2H), 6.80 (d, *J* = 8.6 Hz, 2H), 6.51 (s, 2H), 5.24 (dd, *J* = 4.0, 9.7 Hz, 1H), 5.07 (q, *J* = 12.0 Hz, 2H), 3.94 (m, 6H), 3.85 (m, 4H), 3.18 (m, 2H), 3.10 (m, 4H), 3.00 (d, *J* = 10.3, 14.3 Hz, 1H), 2.21 (s, 3H), 1.76 (m, 6H), 1.47 (m, 6H), 1.34-1.25 (complex m, 87H), 0.88 (t, *J* = 6.9 Hz, 9H), 0.80 (d, *J* = 6.9 Hz, 3H), 0.79 (d, *J* = 6.9 Hz, 3H).
HRMS (FAB, NBA matrix) *m*/*z* : 1274.0986 [(M+H)⁺, calcd for C₈₁H₁₄₅N₂O₈: 1274. 1001]

### N-Fmoc-N-MeLeu-D-Lac-N-MeLeu-D-morphPhLac-O-TAGa (16)

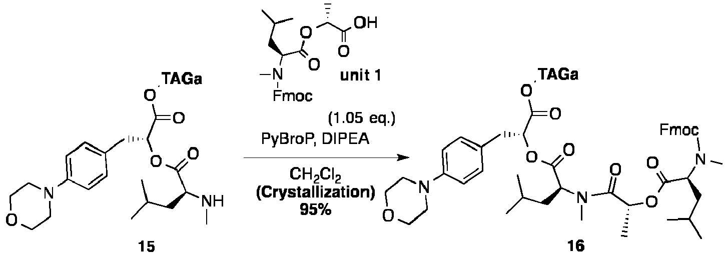

To a stirred solution of **15** (530 mg, 0.417 mmol) in CH₂Cl₂ (8.4 mL) was added 0.2 M toluene solution of **unit 1** (0.22 mL, 0.44 mmol), *N*,*N*-diisopropylethylamine (0.212 mL, 1.25 mmol), and PyBroP (291 mg, 0.62 mmol) at room temperature. After stirring for 16 h, the reaction mixture was crystallized by the procedure described in synthesis of ***N*-Fmoc-*N*-MeLeu-D-Lac-O-TAGa** to afford **16** (670 mg, 95%) as a colorless powder.
mp : 48-49 °C
[α]_{D}²⁷ = -12.4 (c 1.0, CHCl₃)
¹H-NMR (500 MHz, CDCl₃) δ : 7.76 (m, 2H), 7.61 (m, 2H), 7.38 (m, 2H), 7.30 (m, 2H), 7.02 (m, 2H), 6.74 (m, 2H), 6.49 (2 s, rotamer, 2H), 5.38-5.22 (complex m, 2H), 5.15-4.98 (complex m, 4H), 4.47 (complex m, 3H), 3.94 (m, 6H), 3.81 (m, 4H), 3.12-2.78 (complex m, 12H), 1.82-1.56 (complex m, 9H), 1.53-1.40 (complex m, 8H), 1.34-1.26 (complex m, 88H), 0.98-0.75 (complex m, 21H).
HRMS (FAB, NBA matrix) *m*/*z*: 1694.2828 (M⁺, calcd for C₁₀₆H₁₇₁N₃O₁₃: 1694.2812)

### N-Fmoc-N-MeLeu-D-Lac-N-MeLeu-D-morphPhLac-OH (17)

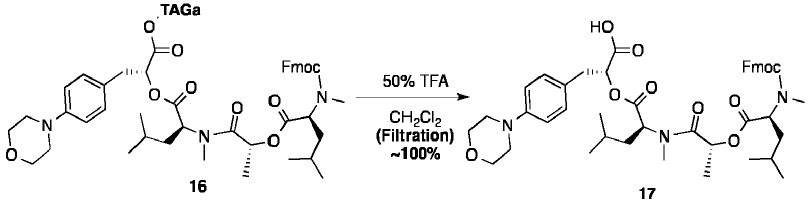

Following the procedure described for general procedure of TAGa cleavage, **16** (376 mg, 0.222 mmol) was converted to **17.** In the case of this substrate, the reaction required longer time than that of the general condition of TAGa cleavage (ca. 1 h). The reaction of **16** in 50% TFA/CH₂Cl₂ at room temperature was needed to stir for 8 h to consume all of starting material, and gave product **17** (178 mg, 0.222 mmol) as a crude oil, which was used next reaction without further purification.

### N-MeLeu-D-Lac-N-MeLeu-D-morphPhLac-O-TAGa (18)

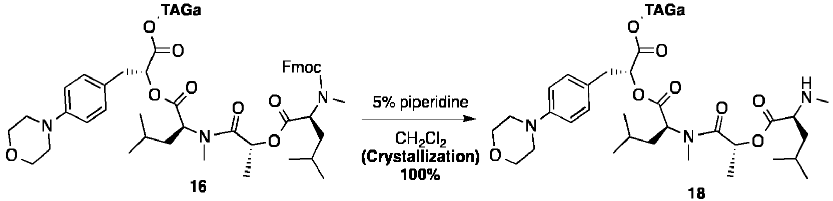

Following the procedure described for general procedure of Fmoc deprotection, **16** (290 mg, 0.171 mmol) was converted to **18** (251 mg, 100%) as a colorless powder.
mp : 43-45 °C
[α]_{D}²⁵ = -2.8 (c 1.0, CHCl₃)
¹H-NMR (500 MHz, CDCl₃) δ : 7.07 (d, *J* = 8.6 Hz, 2H), 6.79 (d, *J* = 8.6 Hz, 2H), 6.49 (s, 2H), 5.46 (q, *J* = 6.9 Hz, 1H), 5.33 (dd, *J* = 5.2, 10.9 Hz, 1H), 5.17 (dd, *J* = 5.2, 7.5 Hz, 1H), 5.06 (m, 2H), 3.95 (m, 6H), 3.84 (m, 4H), 3.33 (t, *J* = 7.5 Hz, 1H), 3.11-3.06 (complex m, 6H), 2.82 (2 s, rotamer 4:1, 3H), 2.40 (s, rotamer, 3H), 1.82-1.59 (complex m, 10H), 1.52-1.43 (complex m, 8H), 1.34-1.25 (complex m, 87H), 0.97-0.86 (complex m, 21H).
HRMS (FAB, NBA matrix + NaI) *m*/*z*: 1473.2222 [(M+H)⁺, calcd for C₉₁H₁₆₂N₃O₁₁: 1473.2209]

### N-Fmoc-N-MeLeu-D-Lac-N-MeLeu-D-morphPhLac-N-MeLeu-D-Lac-N-MeLeu-D-morphPhLac-O-TAGa (19)

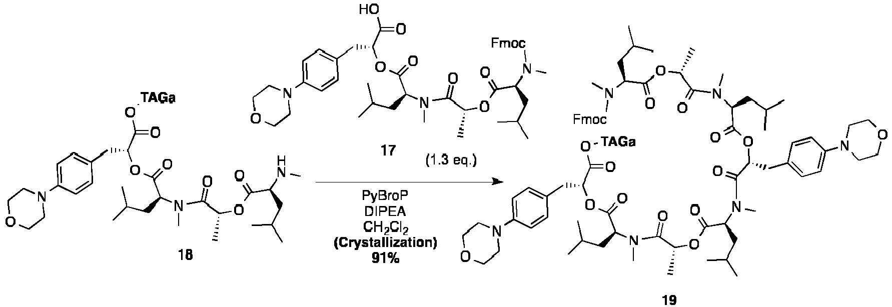

To a stirred solution of **18** (251 mg, 0.171 mmol) in CH₂Cl₂ (3.4 mL) was added **17** (178 mg, 0.222 mmol), *N*,*N*-diisopropylethylamine (87 *µL*, 0.513 mmol), and PyBroP (120 mg, 0.257 mmol) at room temperature. After stirring at 46 h, the reaction mixture was crystallized by the procedure described in synthesis of ***N*-Fmoc-*N*-MeLeu-D-Lac-O-TAGa** to afford **19** (350 mg, 91%) as a colorless powder.
mp : 50-52 °C
[α]_{D}²⁵ = -25.5 (*c* 1.0, CHCl₃)
¹H-NMR (500 MHz, CDCl₃) δ : 7.75 (m, 2H), 7.62 (m, 2H), 7.38 (m, 2H), 7.30 (m, 2H), 7.06 (m, 4H), 6.77 (m, 4H), 6.49 (2 s, rotamer, 2H), 5.42-4.98 (complex m, 10H), 4.73-4.20 (complex m, 3H), 3.94 (m, 6H), 3.84 (m, 8H), 3.17-2.66 (complex m, 24H), 1.80-1.64 (complex m, 14H), 1.46-1.25 (complex m, 100H), 1.00-0.77 (complex m, 33H).
HRMS (FAB, NBA matrix) *m*/*z*: 2276.5940 [(M+Na)⁺, calcd for C₁₃₆H₂₁₆N₆O₂₀Na: 2276.5967]

### N-MeLeu-D-Lac-N-MeLeu-D-morphPhLac-N-MeLeu-D-Lac-N-MeLeu-D- morphPhLac-O-TAGa

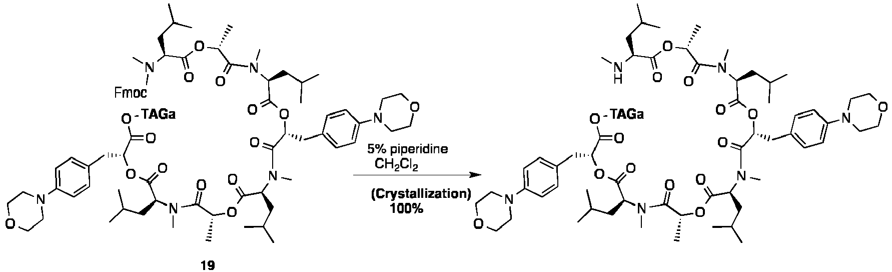

Following the procedure described for general procedure of Fmoc deprotection, **19** (114 mg, 0.0505 mmol) was converted to the corresponding amine (103 mg, 100%) as a colorless powder.
mp : 45-47 °C
[α]D²⁵ = -19.6 (c 1.0, CHCl₃)
¹H-NMR (500 MHz, CDCl₃) δ : 7.76 (m, 4H), 6.79 (m, 4H), 6.48 (s, 2H), 5.50-4.96 (complex m, 9H), 3.94 (m, 6H), 3.83 (m, 8H), 3.30 (m, 1H), 3.16-2.74 (complex m, 21H), 2.38 (m, 3H), 1.80-1.55 (complex m, 9H), 1.47-1.24 (complex m, 105H), 1.00-0.81 (complex m, 33H).
HRMS (FAB, NBA matrix) *m*/*z*: 2032.5468 [(M+H)⁺, calcd for C₁₂₁H₂₀₇N₆O₁₈: 2032.5467]

### N-MeLeu-D-Lac-N-MeLeu-D-morphPhLac-N-MeLeu-D-Lac-N-MeLeu-D- morphPhLac-OH

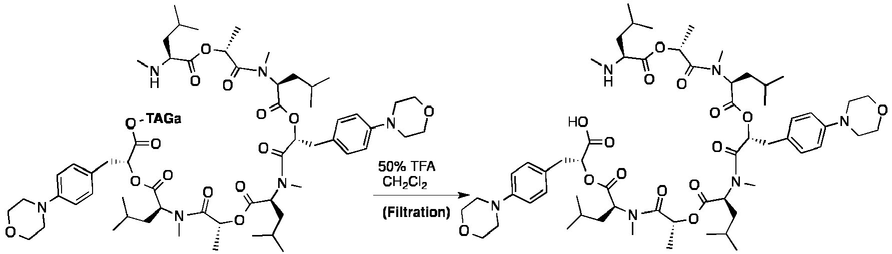

Following the procedure described for general procedure of TAGa cleavage, ***N*-MeLeu-D-Lac-*N-*MeLeu-D-morphPhLac-*N*-MeLeu-D-Lac-*N*-MeLeu-D-morph- PhLac-O-TAGa** (102 mg, 0.0502 mmol) was converted to the corresponding carboxylic acid. In the case of this substrate, the reaction required longer time than that of the general condition of TAGa cleavage (ca. 1 h). The reaction in 50% TFA/CH₂Cl₂ at room temperature was needed to stir for 5 h to consume all of starting material, and gave desired product (∼0.0502 mmol) as a crude oil, which was used next reaction without further purification.

### Emodepside

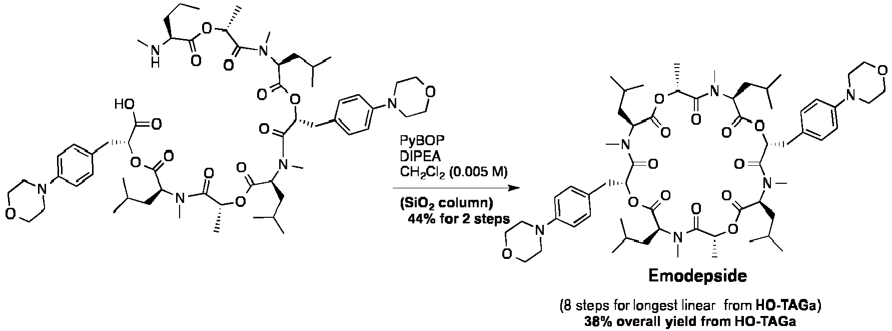

To a crude of carboxylic acid (∼0.0502 mmol) in CH₂Cl₂ (10 ml, 0.005 M) was added *N,N-*diisopropylethylamine (60 *µL*, 0.351 mmol) and PyBOP (52 mg, 0.175 mmol) at room temperature. After stirring for 44 h, the reaction mixture was quenched with saturated aqueous NaHCO₃ (10 mL) at 0 °C and this mixture was extracted with CHCl₃ (20 ml x 3). The combined organic layers were washed with 10% aqueous NaHSO₄ (60 ml) and brine (60 ml), dried over Na₂SO₄, filtered and concentrated *in vacuo.* The crude was purified by column chromatography on silica gel (CHCl₃: MeOH = 400 : 1 to 100 : 1) to provide **Emodepside** (25 mg, 44% for 2 steps) as a light brown solid.

All physical data for synthetic Emodepside matched with the data of authentic compound.

### Comparative Example 1

A tag analogous to TAGa, but with C₁₂ instead of C₁₈ alkyl chains (C12-TAG), was prepared and coupled with *N*-Fmoc-*N*-MeLeu-D-Lac-OH (unit 1) according to the following reaction scheme:

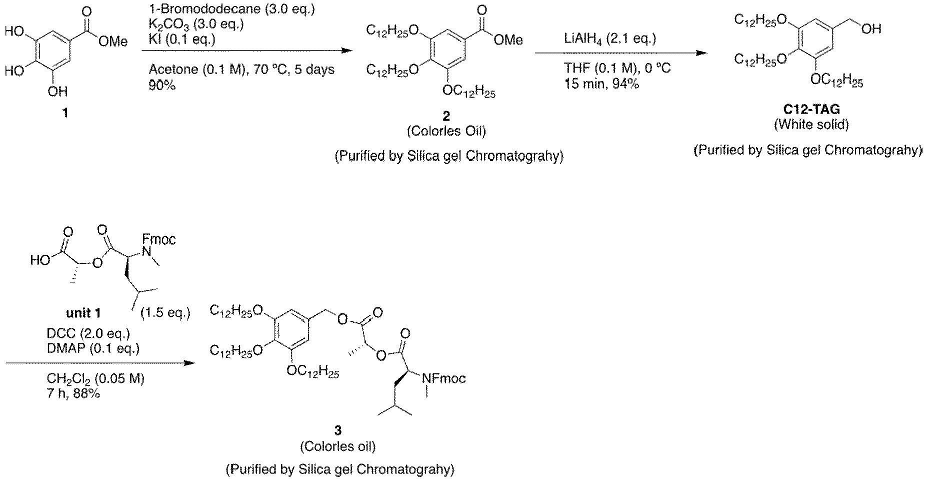

Purification via silica gel chromatography was necessary after each reaction step. The compounds 2, C12-TAG and 3 did not crystallize in methanol. This is in contrast to, for example, the synthetic procedure for *N*-Fmoc-*N*-MeLeu-D-Lac-O-TAGa (section 2.1 above). The C12-TAG in this comparative example was not suitable for the intended tag-assisted synthesis and the further functionalization of compound 3 was not investigated further.

### Comparative example 2

The commercially available C1-TAG was coupled with *N*-Fmoc-*N*-MeLeu-D-Lac-OH (unit 1) according to the following reaction scheme:

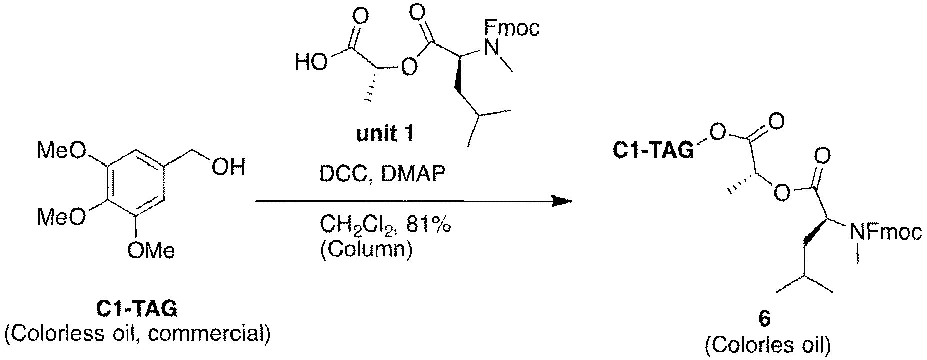

Purification via column chromatography was necessary after the reaction step. The compounds C1-TAG and 6 did not crystallize in methanol. The C1-TAG in this comparative example was not suitable for the intended tag-assisted synthesis and the further functionalization of compound 6 was not investigated further.

## Claims

1. A method for the synthesis of cyclic depsipeptides according to the general formula (1) from depsipeptides according to the general formula (II): 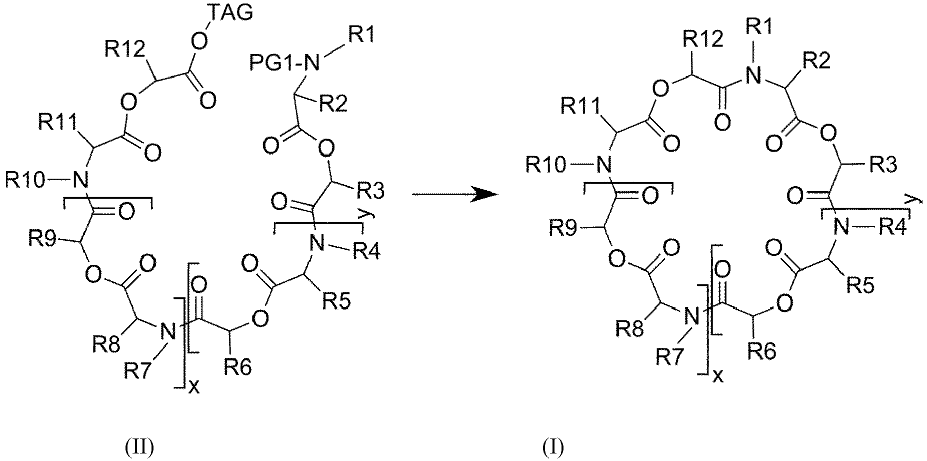
wherein PG1 is an amine protecting group and TAG is a carboxylic acid protecting group,
the method comprising the steps of:
- deprotecting the amine group which is protected by the PG1 group in the presence of a base, thereby obtaining a deprotected amine group;
- deprotecting the carboxylic acid which is protected by the TAG group in the presence of an acid, thereby obtaining a deprotected carboxylic acid group;
- condensation of the deprotected amine and carboxylic acid groups, thereby obtaining the cyclic depsipeptide (I);
**characterized in that** the TAG group comprises a moiety Aryl-O-(CH₂)n- with Aryl representing an aromatic moiety and n being ≥ 13,
wherein x and y are, independent of each other, 0, 1 or 2 with the proviso that x+y ≥ 1
and wherein R1, R2, R3, R4, R5, R6, R7, R8, R9, R10, R11 and R12 each, independent of each other, represent hydrogen, straight-chain or branched C1-C8-alkyl, straight-chain or branched halogenated C1-C8-alkyl, hydroxy-C1-C6-alkyl, C1-C4-alkanoyloxy-C1-C6-alkyl, C1-C4-alkoxy-C1-C6-alkyl, aryl-C1-C4-alkyloxy-C 1 -C6-alkyl, mercapto-C 1 -C6-alkyl, C1-C4-alkylthio-C1 -C6-alkyl, C1-C4-alkylsulphinyl-C1-C6-alkyl, C1-C4-alkylsulphonyl-C1-C6-alkyl, carboxy-C1-C6-alkyl, C1-C4-alkoxycarbonyl-C1-C6-alkyl, C1-C4-arylalkoxycarbonyl-C1-C6-alkyl, carbamoyl-C1-C6-alkyl, amino-C1-C6-alkyl, C1-C4-alkylamino-C 1-C6-alkyl, C1-C4-dialkylamino-C1-C6-alkyl, guanidino-C1-C6-alkyl, C1-C4-alkoxycarbonylamino-C 1 -C6-alkyl, tert-butoxycarbonylaminobutyl, 9-fluorenylmethoxycarbonyl(Fmoc)amino-C1-C6-alkyl, C2-C8-alkenyl, C3-C7-cycloalkyl, C3-C7-cycloalkyl-C1-C4-alkyl, benzyl, substituted benzyl, phenyl, phenyl-C1-C4-alkyl, hydroxyl, C1-C4-alkoxy or C1-C4-alkyl.

2. The method according to claim 1, wherein x is 1, y is 1, R1, R4, R7 and R10 are methyl, R6 and R12 are methyl, R5 and R11 are, independent of each other, straight-chain or branched C1-C4-alkyl or straight-chain or branched halogenated C1-C4-alkyl and R3 and R9 are, independent of each other, benzyl or substituted benzyl.

3. The method according to claim 1 or 2, wherein PG1 is 9-fluorenylmethoxycarbonyl (Fmoc), t-butyl carbamate (Boc), benzyl carbamate (Z), acetamide, trifluoroacetamide, phthalimide, benzyl (Bn), triphenylmethyl (Tr), benzylidene or p-toluenesulfonamide (Ts)
and TAG is: 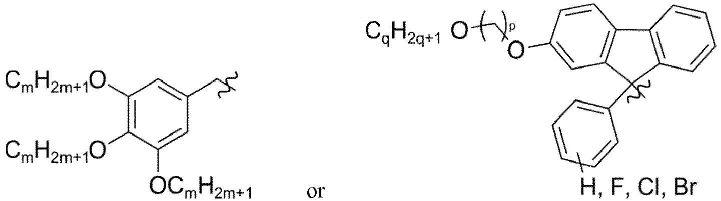 wherein m is ≥ 15 to ≤ 25, p is ≥ 8 to ≤ 18 and q is ≥ 15 to ≤ 25.

4. The method according to one of claims 1 to 3, wherein the depsipeptide according to the general formula (II) is obtained from precursors according to the general formulas (IV) and (III): 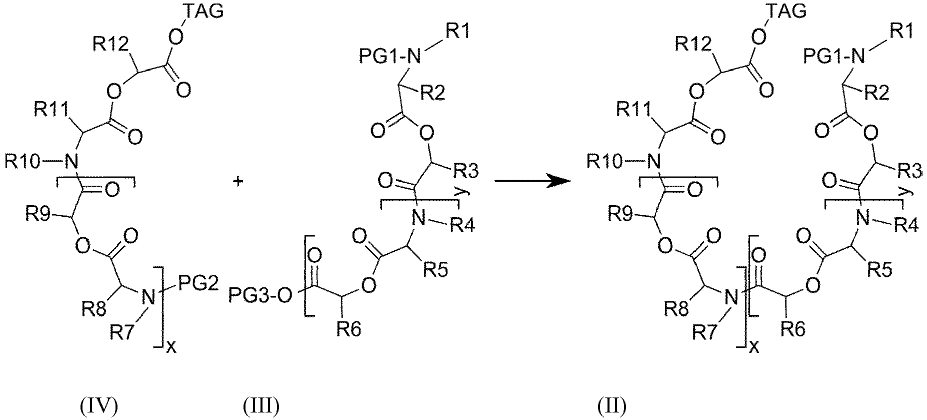 by:
- in precursor (IV), deprotecting the amine group which is protected by the PG2 group in the presence of a base, thereby obtaining a deprotected amine group;
- in precursor (III), deprotecting the carboxylic acid which is protected by the PG3 group in the presence of an acid, thereby obtaining a deprotected carboxylic acid group;
- condensation of the deprotected amine and carboxylic acid groups, thereby obtaining the depsipeptide (II);
wherein R1 to R12, TAG, PG1, x, and y have a meaning as defined in one of claims 1 to 3, PG2 is an amine protecting group and PG3 is a carboxylic acid protecting group.

5. The method according to claim 4, wherein the precursor according to the general formula (IV) is obtained from precursors according to the general formulas (VI) and (V): 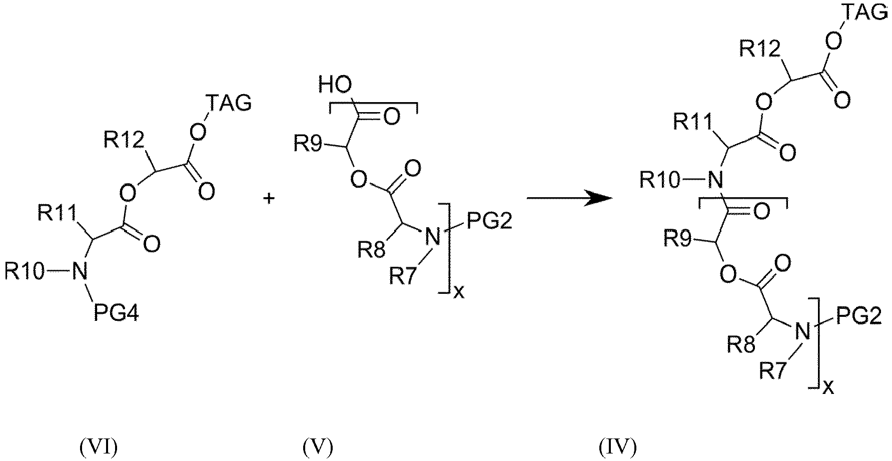 by:
- in precursor (VI), deprotecting the amine group which is protected by the PG4 group in the presence of a base, thereby obtaining a deprotected amine group;
- condensation of the deprotected amine group of precursor (VI) and carboxylic acid group of precursor (V), thereby obtaining the precursor (IV);
wherein R7 to R12, TAG and x have a meaning as defined in one of claims 1 to 4, PG2 has a meaning as defined in claim 4 and PG4 is an amine protecting group.

6. The method according to claim 4, wherein the precursor according to the general formula (III) is obtained from precursors according to the general formulas (VIII) and (VII): 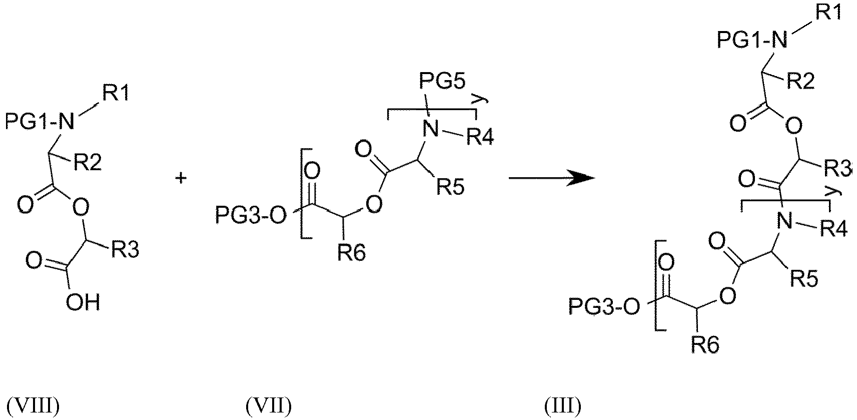 by:
- in precursor (VII), deprotecting the amine group which is protected by the PG5 group in the presence of a base, thereby obtaining a deprotected amine group;
- condensation of the deprotected amine group of precursor (VII) and carboxylic acid group of precursor (VIII), thereby obtaining the precursor (III);
wherein R1 to R6, PG1 and y have a meaning as defined in one of claims 1 to 4, PG3 has a meaning as defined in claim 4 and PG5 is an amine protecting group.

7. The method according to claim 5, wherein the precursor according to the general formula (VI) is obtained from the esterification of a precursor according to the general formula (IX) with TAG-OH: 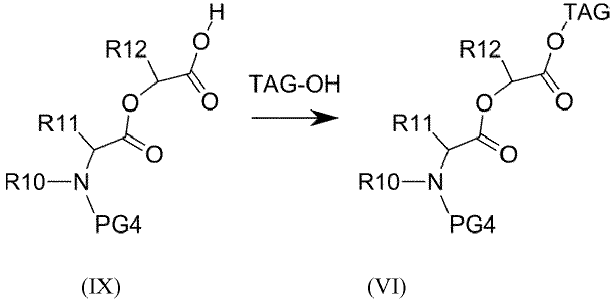 wherein R10 to R12 and TAG have a meaning as defined in one of claims 1 to 4 and PG4 has a meaning as defined in claim 5.

8. The method according to claim 6, wherein the precursor according to the general formula (VII) is obtained from the esterification of a precursor according to the general formula (X) with PG3-OH: 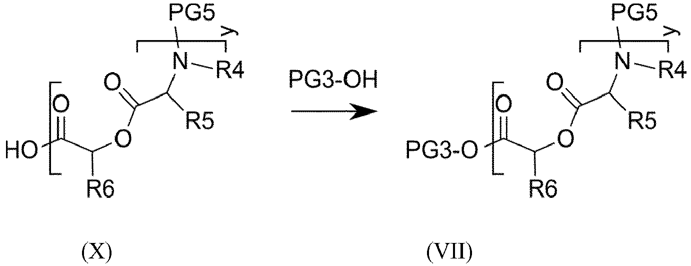 wherein R4 to R6 and y have a meaning as defined in one of claims 1 to 4, PG3 has a meaning as defined in claim 4 and PG5 has a meaning as defined in claim 6.

9. The method according to one of claims 4, 6 or 8, wherein PG3 is TAG as defined in claim 1 or claim 3 or in claim 4 or claim 5.

10. The method according to one of claims 1 to 9, wherein at least one reaction step of the reaction steps resulting in a TAG-bearing molecule is followed by precipitation of crude reaction product in methanol, thereby purifying the crude reaction product.

11. The method according to one of claims 1 to 10, wherein at least one step of the reaction steps in which TAG-protected carboxylic acid groups are deprotected is followed by precipitation of cleaved TAG-OH in methanol and removal of the precipitate by filtration, thereby purifying the crude reaction product.

12. The method according to claim 4, wherein precursors (III) and (IV) are identical.

13. The method according to claim 4, wherein R3 and R9 are different from each other, R1 and R7 are identical, R2 and R8 are identical, R4 and R10 are identical, R5 and R11 are identical and R6 and R12 are identical.

14. The method according to one of claims 1 to 13, wherein the depsipeptide (II) is selected from one of the general formulas (II-1) to (II-14): 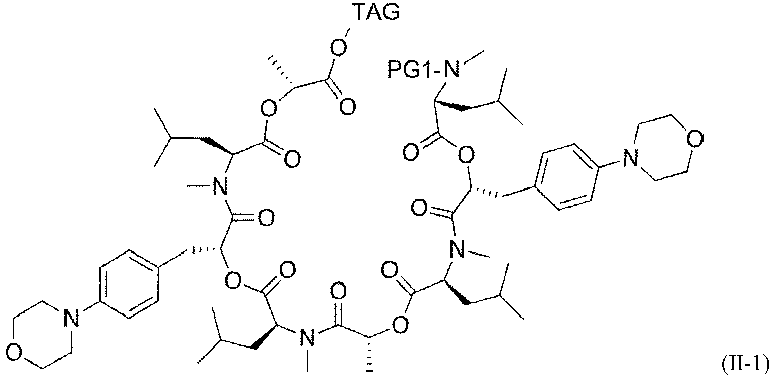 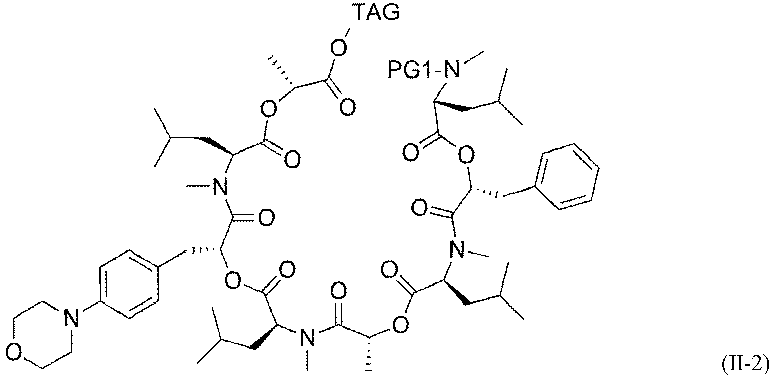 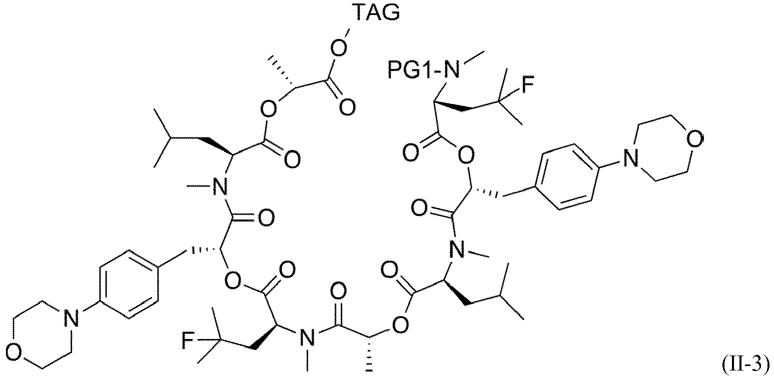 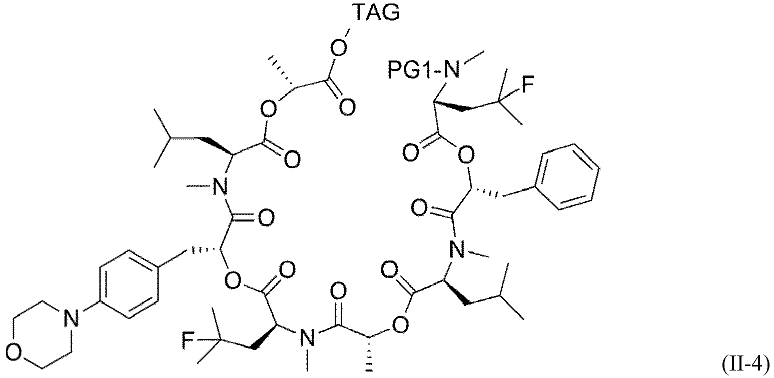 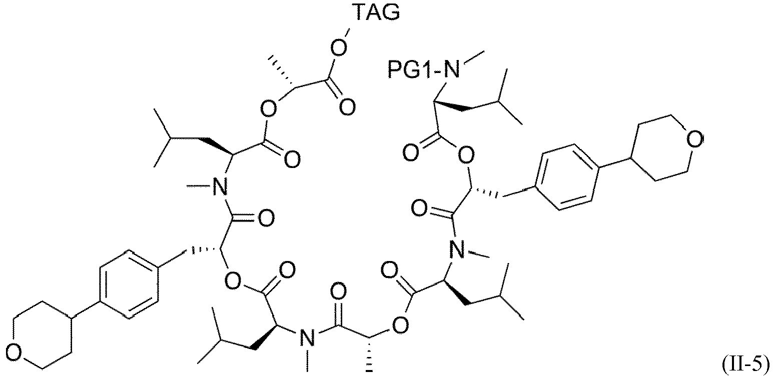 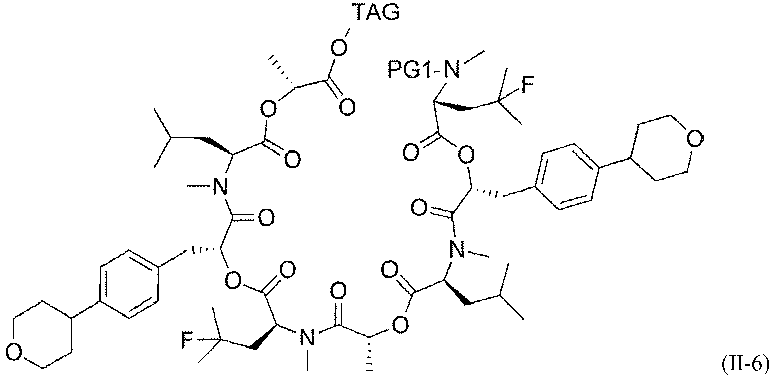 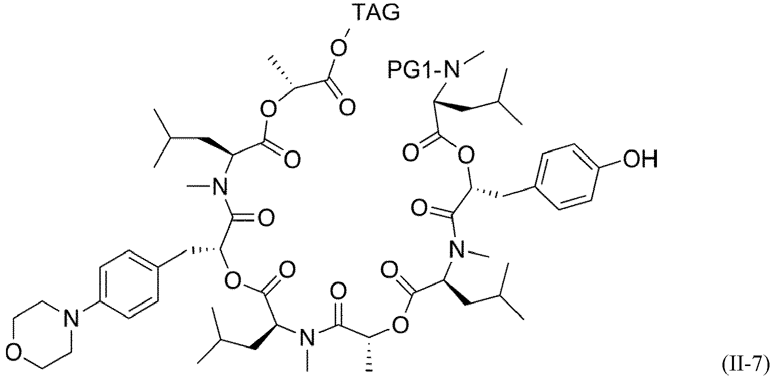 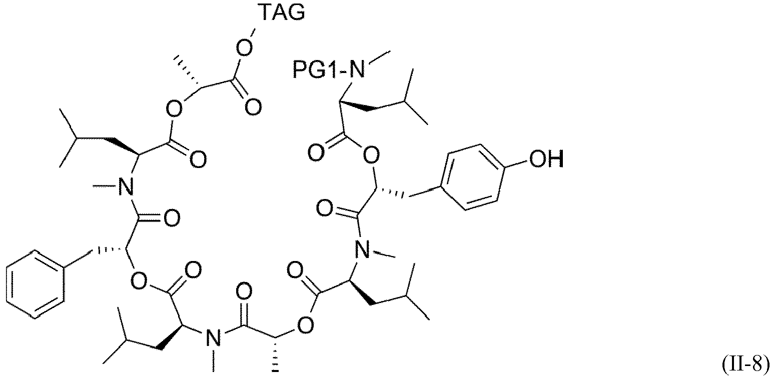 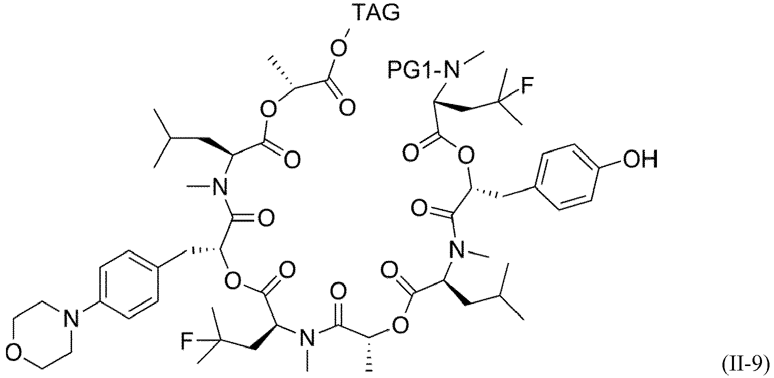 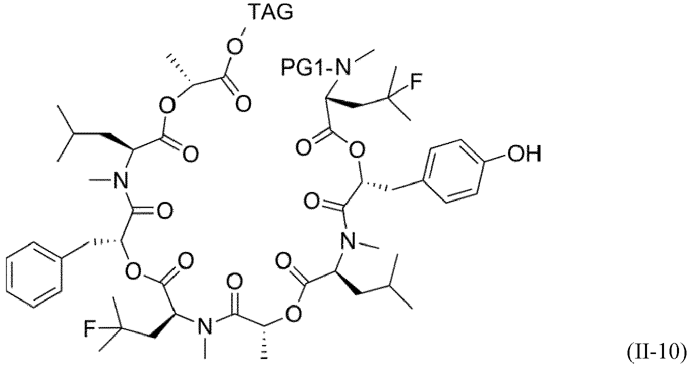 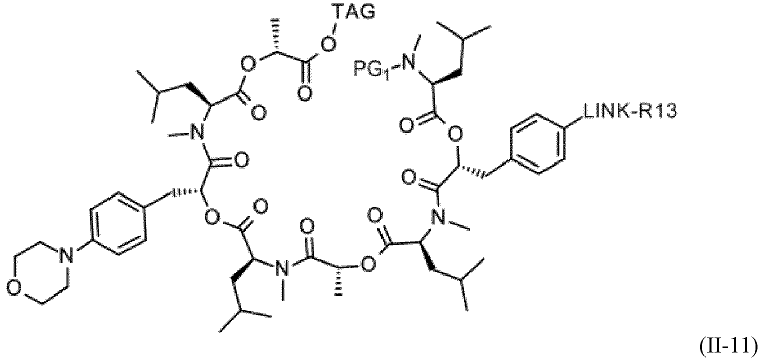 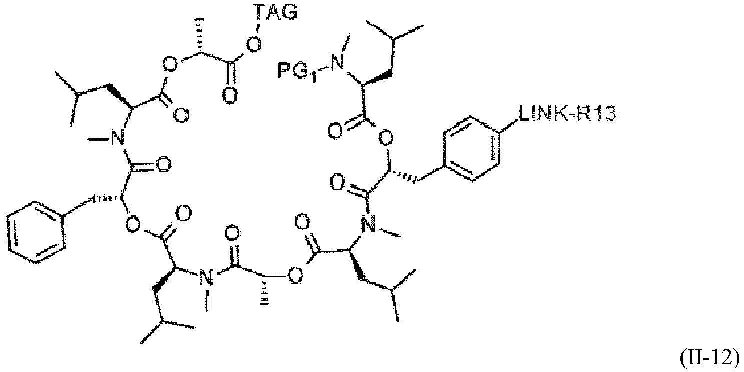 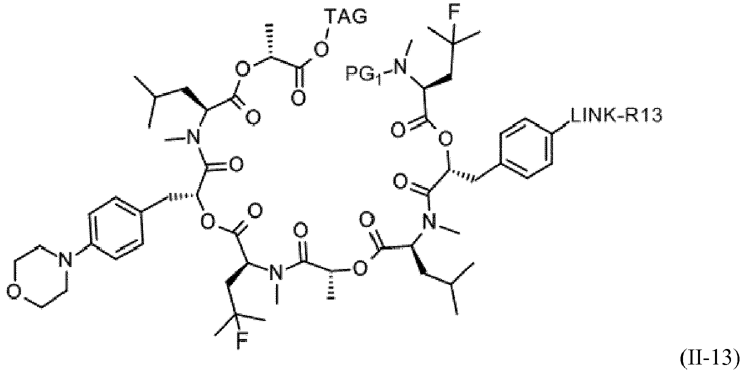 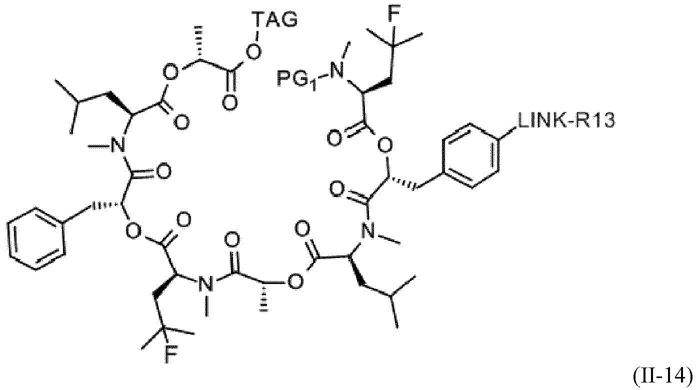 wherein in formulas (II-11) to (II-14) -LINK- is selected from: 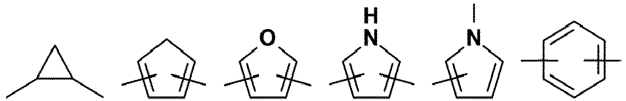 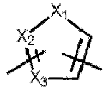 wherein X1 can be C, N, S, O, X2 and X3 can be C or N; 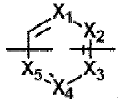 wherein X1 can be C, N, S, O, X2, X3 and X4 can be C or N; 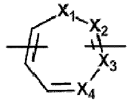 wherein X1, X2, X3 and X4 can be C or N; and R13 is selected from SO₂NH(CH₃), SO₂NH₂, OC(O)CH₃, CF₃ or one the following lactone structures:

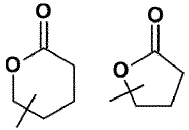

15. A linear or cyclic depsipeptide selected from one of the general formulas (II-1) to (II-14) or (I-1) to (1-9) or a pharmaceutically or veterinarily acceptable salt thereof: 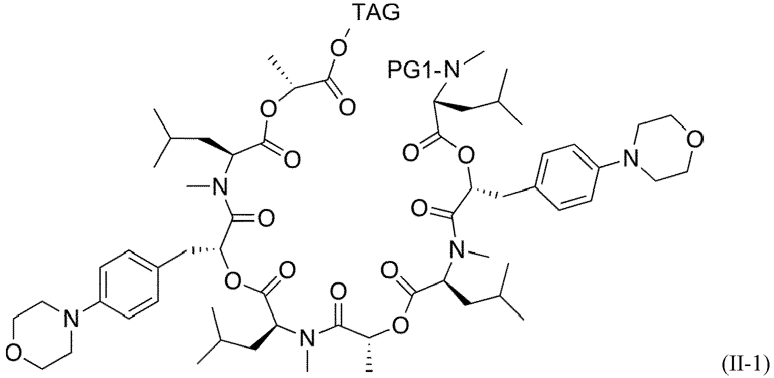 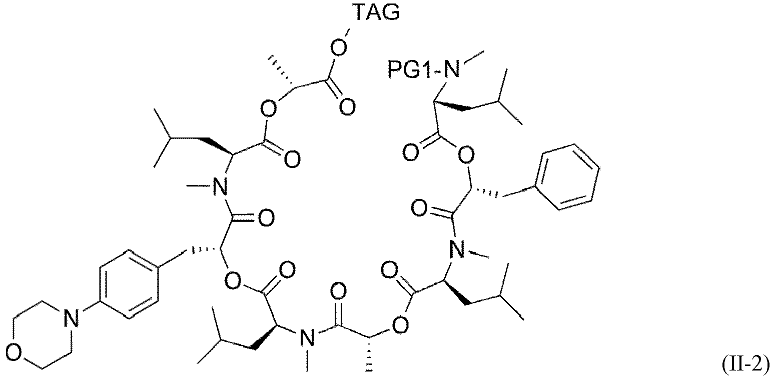 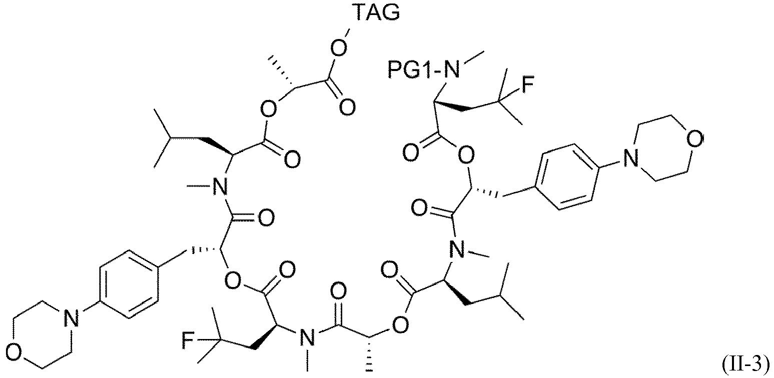 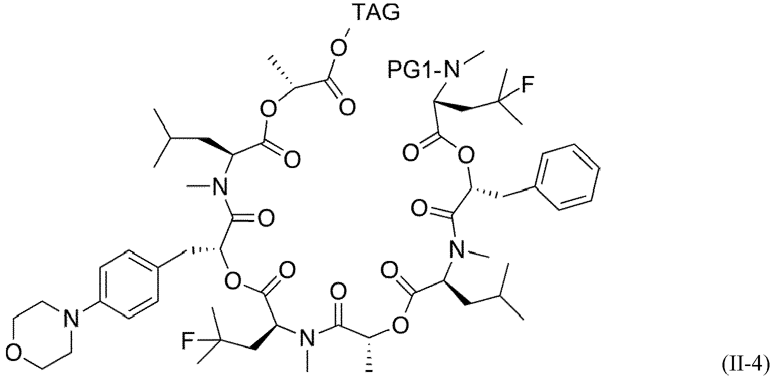 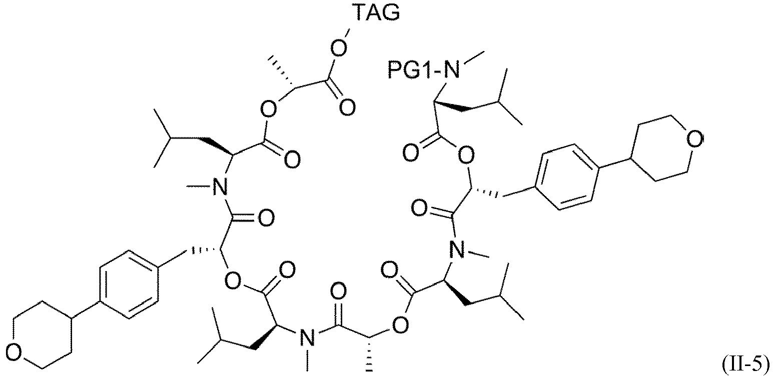 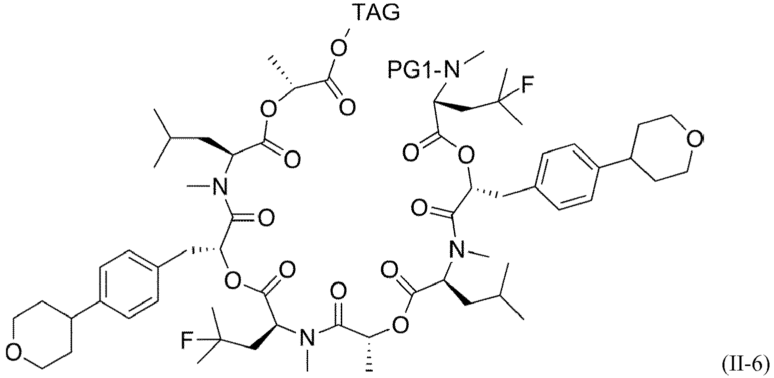 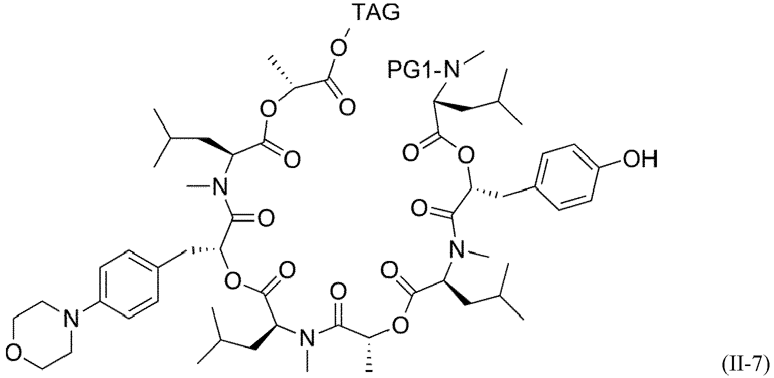 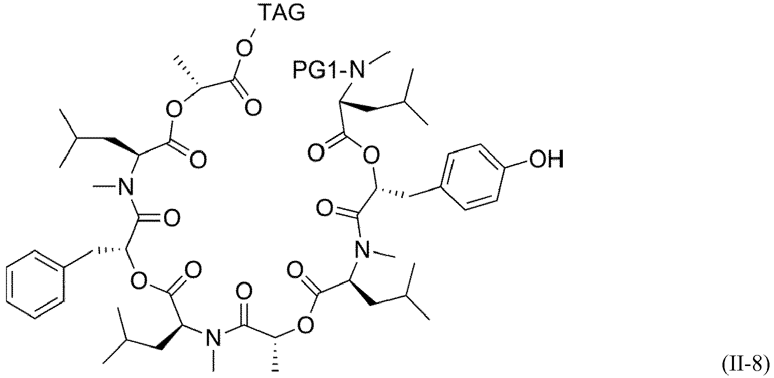 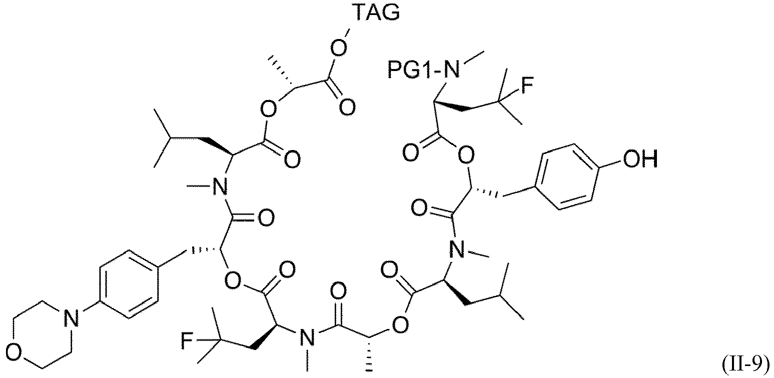 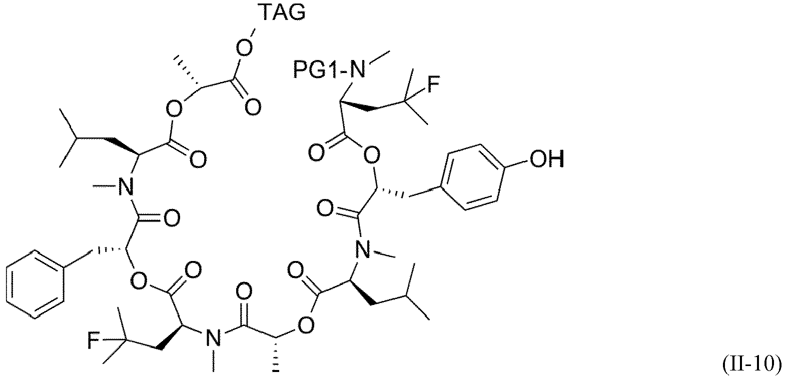 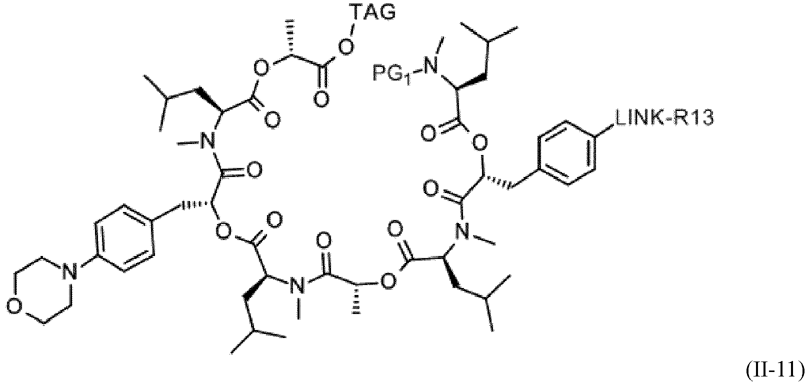 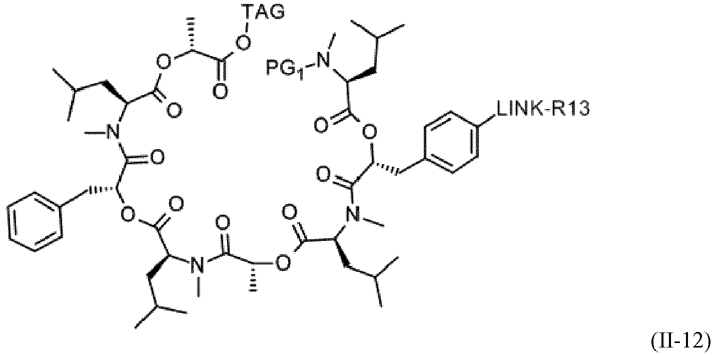 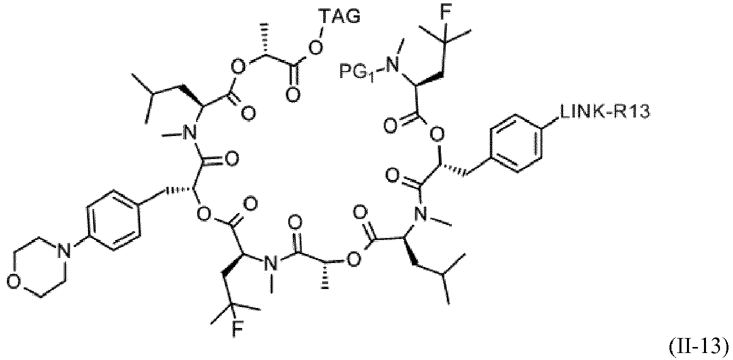 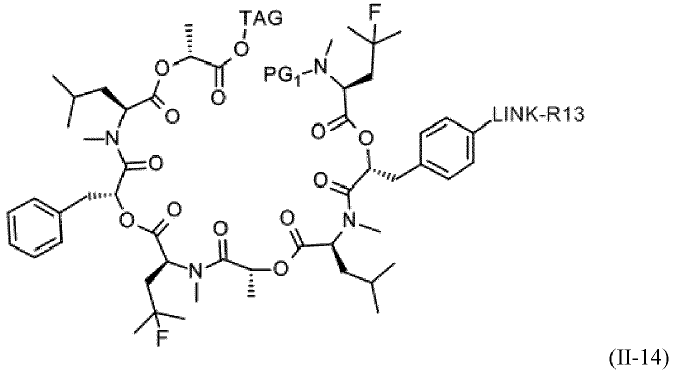 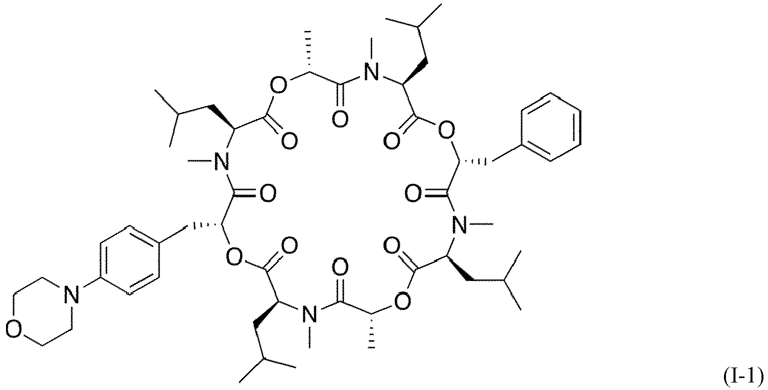 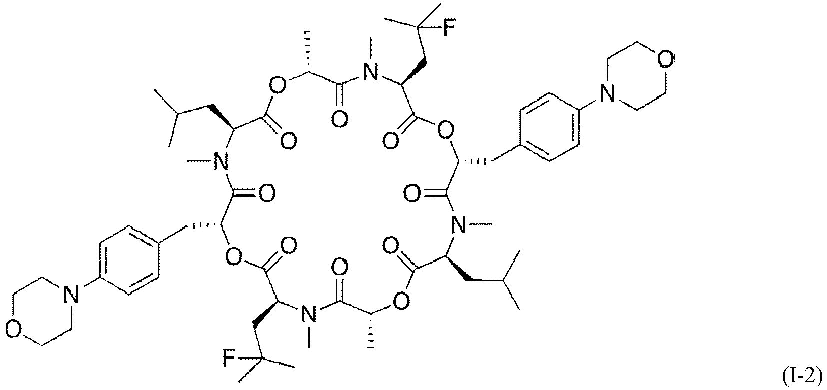 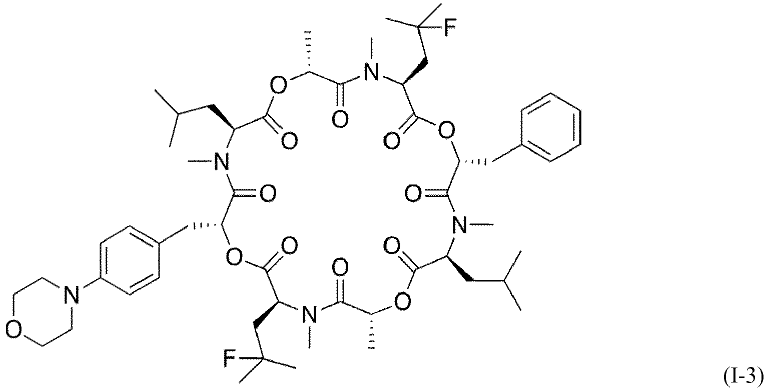 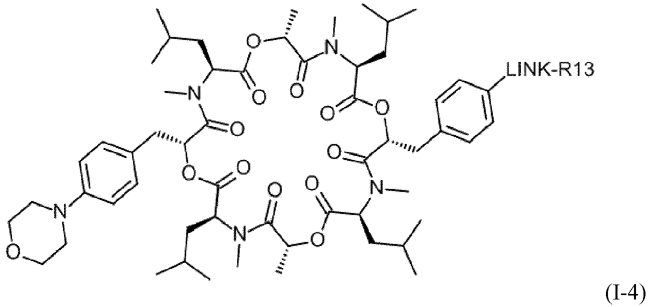 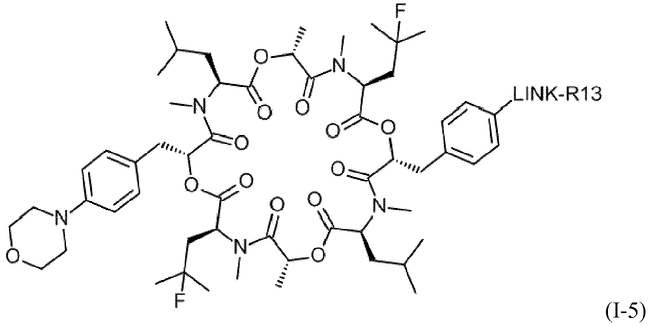 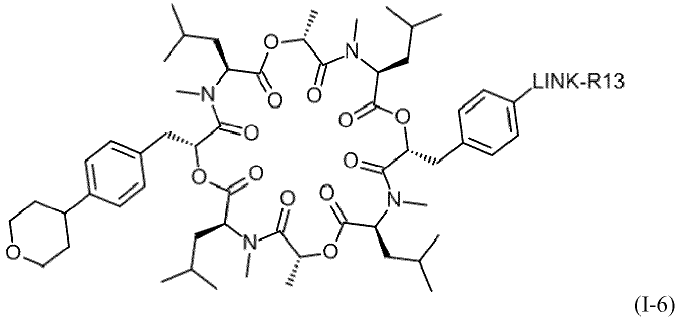 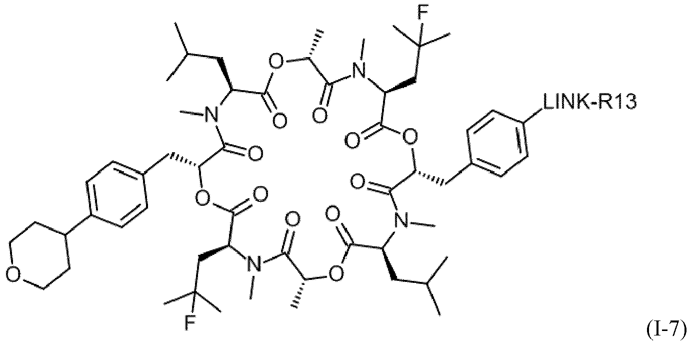 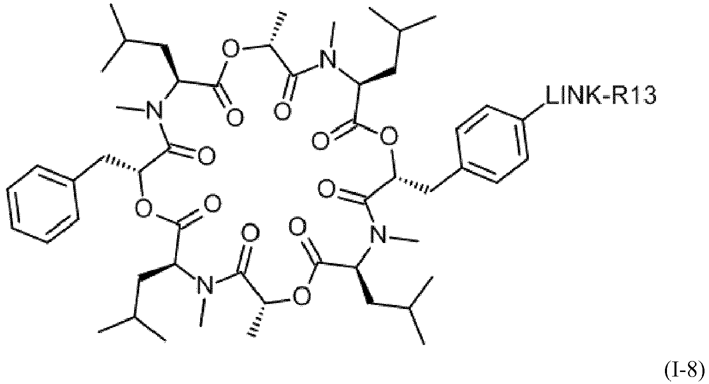 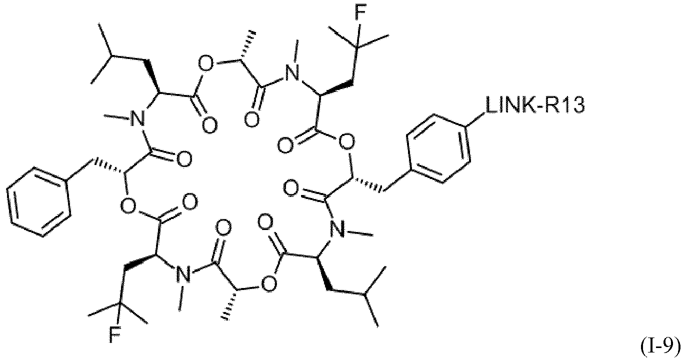 wherein in formulas (II-11) to (II-14) and (I-1) to (1-9), respectively:
PG1 is 9-fluorenylmethoxycarbonyl (Fmoc), t-butyl carbamate (Boc), benzyl carbamate (Z), acetamide, trifluoroacetamide, phthalimide, benzyl (Bn), triphenylmethyl (Tr), benzylidene or p-toluenesulfonamide (Ts),
TAG is: 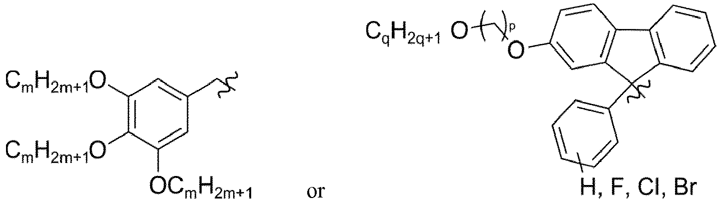 wherein m is ≥ 15 to ≤ 25, p is ≥ 8 to ≤ 18 and q is ≥ 15 to ≤ 25,
-LINK- is selected from: 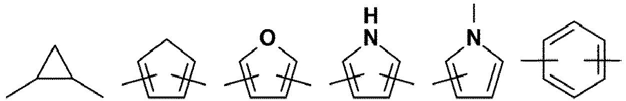
and R13 is selected from SO₂NH(CH₃), SO₂NH₂, OC(O)CH₃, CF₃ or one the following lactone structures:
